(19) Europäisches Patentamt
European Patent Office
Office européen des brevets

(11) EP 1 958 959 A2

(12) # EUROPEAN PATENT APPLICATION
published in accordance with Art. 158(3) EPC

(43) Date of publication:
**20.08.2008 Bulletin 2008/34**

(21) Application number: **06817996.9**

(22) Date of filing: **21.11.2006**

(51) Int Cl.:
*C07K 14/18* (2006.01) *C07K 19/00* (2006.01)
*C07K 16/10* (2006.01) *C12N 15/40* (2006.01)
*C12N 15/62* (2006.01) *C12N 15/63* (2006.01)
*A61K 39/12* (2006.01)

(86) International application number:
**PCT/CU2006/000015**

(87) International publication number:
**WO 2007/059715 (31.05.2007 Gazette 2007/22)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **22.11.2005 CU 2292005**

(71) Applicant: **CENTRO DE INGENIERIA GENETICA Y BIOTECNOLOGIA (CIGB)**
**Ciudad de la Habana 10600 (CU)**

(72) Inventors:
- **CHINEA SANTIAGO, Glay**
**Ciudad de la Habana 11600 (CU)**
- **HUERTA GALINDO, Vivian**
**Ciudad de la Habana 11600 (CU)**
- **MARTÍN DUNN, Alejandro, Miguel**
**Ciudad de la Habana 11600 (CU)**
- **GAVILONDO COWLEY, Jorge, Victor**
**Ciudad de la Habana 10400 (CU)**
- **FLEITAS SALAZAR, Noralvis**
**La Habana 33100 (CU)**
- **GUIROLA CRUZ, Osmany**
**Ciudad de la Habana 10300 (CU)**
- **GIL VALDÉS, Jeovanis**
**Ciudad de la Habana 10900 (CU)**
- **ZULUETA MORALES, Aída**
**Ciudad de la Habana 12500 (CU)**
- **HERMIDA CRUZ, Lisset**
**Ciudad de la Habana 11000 (CU)**
- **AYALA AVILA, Marta**
**Ciudad de la Habana 11600 (CU)**
- **GONZÁLEZ ROCHE, Diamilé**
**Ciudad de la Habana 11600 (CU)**
- **PAEZ MEIRELES, Rolando**
**Ciudad de la Habana 11600 (CU)**
- **TOLEDO MAYORA, Patricia, Gabriela**
**Ciudad de la Habana 11300 (CU)**
- **SARRÍA NÚÑEZ, Mónica**
**Ciudad de la Habana 12800 (CU)**
- **MUSACCHIO LASA, Alexis**
**32100 La Habana (CU)**
- **MAZOLA REYES, Yuliet**
**La Habana 32500 (CU)**

(74) Representative: **Hatzmann, Martin et al**
**Vereenigde**
**Johan de Wittlaan 7**
**2517 JR Den Haag (NL)**

(54) # METHODS AND PROTEINS FOR THE PROPHYLACTIC AND/OR THERAPEUTIC TREATMENT OF THE FOUR SEROTYPES OF DENGUE VIRUS AND OTHER FLAVIVIRUSES

(57) The present invention is related to the field of the pharmaceutical industry, and describes a conserved area on the surface of the E protein that can be used for the development of wide-spectrum antiviral molecules to be employed in the prophylaxis and/or treatment of infections due to Dengue Virus serotypes 1-4 and other flaviviruses. The invention also covers chimeric proteins to be used as vaccines or as a prophylactic or therapeutic treatment against the four serotypes of Dengue Virus and other flaviviruses.

EP 1 958 959 A2

SEQUENCE LISTING

<110> Centro de Ingeniería Genética y Biotecnología

<120> METHODS AND PROTEINS FOR THE PROPHYLACTIC AND/OR THERAPEUTIC TREATMENT OF FOUR SEROTYPES OF DENGUE VIRUS AND OTHER FLAVIVIRUSES

<130> P84850ID00

<150> PCT/CU2006/000015
<151> 2006-11-21

<150> CU 2005-0229
<151> 2005-11-22

<160> 56

<170> PatentIn Ver. 2.1

<210> 1
<211> 75
<212> PRT
<213> Artificial Sequence

<220>
<223> Description of the Artificial Sequence: Polypeptide Sequence PMEC1

<400> 1
Val Thr Lys Asn His Ala Lys Lys Asp Val Val Val Gly Gly Lys Thr
1 5 10 15

Asn Thr Thr Thr Ser Arg Cys Thr Gly Ser Asn Asp Lys Arg Cys Lys
20 25 30

His Ser Met Val Asp Arg Gly Trp Gly Asn Gly Cys Gly Gly Lys Gly
35 40 45

Gly Val Thr Cys Ala Met Thr
50 55

<210> 2
<211> 1485
<212> DNA
<213> Dengue virus type 2

<220>
<221> gene
<222> (1)..(1485)
<223> Gene of the envelope E-glycoprotein of DEN2 virus strain Jamaica 1409 This gene is contained in the plasmid p30-VD2

<400> 2
atgcgttgca taggaatatc aaatagagac tttgtagaag gggtttcagg aggaagctgg 60
gttgacatag tcttagaaca tggaagttgt gtgacgacga tggcaaaaaa taaaccaaca 120
ttggattttg aactgataaa aacagaagcc aaacaacctg ccactctaag gaagtactgt 180
atagaagcaa agctgaccaa tacaacaaca gaatctcgtt gcccaacaca aggggaaccc 240
agtctaaatg aagagcagga caaaaggttc ctctgcaaac actccatggt agacagagga 300
tggggaaatg gatgtggatt atttggaaag ggaggcattg tgacctgtgc tatgtttaca 360
tgcaaaaaga acatggaagg aaaagtcgtg ctgccagaaa atttggaata caccatcgtg 420
ataacacctc actcaggaga agagcacgct gtaggtaatg acacaggaaa acatggcaag 480
gaaatcaaaa taacaccaca gagttccatc acagaagcag aactgacagg ctatggcact 540
gtcacgatgg agtgctctcc gagaacgggc ctcgacttca atgagatggt gctgctgcag 600
atggaagaca aagcttggct ggtgcacagg caatggttcc tagacctgcc gttaccatgg 660
ctacccggag cggacacaca aggatcaaat tggatacaga aagagacatt ggtcactttc 720

aaaaatcccc acgcgaagaa acaagatgtc gttgttttag gatctcaaga aggggccatg 780
cacacggcac tcacaggggc cacagaaatc cagatgtcat caggaaactt actgttcaca 840
ggacatctca agtgcaggct gagaatggac aaactacagc tcaaaggaat gtcatactct 900
atgtgtacag gaaagtttaa aattgtgaag gaaatagcag aaacacaaca tggaacaata 960
gttatcagag tacaatatga aggggacggc tctccatgta agatcccttt tgagataatg 1020
gatttggaaa aaagacacgt cttaggtcgc ctgattacag ttaacccgat cgtaacagaa 1080
aaagatagcc cagtcaacat agaagcagaa cctccattcg gagacagcta catcatcata 1140
ggagtagagc cgggacaatt gaaactcaac tggtttaaga aaggaagttc catcggccaa 1200
atgtttgaga caacaatgag aggagcgaag agaatggcca ttttaggtga cacagcctgg 1260
gattttggat ccctgggagg agtgtttaca tctataggaa aggctctcca ccaagttttc 1320
ggagcaatct atggggctgc ttttagtggg gtctcatgga ctatgaaaat cctcatagga 1380
gtcatcatca catggatagg aatgaattca cgtagcacct cactgtctgt gtcactagta 1440
ttggtgggag tcgtgacact gtacctggga gctatggtgc aggct 1485

<210> 3
<211> 495
<212> PRT
<213> Dengue virus type 2

<220>
<223> Polypeptide. Envelope E-glycoprotein of DEN2 virus strain Jamaica 1409 translation of the gene E of plasmid p30-VD2

<400> 3
Met Arg Cys Ile Gly Ile Ser Asn Arg Asp Phe Val Glu Gly Val Ser
1 5 10 15

Gly Gly Ser Trp Val Asp Ile Val Leu Glu His Gly Ser Cys Val Thr
20 25 30

Thr Met Ala Lys Asn Lys Pro Thr Leu Asp Phe Glu Leu Ile Lys Thr
35 40 45

Glu Ala Lys Gln Pro Ala Thr Leu Arg Lys Tyr Cys Ile Glu Ala Lys
50 55 60

Leu Thr Asn Thr Thr Thr Glu Ser Arg Cys Pro Thr Gln Gly Glu Pro
65 70 75 80

Ser Leu Asn Glu Glu Gln Asp Lys Arg Phe Leu Cys Lys His Ser Met
85 90 95

Val Asp Arg Gly Trp Gly Asn Gly Cys Gly Leu Phe Gly Lys Gly Gly
100 105 110

Ile Val Thr Cys Ala Met Phe Thr Cys Lys Lys Asn Met Glu Gly Lys
115 120 125

Val Val Leu Pro Glu Asn Leu Glu Tyr Thr Ile Val Ile Thr Pro His
130 135 140

Ser Gly Glu Glu His Ala Val Gly Asn Asp Thr Gly Lys His Gly Lys
145 150 155 160

Glu Ile Lys Ile Thr Pro Gln Ser Ser Ile Thr Glu Ala Glu Leu Thr
165 170 175

Gly Tyr Gly Thr Val Thr Met Glu Cys Ser Pro Arg Thr Gly Leu Asp
180 185 190

Phe Asn Glu Met Val Leu Leu Gln Met Glu Asp Lys Ala Trp Leu Val
195 200 205

His Arg Gln Trp Phe Leu Asp Leu Pro Leu Pro Trp Leu Pro Gly Ala
210 215 220

Asp Thr Gln Gly Ser Asn Trp Ile Gln Lys Glu Thr Leu Val Thr Phe

```
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Ile Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg His Val Leu Gly Arg Leu Ile
            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Ser Leu Val
465                 470                 475                 480

Leu Val Gly Val Val Thr Leu Tyr Leu Gly Ala Met Val Gln Ala
                485                 490                 495


<210> 4
<211> 242
<212> DNA
<213> Artificial Sequence

<220>
<223> Description of the Artificial Sequence:  Codifies
      for the protein PMEC1, contains restriction sites
      for cloning in DNA vectors,    contains a silence
      mutation distroying the internal Nco I site

<220>
<223> Synthetic Double stranded DNA

<400> 4
ccatggcatt ggtcactttc aaaaatcccc acgcgaagaa acaagatgtc gttgttggag 60
gtggaaagct gaccaataca acaacagaat ctcgttgccc aacacaaggg gaacccagtc 120
taaatgaaga gcaggacaaa aggttcctct gcaaacactc tatggtagac agaggatggg 180
```

gaaatggatg tggattattt ggaaagggag gcattgtgac ctgtgctatg tttacactcg 240
ag 242

<210> 5
<211> 7
<212> DNA
<213> Artificial Sequence

<220>
<223> Description of the Artificial Sequence: Contains a Nco I restriction site, an ATG codon codifing for methionine and a GCA codon for alanine

<220>
<223> Oligonucleotide fragment

<400> 5
catggca 7

<210> 6
<211> 48
<212> DNA
<213> Dengue virus type 2

<220>
<223> Oligonucleotide fragment

<220>
<223> Contains the sequence 709-756 of the gene codifying for the E-protein of DEN2 virus strain Jamaica 1409

<400> 6
ttggtcactt tcaaaaatcc ccacgcgaag aaacaagatg tcgttgtt 48

<210> 7
<211> 16
<212> PRT
<213> Dengue virus type 2

<220>
<223> Peptide fragment. Corresponds to the fragment 237-252 of the E-protein of DEN2 virus strain Jamaica 1409

<400> 7
Leu Val Thr Phe Lys Asn Pro His Ala Lys Lys Gln Asp Val Val Val
1 5 10 15

<210> 8
<211> 9
<212> DNA
<213> Artificial Sequence

<220>
<223> Description of the Artificial Sequence: Contains 3 codons codifying for 3 consecutive glycines

<220>
<223> Oligonucleotide fragment

<400> 8
ggaggtgga 9

<210> 9

<211> 57
<212> PRT
<213> Dengue virus type 2

<220>
<223> Peptide fragment. Corresponds to the fragment
64-120 of the E-protein of DEN2 virus strain
Jamaica 1409

<400> 9

```
Lys Leu Thr Asn Thr Thr Thr Glu Ser Arg Cys Pro Thr Gln Gly Glu
  1               5                  10                  15

Pro Ser Leu Asn Glu Glu Gln Asp Lys Arg Phe Leu Cys Lys His Ser
             20                  25                  30

Met Val Asp Arg Gly Trp Gly Asn Gly Cys Gly Leu Phe Gly Lys Gly
         35                  40                  45

Gly Ile Val Thr Cys Ala Met Phe Thr
     50                  55
```

<210> 10
<211> 171
<212> DNA
<213> Dengue virus type 2

<220>
<223> Oligonucleotide fragment. Contains the sequence
190-360 of the gene codifying for the E-protein
of DEN2 virus strain Jamaica 1409.

<400> 10

```
aagctgacca atacaacaac agaatctcgt tgcccaacac aaggggaacc cagtctaaat 60
gaagagcagg acaaaaggtt cctctgcaaa cactccatgg tagacagagg atggggaaat 120
ggatgtggat tatttggaaa gggaggcatt gtgacctgtg ctatgtttac a          171
```

<210> 11
<211> 6
<212> DNA
<213> Artificial Sequence

<220>
<223> Description of the Artificial Sequence:Contains
a Xho I restriction site which contains a CTC
codon codifying for leucine and a GAG codon
codifying for a glutamic acid

<220>
<223> Oligonucleotide fragment.

<400> 11

```
ctcgag                                                            6
```

<210> 12
<211> 5664
<212> DNA
<213> Artificial Sequence

<220>
<223> Description of the Artificial Sequence :Codifies
for the expression of the protein sPMEC1-His6
under the T7 promoter

<220>
<223> Plasmid.

<400> 12
taatacgact cactataggg gaattgtgag cggataacaa ttcccctcta gaaataattt 60
tgtttaactt taagaaggag atatacatat gaaatacctg ctgccgaccg ctgctgctgg 120
tctgctgctc ctcgctgccc agccggcgat ggccatggca ttggtcactt tcaaaaatcc 180
ccacgcgaag aaacaagatg tcgttgttgg aggtaagctg accaatacaa caacagaatc 240
tcgttgccca acacaagggg aacccagtct aaatgaagag caggacaaaa ggttcctctg 300
caaacactct atggtagaca gaggatgggg aaatggatgt ggattatttg gaaagggagg 360
cattgtgacc tgtgctatgt ttacactcga gcaccaccac caccaccact gagatccggc 420
tgctaacaaa gcccgaaagg aagctgagtt ggctgctgcc accgctgagc aataactagc 480
ataacccctt ggggcctcta aacgggtctt gaggggtttt ttgctgaaag gaggaactat 540
atccggattg gcgaatggga cgcgccctgt agcggcgcat taagcgcggc gggtgtggtg 600
gttacgcgca gcgtgaccgc tacacttgcc agcgccctag cgcccgctcc tttcgctttc 660
ttcccttcct ttctcgccac gttcgccggc tttccccgtc aagctctaaa tcgggggctc 720
cctttagggt tccgatttag tgctttacgg cacctcgacc ccaaaaaact tgattagggt 780
gatggttcac gtagtgggcc atcgccctga tagacggttt ttcgcccttt gacgttggag 840
tccacgttct ttaatagtgg actcttgttc caaactggaa caacactcaa ccctatctcg 900
gtctattctt ttgatttata agggattttg ccgatttcgg cctattggtt aaaaaatgag 960
ctgatttaac aaaaatttaa cgcgaatttt aacaaaatat taacgtttac aatttcaggt 1020
ggcacttttc ggggaaatgt gcgcggaacc cctatttgtt tatttttcta aatacattca 1080
aatatgtatc cgctcatgag acaataaccc tgataaatgc ttcaataata ttgaaaaagg 1140
aagagtatga gtattcaaca tttccgtgtc gcccttattc cctttttgc ggcattttgc 1200
cttcctgttt ttgctcaccc agaaacgctg gtgaaagtaa aagatgctga agatcagttg 1260
ggtgcacgag tgggttacat cgaactggat ctcaacagcg gtaagatcct tgagagtttt 1320
cgccccgaag aacgttttcc aatgatgagc acttttaaag ttctgctatg tggcgcggta 1380
ttatcccgta ttgacgccgg gcaagagcaa ctcggtcgcc gcatacacta ttctcagaat 1440
gacttggttg agtactcacc agtcacagaa aagcatctta cggatggcat gacagtaaga 1500
gaattatgca gtgctgccat aaccatgagt gataacactg cggccaactt acttctgaca 1560
acgatcggag gaccgaagga gctaaccgct tttttgcaca acatggggga tcatgtaact 1620
cgccttgatc gttgggaacc ggagctgaat gaagccatac caaacgacga gcgtgacacc 1680
acgatgcctg cagcaatggc aacaacgttg cgcaaactat taactggcga actacttact 1740
ctagcttccc ggcaacaatt aatagactgg atggaggcgg ataaagttgc aggaccactt 1800
ctgcgctcgg cccttccggc tggctggttt attgctgata aatctggagc cggtgagcgt 1860
gggtctcgcg gtatcattgc agcactgggg ccagatggta agccctcccg tatcgtagtt 1920
atctacacga cggggagtca ggcaactatg gatgaacgaa atagacagat cgctgagata 1980
ggtgcctcac tgattaagca ttggtaactg tcagaccaag tttactcata tatactttag 2040
attgatttaa aacttcattt ttaatttaaa aggatctagg tgaagatcct ttttgataat 2100
ctcatgacca aaatccctta acgtgagttt tcgttccact gagcgtcaga ccccgtagaa 2160
aagatcaaag gatcttcttg agatcctttt tttctgcgcg taatctgctg cttgcaaaca 2220
aaaaaaccac cgctaccagc ggtggtttgt ttgccggatc aagagctacc aactcttttt 2280
ccgaaggtaa ctggcttcag cagagcgcag ataccaaata ctgtccttct agtgtagccg 2340
tagttaggcc accacttcaa gaactctgta gcaccgccta catacctcgc tctgctaatc 2400
ctgttaccag tggctgctgc cagtggcgat aagtcgtgtc ttaccgggtt ggactcaaga 2460
cgatagttac cggataaggc gcagcggtcg ggctgaacgg ggggttcgtg cacacagccc 2520
agcttggagc gaacgaccta caccgaactg agatacctac agcgtgagct atgagaaagc 2580
gccacgcttc ccgaagggag aaaggcggac aggtatccgg taagcggcag ggtcggaaca 2640
ggagagcgca cgagggagct tccaggggga aacgcctggt atctttatag tcctgtcggg 2700
tttcgccacc tctgacttga gcgtcgattt ttgtgatgct cgtcaggggg gcggagccta 2760
tggaaaaacg ccagcaacgc ggccttttta cggttcctgg ccttttgctg gccttttgct 2820
cacatgttct ttcctgcgtt atcccctgat tctgtggata accgtattac cgcctttgag 2880
tgagctgata ccgctcgccg cagccgaacg accgagcgca gcgagtcagt gagcgaggaa 2940
gcggaagagc gcctgatgcg gtattttctc cttacgcatc tgtgcggtat ttcacaccgc 3000
atatatggtg cactctcagt acaatctgct ctgatgccgc atagttaagc cagtatacac 3060
tccgctatcg ctacgtgact gggtcatggc tgcgccccga cacccgccaa cacccgctga 3120
cgcgccctga cgggcttgtc tgctcccggc atccgcttac agacaagctg tgaccgtctc 3180
cgggagctgc atgtgtcaga ggttttcacc gtcatcaccg aaacgcgcga ggcagctgcg 3240
gtaaagctca tcagcgtggt cgtgaagcga ttcacagatg tctgcctgtt catccgcgtc 3300
cagctcgttg agtttctcca gaagcgttaa tgtctggctt ctgataaagc gggccatgtt 3360
aagggcggtt ttttcctgtt tggtcactga tgcctccgtg taagggggat ttctgttcat 3420
gggggtaatg ataccgatga aacgagagag gatgctcacg atacgggtta ctgatgatga 3480
acatgcccgg ttactggaac gttgtgaggg taaacaactg gcggtatgga tgcggcggga 3540
ccagagaaaa atcactcagg gtcaatgcca gcgcttcgtt aatacagatg taggtgttcc 3600
acagggtagc cagcagcatc ctgcgatgca gatccggaac ataatggtgc agggcgctga 3660
cttccgcgtt tccagacttt acgaaacacg gaaaccgaag accattcatg ttgttgctca 3720
ggtcgcagac gttttgcagc agcagtcgct tcacgttcgc tcgcgtatcg gtgattcatt 3780
ctgctaacca gtaaggcaac cccgccagcc tagccgggtc ctcaacgaca ggagcacgat 3840
catgcgcacc cgtggggccg ccatgccggc gataatggcc tgcttctcgc cgaaacgttt 3900
ggtggcggga ccagtgacga aggcttgagc gagggcgtgc aagattccga ataccgcaag 3960
cgacaggccg atcatcgtcg cgctccagcg aaagcggtcc tcgccgaaaa tgacccagag 4020

```
cgctgccggc acctgtccta cgagttgcat gataaagaag acagtcataa gtgcggcgac 4080
gatagtcatg ccccgcgccc accggaagga gctgactggg ttgaaggctc tcaagggcat 4140
cggtcgagat cccggtgcct aatgagtgag ctaacttaca ttaattgcgt tgcgctcact 4200
gcccgctttc cagtcgggaa acctgtcgtg ccagctgcat taatgaatcg gccaacgcgc 4260
ggggagaggc ggtttgcgta ttgggcgcca gggtggtttt tcttttcacc agtgagacgg 4320
gcaacagctg attgcccttc accgcctggc cctgagagag ttgcagcaag cggtccacgc 4380
tggtttgccc cagcaggcga aaatcctgtt tgatggtggt taacggcggg atataacatg 4440
agctgtcttc ggtatcgtcg tatcccacta ccgagatatc cgcaccaacg cgcagcccgg 4500
actcggtaat ggcgcgcatt gcgcccagcg ccatctgatc gttggcaacc agcatcgcag 4560
tgggaacgat gccctcattc agcatttgca tggtttgttg aaaaccggac atggcactcc 4620
agtcgccttc ccgttccgct atcggctgaa tttgattgcg agtgagatat ttatgccagc 4680
cagccagacg cagacgcgcc gagacagaac ttaatgggcc cgctaacagc gcgatttgct 4740
ggtgacccaa tgcgaccaga tgctccacgc ccagtcgcgt accgtcttca tgggagaaaa 4800
taatactgtt gatgggtgtc tggtcagaga catcaagaaa taacgccgga acattagtgc 4860
aggcagcttc cacagcaatg gcatcctggt catccagcgg atagttaatg atcagcccac 4920
tgacgcgttg cgcgagaaga ttgtgcaccg ccgctttaca ggcttcgacg ccgcttcgtt 4980
ctaccatcga caccaccacg ctggcaccca gttgatcggc gcgagattta atcgccgcga 5040
caatttgcga cggcgcgtgc agggccagac tggaggtggc aacgccaatc agcaacgact 5100
gtttgcccgc cagttgttgt gccacgcggt tgggaatgta attcagctcc gccatcgccg 5160
cttccacttt ttcccgcgtt ttcgcagaaa cgtggctggc ctggttcacc acgcgggaaa 5220
cggtctgata agagacaccg gcatactctg cgacatcgta taacgttact ggtttcacat 5280
tcaccaccct gaattgactc tcttccgggc gctatcatgc cataccgcga aaggttttgc 5340
gccattcgat ggtgtccggg atctcgacgc tctcccttat gcgactcctg cattaggaag 5400
cagcccagta gtaggttgag gccgttgagc accgccgccg caaggaatgg tgcatgcaag 5460
gagatggcgc ccaacagtcc cccggccacg gggcctgcca ccatacccac gccgaaacaa 5520
gcgctcatga gcccgaagtg gcgagcccga tcttccccat cggtgatgtc ggcgatatag 5580
gcgccagcaa ccgcacctgt ggcgccggtg atgccggcca cgatgcgtcc ggcgtagagg 5640
atcgagatct cgatcccgcg aaat                                        5664
```

<210> 13
<211> 108
<212> PRT
<213> Artificial Sequence

<220>
<223> Description of the Artificial Sequence: Immature protein sPMEC1-His6 of plasmid pET-PMEC1, before secretion to the periplasm

<220>
<223> Protein.

<400> 13

```
Met Lys Tyr Leu Leu Pro Thr Ala Ala Ala Gly Leu Leu Leu Leu Ala
  1               5                  10                  15

Ala Gln Pro Ala Met Ala Met Ala Leu Val Thr Phe Lys Asn Pro His
             20                  25                  30

Ala Lys Lys Gln Asp Val Val Val Gly Gly Gly Lys Leu Thr Asn Thr
         35                  40                  45

Thr Thr Glu Ser Arg Cys Pro Thr Gln Gly Glu Pro Ser Leu Asn Glu
     50                  55                  60

Glu Gln Asp Lys Arg Phe Leu Cys Lys His Ser Met Val Asp Arg Gly
 65                  70                  75                  80

Trp Gly Asn Gly Cys Gly Leu Phe Gly Lys Gly Gly Ile Val Thr Cys
                 85                  90                  95

Ala Met Phe Thr Leu Glu His His His His His His
            100                 105
```

<210> 14
<211> 83
<212> PRT

<213> Artificial Sequence

<220>
<223> Description of the Artificial Sequence :This is the mature protein codifyied by the plasmid pET-PMEC1, after secreted to the periplasm the signal peptide is removed

<220>
<223> Protein.

<400> 14
```
Leu Val Thr Phe Lys Asn Pro His Ala Lys Lys Gln Asp Val Val Val
1               5                   10                  15

Gly Gly Lys Leu Thr Asn Thr Thr Thr Glu Ser Arg Cys Pro Thr Gln
            20                  25                  30

Gly Glu Pro Ser Leu Asn Glu Glu Gln Asp Lys Arg Phe Leu Cys Lys
        35                  40                  45

His Ser Met Val Asp Arg Gly Trp Gly Asn Gly Cys Gly Leu Phe Gly
    50                  55                  60

Lys Gly Gly Ile Val Thr Cys Ala Met Phe Thr Leu Glu His His His
65                  70                  75                  80

His His His
```

<210> 15
<211> 734
<212> DNA
<213> Artificial Sequence

<220>
<223> Description of the Artificial Sequence : Contains a gene codifying for a single chain antibody fragment displaying the variable regions of mAb 4G2

<220>
<223> Double stranded synthetic DNA.

<400> 15
```
ccatggcaaa cattgtgatg acccaatctc ccaaatccat gtccatgtca gtaggagaga 60
gggtcacctt gacctgcaag gccagtgaga atgtggttac ttatgtttcc tggtatcaac 120
agaaaccaga gcagtctcct aaactgctga tatacggggc atccaaccgg tacactgggg 180
tccccgatcg cttcacaggc agtggatctg caacagattt cactctgacc atcagcagtg 240
tgcaggctga agaccttgca gattatcact gtggacaggg ttacagctat ccgtacacgt 300
tcggaggggg gaccaagctg gaaataaaag agggtaaatc ctcaggatca ggctccgaat 360
ccaaagtcga cgaggtccag ctgcaacaat ctggacctga gctggtgaag cctgggactt 420
cagtgaagat atcctgcaag acttctggat acacattcac tgaatatacc atacactggg 480
tgaagcagag ccacggaaag agccttgcgt ggattggagg tattgatcct aacagtggtg 540
gtactaacta cagcccgaac ttcaagggca aggccacatt gactgttgac aagtcctcca 600
gcacagccta catggacctc cgcagcctgt catctgagga ttctgcagtc tacttctgtg 660
caaggatcta tcattacgac ggatacttcg atgtctgggg cgcagggacc gccgtcaccg 720
tctcctcact cgag                                                   734
```

<210> 16
<211> 905
<212> DNA
<213> Artificial Sequence

<220>
<223> Description of the Artificial Sequence :Contains a gene codifying for a protein displaying the variable regions of mAb 4G2 and a trimeric domain of

matrilin

<220>
<223> Polinucleótido sintético.

<400> 16
ccatggcaaa cattgtgatg acccaatctc ccaaatccat gtccatgtca gtaggagaga 60
gggtcacctt gacctgcaag gccagtgaga atgtggttac ttatgtttcc tggtatcaac 120
agaaaccaga gcagtctcct aaactgctga tatacggggc atccaaccgg tacactgggg 180
tccccgatcg cttcacaggc agtggatctg caacagattt cactctgacc atcagcagtg 240
tgcaggctga agaccttgca gattatcact gtggacaggg ttacagctat ccgtacacgt 300
tcggaggggg gaccaagctg gaaataaaag agggtaaatc ctcaggatca ggctccgaat 360
ccaaagtcga cgaggtccag ctgcaacaat ctggacctga gctggtgaag cctgggactt 420
cagtgaagat atcctgcaag acttctggat acacattcac tgaatatacc atacactggg 480
tgaagcagag ccacggaaag agccttgcgt ggattggagg tattgatcct aacagtggtg 540
gtactaacta cagcccgaac ttcaagggca aggccacatt gactgttgac aagtcctcca 600
gcacagccta catggacctc cgcagcctgt catctgagga ttctgcagtc tacttctgtg 660
caaggatcta tcattacgac ggatacttcg atgtctgggg cgcagggacc gccgtcaccg 720
tctcctcagg tggtggttcc ggtggtggtt ccggtggtgg ttccggtggt ggtgaagacc 780
cgtgcgcttg cgaatccctg gttaaattcc aggctaaagt tgaaggtctg ctgcaggctc 840
tgacccgtaa actggaagct gtttccaaac gtctggctat cctggaaaac accgttgttc 900
tcgag 905

<210> 17
<211> 1439
<212> DNA
<213> Artificial Sequence

<220>
<223> Description of the Artificial Sequence : Contains
a gene codifying for the synthesis of a
single chain miniantibody displaying
the variable regions of mAb 4G2

<220>
<223> Double stranded synthetic DNA.

<400> 17
ccatggcaaa cattgtgatg acccaatctc ccaaatccat gtccatgtca gtaggagaga 60
gggtcacctt gacctgcaag gccagtgaga atgtggttac ttatgtttcc tggtatcaac 120
agaaaccaga gcagtctcct aaactgctga tatacggggc atccaaccgg tacactgggg 180
tccccgatcg cttcacaggc agtggatctg caacagattt cactctgacc atcagcagtg 240
tgcaggctga agaccttgca gattatcact gtggacaggg ttacagctat ccgtacacgt 300
tcggaggggg gaccaagctg gaaataaaag agggtaaatc ctcaggatca ggctccgaat 360
ccaaagtcga cgaggtccag ctgcaacaat ctggacctga gctggtgaag cctgggactt 420
cagtgaagat atcctgcaag acttctggat acacattcac tgaatatacc atacactggg 480
tgaagcagag ccacggaaag agccttgcgt ggattggagg tattgatcct aacagtggtg 540
gtactaacta cagcccgaac ttcaagggca aggccacatt gactgttgac aagtcctcca 600
gcacagccta catggacctc cgcagcctgt catctgagga ttctgcagtc tacttctgtg 660
caaggatcta tcattacgac ggatacttcg atgtctgggg cgcagggacc gccgtcaccg 720
tctcctcagg cggtggcgag cccaaatctt ctgacaaaac tcacacatgc ccaccgtgcc 780
cagcacctga actcctgggg ggaccgtcag tcttcctctt ccccccaaaa cccaaggaca 840
ccctcatgat ctcccggacc cctgaggtca catgcgtggt ggtggacgtg agccacgaag 900
accctgaggt caagttcaac tggtacgtgg acggcgtgga ggtgcataat gccaagacaa 960
agccgcggga ggagcagtac cagagcacgt accgtgtggt cagcgtcctc accgtcctgc 1020
accaggactg gctgaatggc aaggagtaca agtgcaaggt ctccaacaaa gccctcccag 1080
cccccatcga gaaaaccatc tccaaagcca aagggcagcc ccgagaacca caggtgtaca 1140
ccctgccccc atcccgggag gagatgacca agaaccaggt cagcctgacc tgcctggtca 1200
aaggcttcta tcccagcgac atcgccgtgg agtgggagag caatgggcag ccggagaaca 1260
actacaagac cacgcctccc gtgctggact ccgacggctc cttcttcctc tatagcaagc 1320
tcaccgtgga caagagcagg tggcagcagg ggaacgtctt ctcatgctcc gtgatgcatg 1380
aggctctgca caaccactac acgcagaaga gcctctccct gtccccgggt aaactcgag 1439

<210> 18
<211> 6159
<212> DNA
<213> Artificial Sequence

<220>
<223> Description of the Artificial Sequence :Expression plasmid under the T7 promoter corresponding to a single chain antibody fragment displaying the variables regions of mAb 4G2

<220>
<223> Plasmidio.

<400> 18
ccatggcaaa cattgtgatg acccaatctc ccaaatccat gtccatgtca gtaggagaga 60
gggtcacctt gacctgcaag gccagtgaga atgtggttac ttatgtttcc tggtatcaac 120
agaaaccaga gcagtctcct aaactgctga tatacggggc atccaaccgg tacactgggg 180
tccccgatcg cttcacaggc agtggatctg caacagattt cactctgacc atcagcagtg 240
tgcaggctga agaccttgca gattatcact gtggacaggg ttacagctat ccgtacacgt 300
tcggaggggg gaccaagctg gaaataaaag agggtaaatc ctcaggatca ggctccgaat 360
ccaaagtcga cgaggtccag ctgcaacaat ctggacctga gctggtgaag cctgggactt 420
cagtgaagat atcctgcaag acttctggat acacattcac tgaatatacc atacactggg 480
tgaagcagag ccacggaaag agccttgcgt ggattggagg tattgatcct aacagtggtg 540
gtactaacta cagcccgaac ttcaagggca aggccacatt gactgttgac aagtcctcca 600
gcacagccta catggacctc cgcagcctgt catctgagga ttctgcagtc tacttctgtg 660
caaggatcta tcattacgac ggatacttcg atgtctgggg cgcagggacc gccgtcaccg 720
tctcctcact cgagcaccac caccaccacc actgagatcc ggctgctaac aaagcccgaa 780
aggaagctga gttggctgct gccaccgctg agcaataact agcataaccc cttggggcct 840
ctaaacgggt cttgaggggt tttttgctga aaggaggaac tatatccgga ttggcgaatg 900
ggacgcgccc tgtagcggcg cattaagcgc ggcgggtgtg gtggttacgc gcagcgtgac 960
cgctacactt gccagcgccc tagcgcccgc tcctttcgct ttcttccctt cctttctcgc 1020
cacgttcgcc ggctttcccc gtcaagctct aaatcggggg ctccctttag ggttccgatt 1080
tagtgcttta cggcacctcg accccaaaaa acttgattag ggtgatggtt cacgtagtgg 1140
gccatcgccc tgatagacgg tttttcgccc tttgacgttg gagtccacgt tctttaatag 1200
tggactcttg ttccaaactg gaacaacact caaccctatc tcggtctatt cttttgattt 1260
ataagggatt ttgccgattt cggcctattg gttaaaaaat gagctgattt aacaaaaatt 1320
taacgcgaat tttaacaaaa tattaacgtt tacaatttca ggtggcactt ttcggggaaa 1380
tgtgcgcgga acccctattt gtttattttt ctaaatacat tcaaatatgt atccgctcat 1440
gagacaataa ccctgataaa tgcttcaata atattgaaaa aggaagagta tgagtattca 1500
acatttccgt gtcgccctta ttcccttttt tgcggcattt tgccttcctg tttttgctca 1560
cccagaaacg ctggtgaaag taaaagatgc tgaagatcag ttgggtgcac gagtgggtta 1620
catcgaactg gatctcaaca gcggtaagat ccttgagagt tttcgccccg aagaacgttt 1680
tccaatgatg agcactttta aagttctgct atgtggcgcg gtattatccc gtattgacgc 1740
cgggcaagag caactcggtc gccgcataca ctattctcag aatgacttgg ttgagtactc 1800
accagtcaca gaaaagcatc ttacggatgg catgacagta agagaattat gcagtgctgc 1860
cataaccatg agtgataaca ctgcggccaa cttacttctg acaacgatcg gaggaccgaa 1920
ggagctaacc gcttttttgc acaacatggg ggatcatgta actcgccttg atcgttggga 1980
accggagctg aatgaagcca taccaaacga cgagcgtgac accacgatgc ctgcagcaat 2040
ggcaacaacg ttgcgcaaac tattaactgg cgaactactt actctagctt cccggcaaca 2100
attaatagac tggatggagg cggataaagt tgcaggacca cttctgcgct cggcccttcc 2160
ggctggctgg tttattgctg ataaatctgg agccggtgag cgtgggtctc gcggtatcat 2220
tgcagcactg gggccagatg gtaagccctc ccgtatcgta gttatctaca cgacggggag 2280
tcaggcaact atggatgaac gaaatagaca gatcgctgag ataggtgcct cactgattaa 2340
gcattggtaa ctgtcagacc aagtttactc atatatactt tagattgatt taaaacttca 2400
tttttaattt aaaaggatct aggtgaagat cctttttgat aatctcatga ccaaaatccc 2460
ttaacgtgag ttttcgttcc actgagcgtc agaccccgta gaaaagatca aaggatcttc 2520
ttgagatcct ttttttctgc gcgtaatctg ctgcttgcaa acaaaaaaac caccgctacc 2580
agcggtggtt tgtttgccgg atcaagagct accaactctt tttccgaagg taactggctt 2640
cagcagagcg cagataccaa atactgtcct tctagtgtag ccgtagttag gccaccactt 2700
caagaactct gtagcaccgc ctacatacct cgctctgcta atcctgttac cagtggctgc 2760
tgccagtggc gataagtcgt gtcttaccgg gttggactca agacgatagt taccggataa 2820
ggcgcagcgg tcgggctgaa cggggggttc gtgcacacag cccagcttgg agcgaacgac 2880
ctacaccgaa ctgagatacc tacagcgtga gctatgagaa agcgccacgc ttcccgaagg 2940
gagaaaggcg gacaggtatc cggtaagcgg cagggtcgga acaggagagc gcacgaggga 3000
gcttccaggg ggaaacgcct ggtatcttta tagtcctgtc gggtttcgcc acctctgact 3060
tgagcgtcga tttttgtgat gctcgtcagg ggggcggagc ctatggaaaa acgccagcaa 3120
cgcggccttt ttacggttcc tggccttttg ctggcctttt gctcacatgt tctttcctgc 3180
gttatcccct gattctgtgg ataaccgtat taccgccttt gagtgagctg ataccgctcg 3240
ccgcagccga acgaccgagc gcagcgagtc agtgagcgag gaagcggaag agcgcctgat 3300
gcggtatttt ctccttacgc atctgtgcgg tatttcacac cgcatatatg gtgcactctc 3360
agtacaatct gctctgatgc cgcatagtta agccagtata cactccgcta tcgctacgtg 3420
actgggtcat ggctgcgccc cgacacccgc caacacccgc tgacgcgccc tgacgggctt 3480

```
gtctgctccc ggcatccgct tacagacaag ctgtgaccgt ctccgggagc tgcatgtgtc 3540
agaggttttc accgtcatca ccgaaacgcg cgaggcagct gcggtaaagc tcatcagcgt 3600
ggtcgtgaag cgattcacag atgtctgcct gttcatccgc gtccagctcg ttgagtttct 3660
ccagaagcgt taatgtctgg cttctgataa agcgggccat gttaagggcg gtttttcct 3720
gtttggtcac tgatgcctcc gtgtaagggg gatttctgtt catgggggta atgataccga 3780
tgaaacgaga gaggatgctc acgatacggg ttactgatga tgaacatgcc cggttactgg 3840
aacgttgtga gggtaaacaa ctggcggtat ggatgcggcg ggaccagaga aaaatcactc 3900
agggtcaatg ccagcgcttc gttaatacag atgtaggtgt tccacagggt agccagcagc 3960
atcctgcgat gcagatccgg aacataatgg tgcagggcgc tgacttccgc gtttccagac 4020
tttacgaaac acggaaaccg aagaccattc atgttgttgc tcaggtcgca gacgttttgc 4080
agcagcagtc gcttcacgtt cgctcgcgta tcggtgattc attctgctaa ccagtaaggc 4140
aaccccgcca gcctagccgg gtcctcaacg acaggagcac gatcatgcgc acccgtgggg 4200
ccgccatgcc ggcgataatg gcctgcttct cgccgaaacg tttggtggcg ggaccagtga 4260
cgaaggcttg agcgagggcg tgcaagattc cgaataccgc aagcgacagg ccgatcatcg 4320
tcgcgctcca gcgaaagcgg tcctcgccga aaatgaccca gagcgctgcc ggcacctgtc 4380
ctacgagttg catgataaag aagacagtca taagtgcggc gacgatagtc atgccccgcg 4440
cccaccggaa ggagctgact gggttgaagg ctctcaaggg catcggtcga gatcccggtg 4500
cctaatgagt gagctaactt acattaattg cgttgcgctc actgcccgct ttccagtcgg 4560
gaaacctgtc gtgccagctg cattaatgaa tcggccaacg cgcggggaga ggcggtttgc 4620
gtattgggcg ccagggtggt ttttcttttc accagtgaga cgggcaacag ctgattgccc 4680
ttcaccgcct ggccctgaga gagttgcagc aagcggtcca cgctggtttg ccccagcagg 4740
cgaaaatcct gtttgatggt ggttaacggc gggatataac atgagctgtc ttcggtatcg 4800
tcgtatccca ctaccgagat atccgcacca acgcgcagcc cggactcggt aatggcgcgc 4860
attgcgccca gcgccatctg atcgttggca accagcatcg cagtgggaac gatgccctca 4920
ttcagcattt gcatggtttg ttgaaaaccg gacatggcac tccagtcgcc ttcccgttcc 4980
gctatcggct gaatttgatt gcgagtgaga tatttatgcc agccagccag acgcagacgc 5040
gccgagacag aacttaatgg gcccgctaac agcgcgattt gctggtgacc caatgcgacc 5100
agatgctcca cgcccagtcg cgtaccgtct tcatgggaga aaataatact gttgatgggt 5160
gtctggtcag agacatcaag aaataacgcc ggaacattag tgcaggcagc ttccacagca 5220
atggcatcct ggtcatccag cggatagtta atgatcagcc cactgacgcg ttgcgcgaga 5280
agattgtgca ccgccgcttt acaggcttcg acgccgcttc gttctaccat cgacaccacc 5340
acgctggcac ccagttgatc ggcgcgagat ttaatcgccg cgacaatttg cgacggcgcg 5400
tgcagggcca gactggaggt ggcaacgcca atcagcaacg actgtttgcc cgccagttgt 5460
tgtgccacgc ggttgggaat gtaattcagc tccgccatcg ccgcttccac tttttcccgc 5520
gttttcgcag aaacgtggct ggcctggttc accacgcggg aaacggtctg ataagagaca 5580
ccggcatact ctgcgacatc gtataacgtt actggtttca cattcaccac cctgaattga 5640
ctctcttccg ggcgctatca tgccataccg cgaaaggttt tgcgccattc gatggtgtcc 5700
gggatctcga cgctctccct tatgcgactc ctgcattagg aagcagccca gtagtaggtt 5760
gaggccgttg agcaccgccg ccgcaaggaa tggtgcatgc aaggagatgg cgcccaacag 5820
tcccccggcc acggggcctg ccaccatacc cacgccgaaa caagcgctca tgagcccgaa 5880
gtggcgagcc cgatcttccc catcggtgat gtcggcgata taggcgccag caaccgcacc 5940
tgtggcgccg gtgatgccgg ccacgatgcg tccggcgtag aggatcgaga tctcgatccc 6000
gcgaaattaa tacgactcac tataggggaa ttgtgagcgg ataacaattc ccctctagaa 6060
ataattttgt ttaactttaa gaaggagata tacatatgaa atacctgctg ccgaccgctg 6120
ctgctggtct gctgctcctc gctgcccagc cggcgatgg                        6159
```

<210> 19
<211> 6330
<212> DNA
<213> Artificial Sequence

<220>
<223> Description of the Artificial Sequence :Expression plasmid under the T7 promoter corresponding to a protein displaying variables regions of mAb 4G2 and a trimeric domain of matrilin

<220>
<223> Plasmid.

<400> 19

```
ccatggcaaa cattgtgatg acccaatctc ccaaatccat gtccatgtca gtaggagaga 60
gggtcacctt gacctgcaag gccagtgaga atgtggttac ttatgtttcc tggtatcaac 120
agaaaccaga gcagtctcct aaactgctga tatacggggc atccaaccgg tacactgggg 180
tccccgatcg cttcacaggc agtggatctg caacagattt cactctgacc atcagcagtg 240
tgcaggctga agaccttgca gattatcact gtggacaggg ttacagctat ccgtacacgt 300
tcggaggggg gaccaagctg gaaataaaag agggtaaatc ctcaggatca ggctccgaat 360
```

ccaaagtcga cgaggtccag ctgcaacaat ctggacctga gctggtgaag cctgggactt 420
cagtgaagat atcctgcaag acttctggat acacattcac tgaatatacc atacactggg 480
tgaagcagag ccacggaaag agccttgcgt ggattggagg tattgatcct aacagtggtg 540
gtactaacta cagcccgaac ttcaagggca aggccacatt gactgttgac aagtcctcca 600
gcacagccta catggacctc cgcagcctgt catctgagga ttctgcagtc tacttctgtg 660
caaggatcta tcattacgac ggatacttcg atgtctgggg cgcagggacc gccgtcaccg 720
tctcctcagg tggtggttcc ggtggtggtt ccggtggtgg ttccggtggt ggtgaagacc 780
cgtgcgcttg cgaatccctg gttaaattcc aggctaaagt tgaaggtctg ctgcaggctc 840
tgacccgtaa actggaagct gtttccaaac gtctggctat cctggaaaac accgttgttc 900
tcgagcacca ccaccaccac cactgagatc cggctgctaa caaagcccga aaggaagctg 960
agttggctgc tgccaccgct gagcaataac tagcataacc ccttggggcc tctaaacggg 1020
tcttgagggg ttttttgctg aaaggaggaa ctatatccgg attggcgaat gggacgcgcc 1080
ctgtagcggc gcattaagcg cggcgggtgt ggtggttacg cgcagcgtga ccgctacact 1140
tgccagcgcc ctagcgcccg ctcctttcgc tttcttccct tcctttctcg ccacgttcgc 1200
cggctttccc cgtcaagctc taaatcgggg gctcccttta gggttccgat ttagtgcttt 1260
acggcacctc gaccccaaaa aacttgatta gggtgatggt tcacgtagtg ggccatcgcc 1320
ctgatagacg gtttttcgcc ctttgacgtt ggagtccacg ttctttaata gtggactctt 1380
gttccaaact ggaacaacac tcaaccctat ctcggtctat tcttttgatt tataagggat 1440
tttgccgatt tcggcctatt ggttaaaaaa tgagctgatt taacaaaaat ttaacgcgaa 1500
ttttaacaaa atattaacgt ttacaatttc aggtggcact tttcggggaa atgtgcgcgg 1560
aacccctatt tgtttatttt tctaaataca ttcaaatatg tatccgctca tgagacaata 1620
accctgataa atgcttcaat aatattgaaa aaggaagagt atgagtattc aacatttccg 1680
tgtcgccctt attccctttt ttgcggcatt ttgccttcct gtttttgctc acccagaaac 1740
gctggtgaaa gtaaaagatg ctgaagatca gttgggtgca cgagtgggtt acatcgaact 1800
ggatctcaac agcggtaaga tccttgagag ttttcgcccc gaagaacgtt ttccaatgat 1860
gagcactttt aaagttctgc tatgtggcgc ggtattatcc cgtattgacg ccgggcaaga 1920
gcaactcggt cgccgcatac actattctca gaatgacttg gttgagtact caccagtcac 1980
agaaaagcat cttacggatg gcatgacagt aagagaatta tgcagtgctg ccataaccat 2040
gagtgataac actgcggcca acttacttct gacaacgatc ggaggaccga aggagctaac 2100
cgcttttttg cacaacatgg gggatcatgt aactcgcctt gatcgttggg aaccggagct 2160
gaatgaagcc ataccaaacg acgagcgtga caccacgatg cctgcagcaa tggcaacaac 2220
gttgcgcaaa ctattaactg gcgaactact tactctagct tcccggcaac aattaataga 2280
ctggatggag gcggataaag ttgcaggacc acttctgcgc tcggcccttc cggctggctg 2340
gtttattgct gataaatctg gagccggtga gcgtgggtct cgcggtatca ttgcagcact 2400
ggggccagat ggtaagccct cccgtatcgt agttatctac acgacgggga gtcaggcaac 2460
tatggatgaa cgaaatagac agatcgctga gataggtgcc tcactgatta agcattggta 2520
actgtcagac caagtttact catatatact ttagattgat ttaaaacttc atttttaatt 2580
taaaaggatc taggtgaaga tcctttttga taatctcatg accaaaatcc cttaacgtga 2640
gttttcgttc cactgagcgt cagaccccgt agaaaagatc aaaggatctt cttgagatcc 2700
tttttttctg cgcgtaatct gctgcttgca aacaaaaaaa ccaccgctac cagcggtggt 2760
ttgtttgccg gatcaagagc taccaactct ttttccgaag gtaactggct tcagcagagc 2820
gcagatacca aatactgtcc ttctagtgta gccgtagtta ggccaccact tcaagaactc 2880
tgtagcaccg cctacatacc tcgctctgct aatcctgtta ccagtggctg ctgccagtgg 2940
cgataagtcg tgtcttaccg ggttggactc aagacgatag ttaccggata aggcgcagcg 3000
gtcgggctga acggggggtt cgtgcacaca gcccagcttg gagcgaacga cctacaccga 3060
actgagatac ctacagcgtg agctatgaga aagcgccacg cttcccgaag ggagaaaggc 3120
ggacaggtat ccggtaagcg gcagggtcgg aacaggagag cgcacgaggg agcttccagg 3180
gggaaacgcc tggtatcttt atagtcctgt cgggtttcgc cacctctgac ttgagcgtcg 3240
atttttgtga tgctcgtcag gggggcggag cctatggaaa aacgccagca acgcggcctt 3300
tttacggttc ctggcctttt gctggccttt tgctcacatg ttctttcctg cgttatcccc 3360
tgattctgtg gataaccgta ttaccgcctt tgagtgagct gataccgctc gccgcagccg 3420
aacgaccgag cgcagcgagt cagtgagcga ggaagcggaa gagcgcctga tgcggtattt 3480
tctccttacg catctgtgcg gtatttcaca ccgcatatat ggtgcactct cagtacaatc 3540
tgctctgatg ccgcatagtt aagccagtat acactccgct atcgctacgt gactgggtca 3600
tggctgcgcc ccgacacccg ccaacacccg ctgacgcgcc ctgacgggct tgtctgctcc 3660
cggcatccgc ttacagacaa gctgtgaccg tctccgggag ctgcatgtgt cagaggtttt 3720
caccgtcatc accgaaacgc gcgaggcagc tgcggtaaag ctcatcagcg tggtcgtgaa 3780
gcgattcaca gatgtctgcc tgttcatccg cgtccagctc gttgagtttc tccagaagcg 3840
ttaatgtctg gcttctgata aagcgggcca tgttaagggc ggttttttcc tgtttggtca 3900
ctgatgcctc cgtgtaaggg ggatttctgt tcatgggggt aatgataccg atgaaacgag 3960
agaggatgct cacgatacgg gttactgatg atgaacatgc ccggttactg gaacgttgtg 4020
agggtaaaca actggcggta tggatgcggc gggaccagag aaaaatcact cagggtcaat 4080
gccagcgctt cgttaataca gatgtaggtg ttccacaggg tagccagcag catcctgcga 4140
tgcagatccg gaacataatg gtgcagggcg ctgacttccg cgtttccaga ctttacgaaa 4200
cacggaaacc gaagaccatt catgttgttg ctcaggtcgc agacgttttg cagcagcagt 4260
cgcttcacgt tcgctcgcgt atcggtgatt cattctgcta accagtaagg caaccccgcc 4320
agcctagccg ggtcctcaac gacaggagca cgatcatgcg cacccgtggg gccgccatgc 4380
cggcgataat ggcctgcttc tcgccgaaac gtttggtggc gggaccagtg acgaaggctt 4440
gagcgagggc gtgcaagatt ccgaataccg caagcgacag gccgatcatc gtcgcgctcc 4500

agcgaaagcg gtcctcgccg aaaatgaccc agagcgctgc cggcacctgt cctacgagtt 4560
gcatgataaa gaagacagtc ataagtgcgg cgacgatagt catgccccgc gcccaccgga 4620
aggagctgac tgggttgaag gctctcaagg gcatcggtcg agatcccggt gcctaatgag 4680
tgagctaact tacattaatt gcgttgcgct cactgcccgc tttccagtcg ggaaacctgt 4740
cgtgccagct gcattaatga atcggccaac gcgcggggag aggcggtttg cgtattgggc 4800
gccagggtgg tttttctttt caccagtgag acgggcaaca gctgattgcc cttcaccgcc 4860
tggccctgag agagttgcag caagcggtcc acgctggttt gccccagcag gcgaaaatcc 4920
tgtttgatgg tggttaacgg cgggatataa catgagctgt cttcggtatc gtcgtatccc 4980
actaccgaga tatccgcacc aacgcgcagc ccggactcgg taatggcgcg cattgcgccc 5040
agcgccatct gatcgttggc aaccagcatc gcagtgggaa cgatgccctc attcagcatt 5100
tgcatggttt gttgaaaacc ggacatggca ctccagtcgc cttcccgttc cgctatcggc 5160
tgaatttgat tgcgagtgag atatttatgc cagccagcca gacgcagacg cgccgagaca 5220
gaacttaatg ggcccgctaa cagcgcgatt tgctggtgac ccaatgcgac cagatgctcc 5280
acgcccagtc gcgtaccgtc ttcatgggag aaaataatac tgttgatggg tgtctggtca 5340
gagacatcaa gaaataacgc cggaacatta gtgcaggcag cttccacagc aatggcatcc 5400
tggtcatcca gcggatagtt aatgatcagc ccactgacgc gttgcgcgag aagattgtgc 5460
accgccgctt tacaggcttc gacgccgctt cgttctacca tcgacaccac cacgctggca 5520
cccagttgat cggcgcgaga tttaatcgcc gcgacaattt gcgacggcgc gtgcagggcc 5580
agactggagg tggcaacgcc aatcagcaac gactgtttgc ccgccagttg ttgtgccacg 5640
cggttgggaa tgtaattcag ctccgccatc gccgcttcca ctttttcccg cgttttcgca 5700
gaaacgtggc tggcctggtt caccacgcgg gaaacggtct gataagagac accggcatac 5760
tctgcgacat cgtataacgt tactggtttc acattcacca ccctgaattg actctcttcc 5820
gggcgctatc atgccatacc gcgaaaggtt ttgcgccatt cgatggtgtc cgggatctcg 5880
acgctctccc ttatgcgact cctgcattag gaagcagccc agtagtaggt tgaggccgtt 5940
gagcaccgcc gccgcaagga atggtgcatg caaggagatg gcgcccaaca gtcccccggc 6000
cacggggcct gccaccatac ccacgccgaa acaagcgctc atgagcccga agtggcgagc 6060
ccgatcttcc ccatcggtga tgtcggcgat ataggcgcca gcaaccgcac ctgtggcgcc 6120
ggtgatgccg gccacgatgc gtccggcgta gaggatcgag atctcgatcc cgcgaaatta 6180
atacgactca ctatagggga attgtgagcg gataacaatt cccctctaga aataattttg 6240
tttaacttta agaaggagat atacatatga aatacctgct gccgaccgct gctgctggtc 6300
tgctgctcct cgctgcccag ccggcgatgg 6330

<210> 20
<211> 6864
<212> DNA
<213> Artificial Sequence

<220>
<223> Description of the Artificial Sequence : Expression plasmid under the T7 promoter corresponding to a single chain antibody fragment displaying the variables regions of mAb 4G2

<220>
<223> Plasmid.

<400> 20
catggcaaac attgtgatga cccaatctcc caaatccatg tccatgtcag taggagagag 60
ggtcaccttg acctgcaagg ccagtgagaa tgtggttact tatgtttcct ggtatcaaca 120
gaaaccagag cagtctccta aactgctgat atacggggca tccaaccggt acactggggt 180
ccccgatcgc ttcacaggca gtggatctgc aacagatttc actctgacca tcagcagtgt 240
gcaggctgaa gaccttgcag attatcactg tggacagggt tacagctatc cgtacacgtt 300
cggagggggg accaagctgg aaataaaaga gggtaaatcc tcaggatcag gctccgaatc 360
caaagtcgac gaggtccagc tgcaacaatc tggacctgag ctggtgaagc ctgggacttc 420
agtgaagata tcctgcaaga cttctggata cacattcact gaatatacca tacactgggt 480
gaagcagagc cacggaaaga gccttgcgtg gattggaggt attgatccta acagtggtgg 540
tactaactac agcccgaact tcaagggcaa ggccacattg actgttgaca agtcctccag 600
cacagcctac atggacctcc gcagcctgtc atctgaggat tctgcagtct acttctgtgc 660
aaggatctat cattacgacg gatacttcga tgtctggggc gcagggaccg ccgtcaccgt 720
ctcctcaggc ggtggcgagc ccaaatcttc tgacaaaact cacacatgcc caccgtgccc 780
agcacctgaa ctcctggggg gaccgtcagt cttcctcttc cccccaaaac ccaaggacac 840
cctcatgatc tcccggaccc ctgaggtcac atgcgtggtg gtggacgtga gccacgaaga 900
ccctgaggtc aagttcaact ggtacgtgga cggcgtggag gtgcataatg ccaagacaaa 960
gccgcgggag gagcagtacc agagcacgta ccgtgtggtc agcgtcctca ccgtcctgca 1020
ccaggactgg ctgaatggca aggagtacaa gtgcaaggtc tccaacaaag ccctcccagc 1080
ccccatcgag aaaaccatct ccaaagccaa agggcagccc cgagaaccac aggtgtacac 1140
cctgccccca tcccgggagg agatgaccaa gaaccaggtc agcctgacct gcctggtcaa 1200
aggcttctat cccagcgaca tcgccgtgga gtgggagagc aatgggcagc cggagaacaa 1260

```
ctacaagacc acgcctcccg tgctggactc cgacggctcc ttcttcctct atagcaagct 1320
caccgtggac aagagcaggt ggcagcaggg gaacgtcttc tcatgctccg tgatgcatga 1380
ggctctgcac aaccactaca cgcagaagag cctctccctg tccccgggta aactcgagca 1440
ccaccaccac caccactgag atccggctgc taacaaagcc cgaaaggaag ctgagttggc 1500
tgctgccacc gctgagcaat aactagcata accccttggg gcctctaaac gggtcttgag 1560
gggttttttg ctgaaaggag gaactatatc cggattggcg aatgggacgc gccctgtagc 1620
ggcgcattaa gcgcggcggg tgtggtggtt acgcgcagcg tgaccgctac acttgccagc 1680
gccctagcgc ccgctccttt cgctttcttc ccttcctttc tcgccacgtt cgccggcttt 1740
ccccgtcaag ctctaaatcg ggggctccct ttagggttcc gatttagtgc tttacggcac 1800
ctcgacccca aaaaacttga ttagggtgat ggttcacgta gtgggccatc gccctgatag 1860
acggtttttc gccctttgac gttggagtcc acgttcttta atagtggact cttgttccaa 1920
actggaacaa cactcaaccc tatctcggtc tattcttttg atttataagg gattttgccg 1980
atttcggcct attggttaaa aaatgagctg atttaacaaa aatttaacgc gaattttaac 2040
aaaatattaa cgtttacaat ttcaggtggc acttttcggg gaaatgtgcg cggaacccct 2100
atttgtttat ttttctaaat acattcaaat atgtatccgc tcatgagaca ataaccctga 2160
taaatgcttc aataatattg aaaaaggaag agtatgagta ttcaacattt ccgtgtcgcc 2220
cttattccct tttttgcggc attttgcctt cctgtttttg ctcacccaga aacgctggtg 2280
aaagtaaaag atgctgaaga tcagttgggt gcacgagtgg gttacatcga actggatctc 2340
aacagcggta agatccttga gagttttcgc cccgaagaac gttttccaat gatgagcact 2400
tttaaagttc tgctatgtgg cgcggtatta tcccgtattg acgccgggca agagcaactc 2460
ggtcgccgca tacactattc tcagaatgac ttggttgagt actcaccagt cacagaaaag 2520
catcttacgg atggcatgac agtaagagaa ttatgcagtg ctgccataac catgagtgat 2580
aacactgcgg ccaacttact tctgacaacg atcggaggac cgaaggagct aaccgctttt 2640
ttgcacaaca tgggggatca tgtaactcgc cttgatcgtt gggaaccgga gctgaatgaa 2700
gccataccaa acgacgagcg tgacaccacg atgcctgcag caatggcaac aacgttgcgc 2760
aaactattaa ctggcgaact acttactcta gcttcccggc aacaattaat agactggatg 2820
gaggcggata aagttgcagg accacttctg cgctcggccc ttccggctgg ctggtttatt 2880
gctgataaat ctggagccgg tgagcgtggg tctcgcggta tcattgcagc actggggcca 2940
gatggtaagc cctcccgtat cgtagttatc tacacgacgg ggagtcaggc aactatggat 3000
gaacgaaata gacagatcgc tgagataggt gcctcactga ttaagcattg gtaactgtca 3060
gaccaagttt actcatatat actttagatt gatttaaaac ttcattttta atttaaaagg 3120
atctaggtga agatcctttt tgataatctc atgaccaaaa tcccttaacg tgagttttcg 3180
ttccactgag cgtcagaccc cgtagaaaag atcaaaggat cttcttgaga tccttttttt 3240
ctgcgcgtaa tctgctgctt gcaaacaaaa aaaccaccgc taccagcggt ggtttgtttg 3300
ccggatcaag agctaccaac tctttttccg aaggtaactg gcttcagcag agcgcagata 3360
ccaaatactg tccttctagt gtagccgtag ttaggccacc acttcaagaa ctctgtagca 3420
ccgcctacat acctcgctct gctaatcctg ttaccagtgg ctgctgccag tggcgataag 3480
tcgtgtctta ccgggttgga ctcaagacga tagttaccgg ataaggcgca gcggtcgggc 3540
tgaacggggg gttcgtgcac acagcccagc ttggagcgaa cgacctacac cgaactgaga 3600
tacctacagc gtgagctatg agaaagcgcc acgcttcccg aagggagaaa ggcggacagg 3660
tatccggtaa gcggcagggt cggaacagga gagcgcacga gggagcttcc agggggaaac 3720
gcctggtatc tttatagtcc tgtcgggttt cgccacctct gacttgagcg tcgatttttg 3780
tgatgctcgt caggggggcg gagcctatgg aaaaacgcca gcaacgcggc ctttttacgg 3840
ttcctggcct tttgctggcc ttttgctcac atgttctttc ctgcgttatc ccctgattct 3900
gtggataacc gtattaccgc ctttgagtga gctgataccg ctcgccgcag ccgaacgacc 3960
gagcgcagcg agtcagtgag cgaggaagcg gaagagcgcc tgatgcggta ttttctcctt 4020
acgcatctgt gcggtatttc acaccgcata tatggtgcac tctcagtaca atctgctctg 4080
atgccgcata gttaagccag tatacactcc gctatcgcta cgtgactggg tcatggctgc 4140
gccccgacac ccgccaacac ccgctgacgc gccctgacgg gcttgtctgc tcccggcatc 4200
cgcttacaga caagctgtga ccgtctccgg gagctgcatg tgtcagaggt tttcaccgtc 4260
atcaccgaaa cgcgcgaggc agctgcggta aagctcatca gcgtggtcgt gaagcgattc 4320
acagatgtct gcctgttcat ccgcgtccag ctcgttgagt ttctccagaa gcgttaatgt 4380
ctggcttctg ataaagcggg ccatgttaag ggcggttttt tcctgtttgg tcactgatgc 4440
ctccgtgtaa gggggatttc tgttcatggg ggtaatgata ccgatgaaac gagagaggat 4500
gctcacgata cgggttactg atgatgaaca tgcccggtta ctggaacgtt gtgagggtaa 4560
acaactggcg gtatggatgc ggcgggacca gagaaaaatc actcagggtc aatgccagcg 4620
cttcgttaat acagatgtag gtgttccaca gggtagccag cagcatcctg cgatgcagat 4680
ccggaacata atggtgcagg gcgctgactt ccgcgtttcc agactttacg aaacacggaa 4740
accgaagacc attcatgttg ttgctcaggt cgcagacgtt ttgcagcagc agtcgcttca 4800
cgttcgctcg cgtatcggtg attcattctg ctaaccagta aggcaacccc gccagcctag 4860
ccgggtcctc aacgacagga gcacgatcat gcgcacccgt ggggccgcca tgccggcgat 4920
aatggcctgc ttctcgccga aacgtttggt ggcgggacca gtgacgaagg cttgagcgag 4980
ggcgtgcaag attccgaata ccgcaagcga caggccgatc atcgtcgcgc tccagcgaaa 5040
gcggtcctcg ccgaaaatga cccagagcgc tgccggcacc tgtcctacga gttgcatgat 5100
aaagaagaca gtcataagtg cggcgacgat agtcatgccc cgcgcccacc ggaaggagct 5160
gactgggttg aaggctctca agggcatcgg tcgagatccc ggtgcctaat gagtgagcta 5220
acttacatta attgcgttgc gctcactgcc cgctttccag tcgggaaacc tgtcgtgcca 5280
gctgcattaa tgaatcggcc aacgcgcggg gagaggcggt ttgcgtattg ggcgccaggg 5340
tggtttttct tttcaccagt gagacgggca acagctgatt gcccttcacc gcctggccct 5400
```

```
gagagagttg cagcaagcgg tccacgctgg tttgccccag caggcgaaaa tcctgtttga 5460
tggtggttaa cggcgggata taacatgagc tgtcttcggt atcgtcgtat cccactaccg 5520
agatatccgc accaacgcgc agcccggact cggtaatggc gcgcattgcg cccagcgcca 5580
tctgatcgtt ggcaaccagc atcgcagtgg gaacgatgcc ctcattcagc atttgcatgg 5640
tttgttgaaa accggacatg gcactccagt cgccttcccg ttccgctatc ggctgaattt 5700
gattgcgagt gagatattta tgccagccag ccagacgcag acgcgccgag acagaactta 5760
atgggcccgc taacagcgcg atttgctggt gacccaatgc gaccagatgc tccacgccca 5820
gtcgcgtacc gtcttcatgg gagaaaataa tactgttgat gggtgtctgg tcagagacat 5880
caagaaataa cgccggaaca ttagtgcagg cagcttccac agcaatggca tcctggtcat 5940
ccagcggata gttaatgatc agcccactga cgcgttgcgc gagaagattg tgcaccgccg 6000
ctttacaggc ttcgacgccg cttcgttcta ccatcgacac caccacgctg gcacccagtt 6060
gatcggcgcg agatttaatc gccgcgacaa tttgcgacgg cgcgtgcagg gccagactgg 6120
aggtggcaac gccaatcagc aacgactgtt tgcccgccag ttgttgtgcc acgcggttgg 6180
gaatgtaatt cagctccgcc atcgccgctt ccactttttc ccgcgttttc gcagaaacgt 6240
ggctggcctg gttcaccacg cgggaaacgg tctgataaga gacaccggca tactctgcga 6300
catcgtataa cgttactggt ttcacattca ccaccctgaa ttgactctct tccgggcgct 6360
atcatgccat accgcgaaag gttttgcgcc attcgatggt gtccgggatc tcgacgctct 6420
cccttatgcg actcctgcat taggaagcag cccagtagta ggttgaggcc gttgagcacc 6480
gccgccgcaa ggaatggtgc atgcaaggag atggcgccca acagtccccc ggccacgggg 6540
cctgccacca tacccacgcc gaaacaagcg ctcatgagcc cgaagtggcg agcccgatct 6600
tccccatcgg tgatgtcggc gatataggcg ccagcaaccg cacctgtggc gccggtgatg 6660
ccggccacga tgcgtccggc gtagaggatc gagatctcga tcccgcgaaa ttaatacgac 6720
tcactatagg ggaattgtga gcggataaca attcccctct agaaataatt ttgtttaact 6780
ttaagaagga gatatacata tgaaatacct gctgccgacc gctgctgctg gtctgctgct 6840
cctcgctgcc cagccggcga tggc                                        6864
```

```
<210> 21
<211> 272
<212> PRT
<213> Artificial Sequence

<220>
<223> Description of the Artificial Sequence: Single chain
      antibody fragment displaying the variable regions
      of the mAb 4G2

<220>
<223> Protein.

<400> 21
Met Lys Tyr Leu Leu Pro Thr Ala Ala Ala Gly Leu Leu Leu Leu Ala
  1               5                  10                  15

Ala Gln Pro Ala Met Ala Met Ala Asn Ile Val Met Thr Gln Ser Pro
             20                  25                  30

Lys Ser Met Ser Met Ser Val Gly Glu Arg Val Thr Leu Thr Cys Lys
         35                  40                  45

Ala Ser Glu Asn Val Val Thr Tyr Val Ser Trp Tyr Gln Gln Lys Pro
     50                  55                  60

Glu Gln Ser Pro Lys Leu Leu Ile Tyr Gly Ala Ser Asn Arg Tyr Thr
 65                  70                  75                  80

Gly Val Pro Asp Arg Phe Thr Gly Ser Gly Ser Ala Thr Asp Phe Thr
                 85                  90                  95

Leu Thr Ile Ser Ser Val Gln Ala Glu Asp Leu Ala Asp Tyr His Cys
            100                 105                 110

Gly Gln Gly Tyr Ser Tyr Pro Tyr Thr Phe Gly Gly Gly Thr Lys Leu
        115                 120                 125

Glu Ile Lys Glu Gly Lys Ser Ser Gly Ser Gly Ser Glu Ser Lys Val
    130                 135                 140

Asp Glu Val Gln Leu Gln Gln Ser Gly Pro Glu Leu Val Lys Pro Gly
```

145 150 155 160

Thr Ser Val Lys Ile Ser Cys Lys Thr Ser Gly Tyr Thr Phe Thr Glu
165 170 175

Tyr Thr Ile His Trp Val Lys Gln Ser His Gly Lys Ser Leu Ala Trp
180 185 190

Ile Gly Gly Ile Asp Pro Asn Ser Gly Gly Thr Asn Tyr Ser Pro Asn
195 200 205

Phe Lys Gly Lys Ala Thr Leu Thr Val Asp Lys Ser Ser Ser Thr Ala
210 215 220

Tyr Met Asp Leu Arg Ser Leu Ser Ser Glu Asp Ser Ala Val Tyr Phe
225 230 235 240

Cys Ala Arg Ile Tyr His Tyr Asp Gly Tyr Phe Asp Val Trp Gly Ala
245 250 255

Gly Thr Ala Val Thr Val Ser Ser Leu Glu His His His His His His
260 265 270

<210> 22
<211> 329
<212> PRT
<213> Artificial Sequence

<220>
<223> Description of the Artificial Sequence : Protein displaying the variable regions of the mAb 4G2 and a trimeric domain of matrilin

<220>
<223> Protein.

<400> 22
Met Lys Tyr Leu Leu Pro Thr Ala Ala Ala Gly Leu Leu Leu Leu Ala
1 5 10 15

Ala Gln Pro Ala Met Ala Met Ala Asn Ile Val Met Thr Gln Ser Pro
20 25 30

Lys Ser Met Ser Met Ser Val Gly Glu Arg Val Thr Leu Thr Cys Lys
35 40 45

Ala Ser Glu Asn Val Val Thr Tyr Val Ser Trp Tyr Gln Gln Lys Pro
50 55 60

Glu Gln Ser Pro Lys Leu Leu Ile Tyr Gly Ala Ser Asn Arg Tyr Thr
65 70 75 80

Gly Val Pro Asp Arg Phe Thr Gly Ser Gly Ser Ala Thr Asp Phe Thr
85 90 95

Leu Thr Ile Ser Ser Val Gln Ala Glu Asp Leu Ala Asp Tyr His Cys
100 105 110

Gly Gln Gly Tyr Ser Tyr Pro Tyr Thr Phe Gly Gly Gly Thr Lys Leu
115 120 125

Glu Ile Lys Glu Gly Lys Ser Ser Gly Ser Gly Ser Glu Ser Lys Val
130 135 140

Asp Glu Val Gln Leu Gln Gln Ser Gly Pro Glu Leu Val Lys Pro Gly

```
145                 150                 155                 160

Thr Ser Val Lys Ile Ser Cys Lys Thr Ser Gly Tyr Thr Phe Thr Glu
                165                 170                 175

Tyr Thr Ile His Trp Val Lys Gln Ser His Gly Lys Ser Leu Ala Trp
            180                 185                 190

Ile Gly Gly Ile Asp Pro Asn Ser Gly Gly Thr Asn Tyr Ser Pro Asn
        195                 200                 205

Phe Lys Gly Lys Ala Thr Leu Thr Val Asp Lys Ser Ser Ser Thr Ala
    210                 215                 220

Tyr Met Asp Leu Arg Ser Leu Ser Ser Glu Asp Ser Ala Val Tyr Phe
225                 230                 235                 240

Cys Ala Arg Ile Tyr His Tyr Asp Gly Tyr Phe Asp Val Trp Gly Ala
                245                 250                 255

Gly Thr Ala Val Thr Val Ser Ser Gly Gly Gly Ser Gly Gly Gly Ser
            260                 265                 270

Gly Gly Gly Ser Gly Gly Gly Glu Asp Pro Cys Ala Cys Glu Ser Leu
        275                 280                 285

Val Lys Phe Gln Ala Lys Val Glu Gly Leu Leu Gln Ala Leu Thr Arg
    290                 295                 300

Lys Leu Glu Ala Val Ser Lys Arg Leu Ala Ile Leu Glu Asn Thr Val
305                 310                 315                 320

Val Leu Glu His His His His His His
                325


<210> 23
<211> 507
<212> PRT
<213> Artificial Sequence

<220>
<223> Description of the Artificial Sequence:
      single chain miniantibody displaying
      the variable regions of mAb 4G2

<220>
<223> Protein.

<400> 23
Met Lys Tyr Leu Leu Pro Thr Ala Ala Ala Gly Leu Leu Leu Leu Ala
  1               5                  10                  15

Ala Gln Pro Ala Met Ala Met Ala Asn Ile Val Met Thr Gln Ser Pro
            20                  25                  30

Lys Ser Met Ser Met Ser Val Gly Glu Arg Val Thr Leu Thr Cys Lys
        35                  40                  45

Ala Ser Glu Asn Val Val Thr Tyr Val Ser Trp Tyr Gln Gln Lys Pro
    50                  55                  60

Glu Gln Ser Pro Lys Leu Leu Ile Tyr Gly Ala Ser Asn Arg Tyr Thr
 65                  70                  75                  80

Gly Val Pro Asp Arg Phe Thr Gly Ser Gly Ser Ala Thr Asp Phe Thr
                 85                  90                  95

Leu Thr Ile Ser Ser Val Gln Ala Glu Asp Leu Ala Asp Tyr His Cys
```

100 105 110

Gly Gln Gly Tyr Ser Tyr Pro Tyr Thr Phe Gly Gly Gly Thr Lys Leu
115 120 125

Glu Ile Lys Glu Gly Lys Ser Ser Gly Ser Gly Ser Glu Ser Lys Val
130 135 140

Asp Glu Val Gln Leu Gln Gln Ser Gly Pro Glu Leu Val Lys Pro Gly
145 150 155 160

Thr Ser Val Lys Ile Ser Cys Lys Thr Ser Gly Tyr Thr Phe Thr Glu
165 170 175

Tyr Thr Ile His Trp Val Lys Gln Ser His Gly Lys Ser Leu Ala Trp
180 185 190

Ile Gly Gly Ile Asp Pro Asn Ser Gly Gly Thr Asn Tyr Ser Pro Asn
195 200 205

Phe Lys Gly Lys Ala Thr Leu Thr Val Asp Lys Ser Ser Ser Thr Ala
210 215 220

Tyr Met Asp Leu Arg Ser Leu Ser Ser Glu Asp Ser Ala Val Tyr Phe
225 230 235 240

Cys Ala Arg Ile Tyr His Tyr Asp Gly Tyr Phe Asp Val Trp Gly Ala
245 250 255

Gly Thr Ala Val Thr Val Ser Ser Gly Gly Gly Glu Pro Lys Ser Ser
260 265 270

Asp Lys Thr His Thr Cys Pro Pro Cys Pro Ala Pro Glu Leu Leu Gly
275 280 285

Gly Pro Ser Val Phe Leu Phe Pro Pro Lys Pro Lys Asp Thr Leu Met
290 295 300

Ile Ser Arg Thr Pro Glu Val Thr Cys Val Val Val Asp Val Ser His
305 310 315 320

Glu Asp Pro Glu Val Lys Phe Asn Trp Tyr Val Asp Gly Val Glu Val
325 330 335

His Asn Ala Lys Thr Lys Pro Arg Glu Glu Gln Tyr Gln Ser Thr Tyr
340 345 350

Arg Val Val Ser Val Leu Thr Val Leu His Gln Asp Trp Leu Asn Gly
355 360 365

Lys Glu Tyr Lys Cys Lys Val Ser Asn Lys Ala Leu Pro Ala Pro Ile
370 375 380

Glu Lys Thr Ile Ser Lys Ala Lys Gly Gln Pro Arg Glu Pro Gln Val
385 390 395 400

Tyr Thr Leu Pro Pro Ser Arg Glu Glu Met Thr Lys Asn Gln Val Ser
405 410 415

Leu Thr Cys Leu Val Lys Gly Phe Tyr Pro Ser Asp Ile Ala Val Glu
420 425 430

Trp Glu Ser Asn Gly Gln Pro Glu Asn Asn Tyr Lys Thr Thr Pro Pro
435 440 445

Val Leu Asp Ser Asp Gly Ser Phe Phe Leu Tyr Ser Lys Leu Thr Val
450 455 460

Asp Lys Ser Arg Trp Gln Gln Gly Asn Val Phe Ser Cys Ser Val Met

```
465                 470                 475                 480

His Glu Ala Leu His Asn His Tyr Thr Gln Lys Ser Leu Ser Leu Ser
                485                 490                 495

Pro Gly Lys Leu Glu His His His His His His
            500                 505


<210> 24
<211> 22
<212> PRT
<213> Erwinia carotovora

<220>
<223> Signal peptide.

<220>
<223> pelB signal peptide

<400> 24
Met Lys Tyr Leu Leu Pro Thr Ala Ala Ala Gly Leu Leu Leu Leu Ala
  1               5                  10                  15

Ala Gln Pro Ala Met Ala
             20


<210> 25
<211> 107
<212> PRT
<213> Mus musculus

<220>
<223> Polypeptide.

<220>
<223> Sequence of the variable region of the light
      chain of mAb 4G2.

<400> 25
Asn Ile Val Met Thr Gln Ser Pro Lys Ser Met Ser Met Ser Val Gly
  1               5                  10                  15

Glu Arg Val Thr Leu Thr Cys Lys Ala Ser Glu Asn Val Val Thr Tyr
             20                  25                  30

Val Ser Trp Tyr Gln Gln Lys Pro Glu Gln Ser Pro Lys Leu Leu Ile
         35                  40                  45

Tyr Gly Ala Ser Asn Arg Tyr Thr Gly Val Pro Asp Arg Phe Thr Gly
     50                  55                  60

Ser Gly Ser Ala Thr Asp Phe Thr Leu Thr Ile Ser Ser Val Gln Ala
 65                  70                  75                  80

Glu Asp Leu Ala Asp Tyr His Cys Gly Gln Gly Tyr Ser Tyr Pro Tyr
                 85                  90                  95

Thr Phe Gly Gly Gly Thr Lys Leu Glu Ile Lys
            100                 105


<210> 26
<211> 14
<212> PRT
<213> Artificial Sequence

<220>
```

```
<223> Description of the Artificial Sequence :Sequence
      of the linker connecting the variable regions
      of the scFv fragment

<220>
<223> Péptido.

<400> 26
Glu Gly Lys Ser Ser Gly Ser Gly Ser Glu Ser Lys Val Asp
  1               5                  10


<210> 27
<211> 119
<212> PRT
<213> Mus musculus

<220>
<223> Polypeptide.

<220>
<223> Sequence of the variable region of the heavy chain
      of mAb 4G2.

<400> 27
Glu Val Gln Leu Gln Gln Ser Gly Pro Glu Leu Val Lys Pro Gly Thr
  1               5                  10                  15

Ser Val Lys Ile Ser Cys Lys Thr Ser Gly Tyr Thr Phe Thr Glu Tyr
             20                  25                  30

Thr Ile His Trp Val Lys Gln Ser His Gly Lys Ser Leu Ala Trp Ile
         35                  40                  45

Gly Gly Ile Asp Pro Asn Ser Gly Gly Thr Asn Tyr Ser Pro Asn Phe
     50                  55                  60

Lys Gly Lys Ala Thr Leu Thr Val Asp Lys Ser Ser Ser Thr Ala Tyr
 65                  70                  75                  80

Met Asp Leu Arg Ser Leu Ser Ser Glu Asp Ser Ala Val Tyr Phe Cys
                 85                  90                  95

Ala Arg Ile Tyr His Tyr Asp Gly Tyr Phe Asp Val Trp Gly Ala Gly
            100                 105                 110

Thr Ala Val Thr Val Ser Ser
        115


<210> 28
<211> 15
<212> PRT
<213> Artificial Sequence

<220>
<223> Description of the Artificial Sequence :Sequence
      of the linker segment connecting the matrilin
      domain to the variable domain of heavy of the
      mAb 4G2

<220>
<223> Peptide fragment.

<400> 28
Gly Gly Gly Ser Gly Gly Gly Ser Gly Gly Gly Ser Gly Gly Gly
  1               5                  10                  15
```

<210> 29
<211> 75
<212> PRT
<213> Artificial Sequence

<220>
<223> Description of the Artificial Sequence :This is the mature protein PMEC displaying segments B and C from dengue2 virus

<220>
<223> Protein.

<400> 29
Leu Val Thr Phe Lys Asn Pro His Ala Lys Lys Gln Asp Val Val Val
1 5 10 15

Gly Gly Lys Leu Thr Asn Thr Thr Thr Glu Ser Arg Cys Pro Thr Gln
20 25 30

Gly Glu Pro Ser Leu Asn Glu Glu Gln Asp Lys Arg Phe Leu Cys Lys
35 40 45

His Ser Met Val Asp Arg Gly Trp Gly Asn Gly Cys Gly Leu Phe Gly
50 55 60

Lys Gly Gly Ile Val Thr Cys Ala Met Phe Thr
65 70 75

<210> 30
<211> 75
<212> PRT
<213> Artificial Sequence

<220>
<223> Description of the Artificial Sequence : This is the mature protein PMEC displaying segments B and C from dengue1 virus

<220>
<223> Protein.

<400> 30
Leu Val Thr Phe Lys Thr Ala His Ala Lys Lys Gln Glu Val Val Val
1 5 10 15

Gly Gly Lys Ile Ser Asn Thr Thr Thr Asp Ser Arg Cys Pro Thr Gln
20 25 30

Gly Glu Ala Thr Leu Val Glu Glu Gln Asp Thr Asn Phe Val Cys Arg
35 40 45

Arg Thr Phe Val Asp Arg Gly Trp Gly Asn Gly Cys Gly Leu Phe Gly
50 55 60

Lys Gly Ser Leu Ile Thr Cys Ala Lys Phe Lys
65 70 75

<210> 31
<211> 75
<212> PRT
<213> Artificial Sequence

<220>
<223> Description of the Artificial Sequence : This is the mature protein PMEC displaying segments B and C from dengue3 virus

<220>
<223> Protein.

<400> 31
Leu Val Thr Phe Lys Asn Ala His Ala Lys Lys Gln Glu Val Val Val
1 5 10 15

Gly Gly Lys Ile Thr Asn Ile Thr Thr Asp Ser Arg Cys Pro Thr Gln
20 25 30

Gly Glu Ala Ile Leu Pro Glu Glu Gln Asp Gln Asn Tyr Val Cys Lys
35 40 45

His Thr Tyr Val Asp Arg Gly Trp Gly Asn Gly Cys Gly Leu Phe Gly
50 55 60

Lys Gly Ser Leu Val Thr Cys Ala Lys Phe Gln
65 70 75

<210> 32
<211> 75
<212> PRT
<213> Artificial Sequence

<220>
<223> Description of the Artificial Sequence : This is the mature protein PMEC displaying segments B and C from dengue4 virus

<220>
<223> Protein.

<400> 32
Met Val Thr Phe Lys Val Pro His Ala Lys Arg Gln Asp Val Thr Val
1 5 10 15

Gly Gly Ser Ile Ser Asn Ile Thr Thr Ala Thr Arg Cys Pro Thr Gln
20 25 30

Gly Glu Pro Tyr Leu Lys Glu Glu Gln Asp Gln Gln Tyr Ile Cys Arg
35 40 45

Arg Asp Val Val Asp Arg Gly Trp Gly Asn Gly Cys Gly Leu Phe Gly
50 55 60

Lys Gly Gly Val Val Thr Cys Ala Lys Phe Ser
65 70 75

<210> 33
<211> 75
<212> PRT
<213> Artificial Sequence

<220>
<223> Description of the Artificial Sequence : This is the mature protein PMEC displaying segments B and C from West Nile virus

<220>
<223> Protein.

<400> 33
Leu Met Glu Phe Glu Glu Pro His Ala Thr Lys Gln Ser Val Val Ala
1 5 10 15

Gly Gly Ser Val Ser Asp Leu Ser Thr Arg Ala Ala Cys Pro Thr Met

```
                20                  25                  30

Gly Glu Ala His Asn Glu Lys Arg Ala Asp Pro Ala Phe Val Cys Lys
            35                  40                  45

Gln Gly Val Val Asp Arg Gly Trp Gly Asn Gly Cys Gly Leu Phe Gly
        50                  55                  60

Lys Gly Ser Ile Asp Thr Cys Ala Lys Phe Ala
 65                  70                  75


<210> 34
<211> 75
<212> PRT
<213> Artificial Sequence

<220>
<223> Description of the Artificial Sequence: This is the
      mature protein PMEC displaying segments B and C from
      Japanese Encephalitis virus

<220>
<223> Protein.

<400> 34
Leu Met Glu Phe Glu Gly Ala His Ala Thr Lys Gln Ser Val Val Ala
  1               5                  10                  15

Gly Gly Ser Val Thr Asp Ile Ser Thr Val Ala Arg Cys Pro Thr Thr
                20                  25                  30

Gly Glu Ala His Asn Glu Lys Arg Ala Asp Ser Ser Tyr Val Cys Lys
            35                  40                  45

Gln Gly Phe Thr Asp Arg Gly Trp Gly Asn Gly Cys Gly Leu Phe Gly
        50                  55                  60

Lys Gly Ser Ile Asp Thr Cys Ala Lys Phe Ser
 65                  70                  75


<210> 35
<211> 75
<212> PRT
<213> Artificial Sequence

<220>
<223> Description of the Artificial Sequence : This is the
      mature protein PMEC displaying segments B and C from
      Murray Valley Encephalitis virus

<220>
<223> Protein.

<400> 35
Leu Val Glu Phe Glu Glu Pro His Ala Thr Lys Gln Ser Val Val Ala
  1               5                  10                  15

Gly Gly Thr Val Ser Asp Val Ser Thr Val Ser Asn Cys Pro Thr Thr
                20                  25                  30

Gly Glu Ser His Asn Thr Lys Arg Ala Asp His Asn Tyr Leu Cys Lys
            35                  40                  45

Arg Gly Val Thr Asp Arg Gly Trp Gly Asn Gly Cys Gly Leu Phe Gly
        50                  55                  60

Lys Gly Ser Ile Asp Thr Cys Ala Lys Phe Thr
```

```
65                  70                  75


<210> 36
<211> 75
<212> PRT
<213> Artificial Sequence

<220>
<223> Description of the Artificial Sequence: This is the
      mature protein PMEC displaying segments B and C from
      kunjin virus

<220>
<223> Protein.

<400> 36
Leu Met Glu Phe Glu Glu Pro His Ala Thr Lys Gln Ser Val Ile Ala
  1               5                  10                  15

Gly Gly Thr Val Ser Glu Leu Ser Thr Lys Ala Ala Cys Pro Thr Met
             20                  25                  30

Gly Glu Ala His Asn Asp Lys Arg Ala Asp Pro Ser Phe Val Cys Lys
         35                  40                  45

Gln Gly Val Val Asp Arg Gly Trp Gly Asn Gly Cys Gly Leu Phe Gly
     50                  55                  60

Lys Gly Ser Ile Asp Thr Cys Ala Lys Phe Ala
 65                  70                  75


<210> 37
<211> 75
<212> PRT
<213> Artificial Sequence

<220>
<223> Description of the Artificial Sequence: This is the
      mature protein PMEC displaying segments B and C from
      St Louis Encephalitis virus

<220>
<223> Protein.

<400> 37
Leu Val Glu Phe Glu Glu Pro His Ala Thr Lys Gln Thr Val Val Ala
  1               5                  10                  15

Gly Gly Thr Leu Asp Thr Leu Ser Thr Val Ala Arg Cys Pro Thr Thr
             20                  25                  30

Gly Glu Ala His Asn Thr Lys Arg Ser Asp Pro Thr Phe Val Cys Lys
         35                  40                  45

Arg Asp Val Val Asp Arg Gly Trp Gly Asn Gly Cys Gly Leu Phe Gly
     50                  55                  60

Lys Gly Ser Ile Asp Thr Cys Ala Lys Phe Thr
 65                  70                  75


<210> 38
<211> 75
<212> PRT
<213> Artificial Sequence

<220>
```

```
<223> Description of the Artificial Sequence: This is the
      mature protein PMEC displaying segments B and C from
      Yellow Fever virus

<220>
<223> Protein.

<400> 38
Leu Val Glu Phe Glu Pro Pro His Ala Ala Thr Ile Arg Val Leu Ala
  1               5                  10                  15

Gly Gly Val Leu Thr His Val Lys Ile Asn Asp Lys Cys Pro Ser Thr
             20                  25                  30

Gly Glu Ala His Leu Ala Glu Glu Asn Glu Gly Asp Asn Ala Cys Lys
         35                  40                  45

Arg Thr Tyr Ser Asp Arg Gly Trp Gly Asn Gly Cys Gly Leu Phe Gly
     50                  55                  60

Lys Gly Ser Ile Val Ala Cys Ala Lys Phe Thr
 65                  70                  75


<210> 39
<211> 75
<212> PRT
<213> Artificial Sequence

<220>
<223> Description of the Artificial Sequence : This is the
      mature protein PMEC displaying segments B and C from
      Tick-born Encephalitis virus

<220>
<223> Protein.

<400> 39
Leu Val Glu Phe Gly Ala Pro His Ala Val Lys Met Asp Val Tyr Asn
  1               5                  10                  15

Gly Gly Lys Leu Ser Asp Thr Lys Val Ala Ala Arg Cys Pro Thr Met
             20                  25                  30

Gly Pro Ala Thr Leu Ala Glu Glu His Gln Ser Gly Thr Val Cys Lys
         35                  40                  45

Arg Asp Gln Ser Asp Arg Gly Trp Gly Asn His Cys Gly Leu Phe Gly
     50                  55                  60

Lys Gly Ser Ile Val Thr Cys Val Lys Ala Ser
 65                  70                  75


<210> 40
<211> 75
<212> PRT
<213> Artificial Sequence

<220>
<223> Description of the Artificial Sequence: This is the
      mature protein PMEC displaying segments B and C from
      LANGAT virus

<220>
<223> Protein.

<400> 40
Leu Val Glu Phe Gly Thr Pro His Ala Val Lys Met Asp Val Phe Asn
```

```
1               5                   10                  15

Gly Gly Lys Leu Thr Gly Thr Lys Val Ala Ala Arg Cys Pro Thr Met
            20                  25                  30

Gly Pro Ala Thr Leu Pro Glu Glu His Gln Ser Gly Thr Val Cys Lys
        35                  40                  45

Arg Asp Gln Ser Asp Arg Gly Trp Gly Asn His Cys Gly Leu Phe Gly
    50                  55                  60

Lys Gly Ser Ile Val Thr Cys Val Lys Phe Thr
65                  70                  75


<210> 41
<211> 75
<212> PRT
<213> Artificial Sequence

<220>
<223> Description of the Artificial Sequence : This is the
      mature protein PMEC displaying segments B and C from
      Looping ill virus

<220>
<223> Protein.

<400> 41
Leu Val Glu Phe Gly Ala Pro His Ala Val Lys Met Asp Val Tyr Asn
1               5                   10                  15

Gly Gly Lys Leu Ser Glu Thr Lys Val Ala Ala Arg Cys Pro Thr Met
            20                  25                  30

Gly Pro Ala Ala Leu Ala Glu Glu Arg Gln Ile Gly Thr Val Cys Lys
        35                  40                  45

Arg Asp Gln Ser Asp Arg Gly Trp Gly Asn His Cys Gly Leu Phe Gly
    50                  55                  60

Lys Gly Ser Ile Val Ala Cys Val Lys Ala Ala
65                  70                  75


<210> 42
<211> 75
<212> PRT
<213> Artificial Sequence

<220>
<223> Description of the Artificial Sequence: This is the
      mature protein PMEC displaying segments B and C from
      POWASSAN virus

<220>
<223> Protein.

<400> 42
Leu Val Glu Phe Gly Pro Pro His Ala Val Lys Met Asp Val Phe Asn
1               5                   10                  15

Gly Gly Lys Leu Thr Asn Thr Lys Val Glu Ala Arg Cys Pro Thr Thr
            20                  25                  30

Gly Pro Ala Thr Leu Pro Glu Glu His Gln Ala Asn Met Val Cys Lys
        35                  40                  45

Arg Asp Gln Ser Asp Arg Gly Trp Gly Asn His Cys Gly Phe Phe Gly
```

```
     50                  55                  60

Lys Gly Ser Ile Val Ala Cys Ala Lys Phe Glu
 65                  70                  75


<210> 43
<211> 75
<212> PRT
<213> Artificial Sequence

<220>
<223> Description of the Artificial Sequence: This is a
      sequence corresponding to the mature PMEC protein
      mutated at amino acid positions inaccessible to
      interaction with antibodies

<220>
<223> Protein.

<400> 43
Asp Thr Val Tyr Arg Asn Glu His Asn Lys Lys His Asp Val Val Ser
  1               5                  10                  15

Ala Ser Lys Leu Thr Asn Thr Thr Thr Glu Ser Arg Cys Thr Gly Gln
             20                  25                  30

Gly Val Ala Thr Leu Asn Glu Asp Glu Asp Lys Arg Phe Asn Cys Tyr
         35                  40                  45

Leu Asp Leu Val Tyr Arg Gly Trp Gly Asn Gly Cys Gly Asp Arg Gly
     50                  55                  60

Leu Gly Phe Ile Lys Gln Cys Ser Met Lys Val
 65                  70                  75


<210> 44
<211> 75
<212> PRT
<213> Artificial Sequence

<220>
<223> Description of the Artificial Sequence : This is a
      sequence corresponding to the mature PMEC protein
      mutated at amino acid positions inaccessible to
      interaction with antibodies

<220>
<223> Protein.

<400> 44
Asp Thr Glu Ile Tyr Asn Glu His Gly Lys Lys Thr Asp Val Val Thr
  1               5                  10                  15

Thr Ala Lys Leu Thr Asn Thr Thr Thr Glu Ser Arg Cys Pro Gly Gln
             20                  25                  30

Gly Glu Gln Thr Leu Asn Glu Asp Ala Asp Gln Arg Phe Phe Cys Val
         35                  40                  45

Lys Asp Leu Val Tyr Arg Gly Trp Gly Asn Gly Cys Gly Val Arg Gly
     50                  55                  60

Trp Gly Thr Ile Gln Gln Cys Val Met Lys Val
 65                  70                  75


<210> 45
```

<211> 75
<212> PRT
<213> Artificial Sequence

<220>
<223> Description of the Artificial Sequence : This is a sequence corresponding to the mature PMEC protein mutated at amino acid positions inaccessible to interaction with antibodies

<220>
<223> Protein.

<400> 45
Asp Thr Val Val Arg Thr Glu His Lys Lys Lys Ile Asp Val Val Ser
1 5 10 15

Ser Thr Lys Leu Thr Asn Thr Thr Thr Glu Ser Arg Cys Pro Gly Gln
20 25 30

Gly Glu Ser Thr Leu Asn Glu Glu Glu Asp Glu Arg Phe Asp Cys Gln
35 40 45

Gln Asp Gln Val Leu Arg Gly Trp Gly Asn Gly Cys Gly Val Pro Gly
50 55 60

Trp Gly Asn Ile Lys Lys Cys Ala Met Lys Glu
65 70 75

<210> 46
<211> 75
<212> PRT
<213> Artificial Sequence

<220>
<223> Description of the Artificial Sequence : This is a sequence corresponding to the mature PMEC protein mutated at amino acid positions inaccessible to interaction with antibodies

<220>
<223> Protein.

<400> 46
Glu Ser Val Ser Val Asn Glu His Lys Lys Lys Asn Asp Val Val Ser
1 5 10 15

Asp Thr Lys Leu Thr Asn Thr Thr Thr Glu Ser Arg Cys Pro Gly Gln
20 25 30

Gly Glu Pro Thr Leu Asn Glu Glu Glu Asp Glu Arg Phe Asp Cys Gln
35 40 45

Lys Asp Leu Val Tyr Arg Gly Trp Gly Asn Gly Cys Gly Val Arg Gly
50 55 60

Trp Gly Trp Ile Lys Lys Cys Ala Met Lys Val
65 70 75

<210> 47
<211> 75
<212> PRT
<213> Artificial Sequence

<220>
<223> Description of the Artificial Sequence: This is a sequence corresponding to the mature PMEC protein

mutated at amino acid positions inaccessible to interaction with antibodies

<220>
<223> Protein.

<400> 47

```
Lys Val Val Ser Arg Asn Glu His Arg Lys Lys Asn Asp Val Val Ser
 1               5                  10                  15

Glu Thr Lys Leu Thr Asn Thr Thr Thr Glu Ser Arg Cys Pro Asn Gln
            20                  25                  30

Gly Glu Ala Thr Leu Asn Glu Asp Glu Asp Glu Arg Phe Glu Cys Val
        35                  40                  45

Lys Asp Val Val Tyr Arg Gly Trp Gly Asn Gly Cys Gly Glu Arg Gly
    50                  55                  60

Leu Gly Thr Ile Gln Gln Cys Trp Met Asn Glu
65                  70                  75
```

<210> 48
<211> 75
<212> PRT
<213> Artificial Sequence

<220>
<223> Description of the Artificial Sequence: This is a sequence corresponding to the mature PMEC protein mutated at amino acid positions inaccessible to interaction with antibodies

<220>
<223> Protein.

<400> 48

```
Asp Asp Glu Tyr Tyr Leu Glu His Tyr Lys Lys Leu Asp Val Val Ser
 1               5                  10                  15

Arg Thr Lys Leu Thr Asn Thr Thr Thr Glu Ser Arg Cys Pro Gly Gln
            20                  25                  30

Gly Gln Ala Thr Leu Asn Glu Glu Glu Asp Glu Arg Phe Gln Cys Phe
        35                  40                  45

Val Ala Leu Val Ile Arg Gly Trp Gly Asn Gly Cys Gly Val Val Gly
    50                  55                  60

Trp Gly Ser Ile Val Val Cys Lys Met Lys Ile
65                  70                  75
```

<210> 49
<211> 75
<212> PRT
<213> Artificial Sequence

<220>
<223> Description of the Artificial Sequence: This is a sequence corresponding to the mature PMEC protein mutated at amino acid positions inaccessible to interaction with antibodies

<220>
<223> Protein.

<400> 49

```
Asp Thr Val Val Val Asn Glu His Asn Lys Lys Ile Asp Val Val Ser
  1               5                  10                  15

Thr Ser Lys Leu Thr Asn Thr Thr Thr Glu Ser Arg Cys Pro Gly Gln
             20                  25                  30

Gly Pro Ala Thr Leu Asn Glu Ala Glu Asp Glu Arg Phe Asp Cys Gln
         35                  40                  45

Phe Ser Tyr Val Tyr Arg Gly Trp Gly Asn Gly Cys Gly Lys Arg Gly
     50                  55                  60

Leu Gly Pro Ile Leu Val Cys Ala Met Lys Val
 65                  70                  75


<210> 50
<211> 75
<212> PRT
<213> Artificial Sequence

<220>
<223> Description of the Artificial Sequence: This is a
      sequence corresponding to the mature PMEC protein
      mutated at amino acid positions inaccessible to
      interaction with antibodies

<220>
<223> Protein.

<400> 50
Asp Asn Val Val Val Asn Glu His Tyr Lys Lys Thr Asp Val Val Ser
  1               5                  10                  15

Ser Thr Lys Leu Thr Asn Thr Thr Thr Glu Ser Arg Cys Pro Gly Gln
             20                  25                  30

Gly Tyr Pro Thr Leu Asn Glu Gln Ser Asp Glu Arg Phe Val Cys Phe
         35                  40                  45

Val Asp Tyr Val Ile Arg Gly Trp Gly Asn Gly Cys Gly Val Asp Gly
     50                  55                  60

Trp Gly Pro Ile Val Val Cys Lys Met Lys Ile
 65                  70                  75


<210> 51
<211> 42
<212> PRT
<213> Homo sapiens

<220>
<223> Peptide fragment.

<220>
<223> Sequence of the trimerization domain of matrilin.

<400> 51
Glu Asp Pro Cys Ala Cys Glu Ser Leu Val Lys Phe Gln Ala Lys Val
  1               5                  10                  15

Glu Gly Leu Leu Gln Ala Leu Thr Arg Lys Leu Glu Ala Val Ser Lys
             20                  25                  30

Arg Leu Ala Ile Leu Glu Asn Thr Val Val
         35                  40
```

<210> 52
<211> 232
<212> PRT
<213> Homo sapiens

<220>
<223> Polypeptide.

<220>
<223> Sequence of the FC fragment of human IgG1 molecule hinge domain, CH2 y CH3.

<400> 52

```
Glu Pro Lys Ser Ser Asp Lys Thr His Thr Cys Pro Pro Cys Pro Ala
 1               5                  10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Gln Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230
```

<210> 53
<211> 250
<212> PRT
<213> Artificial Sequence

<220>
<223> Description of the Artificial Sequence: Single chain antibody fragment displaying the variable regions of the mAb 4G2, after secreted to the periplasm

<220>
<223> Protein.

<400> 53
```
Met Ala Asn Ile Val Met Thr Gln Ser Pro Lys Ser Met Ser Met Ser
 1               5                  10                  15

Val Gly Glu Arg Val Thr Leu Thr Cys Lys Ala Ser Glu Asn Val Val
            20                  25                  30

Thr Tyr Val Ser Trp Tyr Gln Gln Lys Pro Glu Gln Ser Pro Lys Leu
        35                  40                  45

Leu Ile Tyr Gly Ala Ser Asn Arg Tyr Thr Gly Val Pro Asp Arg Phe
    50                  55                  60

Thr Gly Ser Gly Ser Ala Thr Asp Phe Thr Leu Thr Ile Ser Ser Val
65                  70                  75                  80

Gln Ala Glu Asp Leu Ala Asp Tyr His Cys Gly Gln Gly Tyr Ser Tyr
                85                  90                  95

Pro Tyr Thr Phe Gly Gly Gly Thr Lys Leu Glu Ile Lys Glu Gly Lys
            100                 105                 110

Ser Ser Gly Ser Gly Ser Glu Ser Lys Val Asp Glu Val Gln Leu Gln
        115                 120                 125

Gln Ser Gly Pro Glu Leu Val Lys Pro Gly Thr Ser Val Lys Ile Ser
    130                 135                 140

Cys Lys Thr Ser Gly Tyr Thr Phe Thr Glu Tyr Thr Ile His Trp Val
145                 150                 155                 160

Lys Gln Ser His Gly Lys Ser Leu Ala Trp Ile Gly Gly Ile Asp Pro
                165                 170                 175

Asn Ser Gly Gly Thr Asn Tyr Ser Pro Asn Phe Lys Gly Lys Ala Thr
            180                 185                 190

Leu Thr Val Asp Lys Ser Ser Ser Thr Ala Tyr Met Asp Leu Arg Ser
        195                 200                 205

Leu Ser Ser Glu Asp Ser Ala Val Tyr Phe Cys Ala Arg Ile Tyr His
    210                 215                 220

Tyr Asp Gly Tyr Phe Asp Val Trp Gly Ala Gly Thr Ala Val Thr Val
225                 230                 235                 240

Ser Ser Leu Glu His His His His His His
                245                 250
```

<210> 54
<211> 307
<212> PRT
<213> Artificial Sequence

<220>
<223> Description of the Artificial Sequence: Sequence of the multivalent protein displaying the variable regions of mAb 4G2 and the trimeric matrilin domain after secreted to the periplasm

<220>
<223> Protein.

<400> 54
```
Met Ala Asn Ile Val Met Thr Gln Ser Pro Lys Ser Met Ser Met Ser
```

```
1               5                   10                  15

Val Gly Glu Arg Val Thr Leu Thr Cys Lys Ala Ser Glu Asn Val Val
            20                  25                  30

Thr Tyr Val Ser Trp Tyr Gln Gln Lys Pro Glu Gln Ser Pro Lys Leu
        35                  40                  45

Leu Ile Tyr Gly Ala Ser Asn Arg Tyr Thr Gly Val Pro Asp Arg Phe
    50                  55                  60

Thr Gly Ser Gly Ser Ala Thr Asp Phe Thr Leu Thr Ile Ser Ser Val
65                  70                  75                  80

Gln Ala Glu Asp Leu Ala Asp Tyr His Cys Gly Gln Gly Tyr Ser Tyr
                85                  90                  95

Pro Tyr Thr Phe Gly Gly Gly Thr Lys Leu Glu Ile Lys Glu Gly Lys
            100                 105                 110

Ser Ser Gly Ser Gly Ser Glu Ser Lys Val Asp Glu Val Gln Leu Gln
        115                 120                 125

Gln Ser Gly Pro Glu Leu Val Lys Pro Gly Thr Ser Val Lys Ile Ser
    130                 135                 140

Cys Lys Thr Ser Gly Tyr Thr Phe Thr Glu Tyr Thr Ile His Trp Val
145                 150                 155                 160

Lys Gln Ser His Gly Lys Ser Leu Ala Trp Ile Gly Gly Ile Asp Pro
                165                 170                 175

Asn Ser Gly Gly Thr Asn Tyr Ser Pro Asn Phe Lys Gly Lys Ala Thr
            180                 185                 190

Leu Thr Val Asp Lys Ser Ser Ser Thr Ala Tyr Met Asp Leu Arg Ser
        195                 200                 205

Leu Ser Ser Glu Asp Ser Ala Val Tyr Phe Cys Ala Arg Ile Tyr His
    210                 215                 220

Tyr Asp Gly Tyr Phe Asp Val Trp Gly Ala Gly Thr Ala Val Thr Val
225                 230                 235                 240

Ser Ser Gly Gly Gly Ser Gly Gly Gly Ser Gly Gly Gly Ser Gly Gly
                245                 250                 255

Gly Glu Asp Pro Cys Ala Cys Glu Ser Leu Val Lys Phe Gln Ala Lys
            260                 265                 270

Val Glu Gly Leu Leu Gln Ala Leu Thr Arg Lys Leu Glu Ala Val Ser
        275                 280                 285

Lys Arg Leu Ala Ile Leu Glu Asn Thr Val Val Leu Glu His His His
    290                 295                 300

His His His
305


<210> 55
<211> 485
<212> PRT
<213> Artificial Sequence

<220>
<223> Description of the Artificial Sequence: Sequence
      of the single chain miniantibody displaying
      the variable regions of mAb 4G2
```

```
<220>
<223> Protein.

<400> 55
Met Ala Asn Ile Val Met Thr Gln Ser Pro Lys Ser Met Ser Met Ser
1               5                   10                  15

Val Gly Glu Arg Val Thr Leu Thr Cys Lys Ala Ser Glu Asn Val Val
            20                  25                  30

Thr Tyr Val Ser Trp Tyr Gln Gln Lys Pro Glu Gln Ser Pro Lys Leu
        35                  40                  45

Leu Ile Tyr Gly Ala Ser Asn Arg Tyr Thr Gly Val Pro Asp Arg Phe
    50                  55                  60

Thr Gly Ser Gly Ser Ala Thr Asp Phe Thr Leu Thr Ile Ser Ser Val
65                  70                  75                  80

Gln Ala Glu Asp Leu Ala Asp Tyr His Cys Gly Gln Gly Tyr Ser Tyr
                85                  90                  95

Pro Tyr Thr Phe Gly Gly Gly Thr Lys Leu Glu Ile Lys Glu Gly Lys
            100                 105                 110

Ser Ser Gly Ser Gly Ser Glu Ser Lys Val Asp Glu Val Gln Leu Gln
        115                 120                 125

Gln Ser Gly Pro Glu Leu Val Lys Pro Gly Thr Ser Val Lys Ile Ser
    130                 135                 140

Cys Lys Thr Ser Gly Tyr Thr Phe Thr Glu Tyr Thr Ile His Trp Val
145                 150                 155                 160

Lys Gln Ser His Gly Lys Ser Leu Ala Trp Ile Gly Gly Ile Asp Pro
                165                 170                 175

Asn Ser Gly Gly Thr Asn Tyr Ser Pro Asn Phe Lys Gly Lys Ala Thr
            180                 185                 190

Leu Thr Val Asp Lys Ser Ser Ser Thr Ala Tyr Met Asp Leu Arg Ser
        195                 200                 205

Leu Ser Ser Glu Asp Ser Ala Val Tyr Phe Cys Ala Arg Ile Tyr His
    210                 215                 220

Tyr Asp Gly Tyr Phe Asp Val Trp Gly Ala Gly Thr Ala Val Thr Val
225                 230                 235                 240

Ser Ser Gly Gly Gly Glu Pro Lys Ser Ser Asp Lys Thr His Thr Cys
                245                 250                 255

Pro Pro Cys Pro Ala Pro Glu Leu Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Asp Val Ser His Glu Asp Pro Glu Val Lys
    290                 295                 300

Phe Asn Trp Tyr Val Asp Gly Val Glu Val His Asn Ala Lys Thr Lys
305                 310                 315                 320

Pro Arg Glu Glu Gln Tyr Gln Ser Thr Tyr Arg Val Val Ser Val Leu
                325                 330                 335

Thr Val Leu His Gln Asp Trp Leu Asn Gly Lys Glu Tyr Lys Cys Lys
```

```
            340                 345                 350

Val Ser Asn Lys Ala Leu Pro Ala Pro Ile Glu Lys Thr Ile Ser Lys
        355                 360                 365

Ala Lys Gly Gln Pro Arg Glu Pro Gln Val Tyr Thr Leu Pro Pro Ser
    370                 375                 380

Arg Glu Glu Met Thr Lys Asn Gln Val Ser Leu Thr Cys Leu Val Lys
385                 390                 395                 400

Gly Phe Tyr Pro Ser Asp Ile Ala Val Glu Trp Glu Ser Asn Gly Gln
                405                 410                 415

Pro Glu Asn Asn Tyr Lys Thr Thr Pro Pro Val Leu Asp Ser Asp Gly
            420                 425                 430

Ser Phe Phe Leu Tyr Ser Lys Leu Thr Val Asp Lys Ser Arg Trp Gln
        435                 440                 445

Gln Gly Asn Val Phe Ser Cys Ser Val Met His Glu Ala Leu His Asn
    450                 455                 460

His Tyr Thr Gln Lys Ser Leu Ser Leu Ser Pro Gly Lys Leu Glu His
465                 470                 475                 480

His His His His His
                485


<210> 56
<211> 479
<212> PRT
<213> Artificial Sequence

<220>
<223> Description of the Artificial Sequence: Sequence
      the single chain miniantibody MA4G2 displaying
      the variable regions of mAb 4G2

<220>
<223> Protein.

<400> 56
Met Ala Asn Ile Val Met Thr Gln Ser Pro Lys Ser Met Ser Met Ser
  1               5                  10                  15

Val Gly Glu Arg Val Thr Leu Thr Cys Lys Ala Ser Glu Asn Val Val
             20                  25                  30

Thr Tyr Val Ser Trp Tyr Gln Gln Lys Pro Glu Gln Ser Pro Lys Leu
         35                  40                  45

Leu Ile Tyr Gly Ala Ser Asn Arg Tyr Thr Gly Val Pro Asp Arg Phe
     50                  55                  60

Thr Gly Ser Gly Ser Ala Thr Asp Phe Thr Leu Thr Ile Ser Ser Val
 65                  70                  75                  80

Gln Ala Glu Asp Leu Ala Asp Tyr His Cys Gly Gln Gly Tyr Ser Tyr
                 85                  90                  95

Pro Tyr Thr Phe Gly Gly Gly Thr Lys Leu Glu Ile Lys Glu Gly Lys
            100                 105                 110

Ser Ser Gly Ser Gly Ser Glu Ser Lys Val Asp Glu Val Gln Leu Gln
        115                 120                 125

Gln Ser Gly Pro Glu Leu Val Lys Pro Gly Thr Ser Val Lys Ile Ser
```

130 135 140

Cys Lys Thr Ser Gly Tyr Thr Phe Thr Glu Tyr Thr Ile His Trp Val
145 150 155 160

Lys Gln Ser His Gly Lys Ser Leu Ala Trp Ile Gly Gly Ile Asp Pro
165 170 175

Asn Ser Gly Gly Thr Asn Tyr Ser Pro Asn Phe Lys Gly Lys Ala Thr
180 185 190

Leu Thr Val Asp Lys Ser Ser Ser Thr Ala Tyr Met Asp Leu Arg Ser
195 200 205

Leu Ser Ser Glu Asp Ser Ala Val Tyr Phe Cys Ala Arg Ile Tyr His
210 215 220

Tyr Asp Gly Tyr Phe Asp Val Trp Gly Ala Gly Thr Ala Val Thr Val
225 230 235 240

Ser Ser Gly Gly Gly Glu Pro Lys Ser Ser Asp Lys Thr His Thr Cys
245 250 255

Pro Pro Cys Pro Ala Pro Glu Leu Leu Gly Gly Pro Ser Val Phe Leu
260 265 270

Phe Pro Pro Lys Pro Lys Asp Thr Leu Met Ile Ser Arg Thr Pro Glu
275 280 285

Val Thr Cys Val Val Val Asp Val Ser His Glu Asp Pro Glu Val Lys
290 295 300

Phe Asn Trp Tyr Val Asp Gly Val Glu Val His Asn Ala Lys Thr Lys
305 310 315 320

Pro Arg Glu Glu Gln Tyr Gln Ser Thr Tyr Arg Val Val Ser Val Leu
325 330 335

Thr Val Leu His Gln Asp Trp Leu Asn Gly Lys Glu Tyr Lys Cys Lys
340 345 350

Val Ser Asn Lys Ala Leu Pro Ala Pro Ile Glu Lys Thr Ile Ser Lys
355 360 365

Ala Lys Gly Gln Pro Arg Glu Pro Gln Val Tyr Thr Leu Pro Pro Ser
370 375 380

Arg Glu Glu Met Thr Lys Asn Gln Val Ser Leu Thr Cys Leu Val Lys
385 390 395 400

Gly Phe Tyr Pro Ser Asp Ile Ala Val Glu Trp Glu Ser Asn Gly Gln
405 410 415

Pro Glu Asn Asn Tyr Lys Thr Thr Pro Pro Val Leu Asp Ser Asp Gly
420 425 430

Ser Phe Phe Leu Tyr Ser Lys Leu Thr Val Asp Lys Ser Arg Trp Gln
435 440 445

Gln Gly Asn Val Phe Ser Cys Ser Val Met His Glu Ala Leu His Asn
450 455 460

His Tyr Thr Gln Lys Ser Leu Ser Leu Ser Pro Gly Lys Leu Glu
465 470 475

1

## Description

### Field of the invention

The present invention is related to the field of the pharmaceutical industry, and describes a conserved area on the surface of the E protein that can be used for the development of wide-spectrum antiviral molecules to be employed in the prophylaxis and/or treatment of infections due to Dengue Virus serotypes 1-4 and other flaviviruses. The invention describes methods and proteins useful for the prophylactic and/or therapeutic treatment of the four serotypes of Dengue Virus and, alternatively, other flaviviruses.

### Previous art

The Dengue Virus (DV) complex belongs to the *Flaviviridae* family, and is composed of four different viruses or serotypes (DV1-DV4), genetically and antigenically related. DV is transmitted to man through mosquitoes, mainly *Aedes aegypti.* The infection produces varying clinical symptoms, ranging from asymptomatic and benign manifestations such as undifferentiated febrile episodes to more severe manifestations like Dengue Hemorrhagic Fever (DHF) and the life-threatening Dengue Shock Syndrome (DSS). The more severe clinical symptoms are usually associated to sequential infections with two different serotypes *(*Halstead,S.B. Neutralization and antibody-dependent enhancement of dengue viruses. Adv. Virus Res. 60:421-67., 421-467, 2003*. Hammon WMc. New haemorragic fever in children in the* Philippines and Thailand. Trans Assoc Physicians 1960; 73: 140-155*),* a finding that has been corroborated by several epidemiological studies *(*Kouri GP, Guzmán MG, Bravo JR. Why dengue hemorrhagic fever in Cuba? 2. An integral analysis. Trans Roy Soc Trop Med Hyg 1987; 72: 821-823*).* This phenomenon has been explained by the theory of antibody-dependant enhancement (ADE), which argues that, in these cases, there is an increase in viral infectivity due to an increase in the entry of virus-antibody complexes to their target (the monocytes), mediated by the Fc receptors present on these cells *(*Halstead SB. Pathogenesis of dengue: challenges to molecular biology. Science 1988; 239: 476-481*).*

The envelope glycoprotein (E-protein) is the largest structural protein of the viral envelope. The three-dimensional structures of a fragment of the ectodomain of E-protein from DEN2 and DEN3 viruses have recently been solved by x-ray diffraction techniques *(*Modis,Y., Ogata, S., Clements, D. & Harrison, S.C. A ligand-binding pocket in the dengue virus envelope glycoprotein. Proc. Natl. Acad. Sci. U. S. A 100, 6986-6991,2003. Modis, Y., Ogata, S., Clements, D., and Harrison, S.C. Variable Surface Epitopes in the Crystal Structure of Dengue Virus Type 3 Envelope Glycoprotein. J. Virol. 79, 1223-1231, 2005*),* showing a high degree of structural similarity to the crystal structure of E-protein from Tick-borne Encephalitis Virus *(*Rey,F.A., Heinz,F.X., Mandl,C., Kunz,C. & Harrison,S.C. The envelope glycoprotein from tick-borne encephalitis virus at 2 A resolution. Nature 375, 291-298, 1995*).* This structural similarity is congruent with their sequence homology, the conservation of 6 disulphide bridges, and the conservation of the localization of residues to which a functional role, such as being part of an antigenic determinant or being involved in attenuation or escape mutations, has been previously assigned in other flaviviruses.

Protein E is formed by three structural domains: domain I, located on the N-terminal part of the sequence but forming the central domain in the 3D structure; domain II, also known as the dimerization domain, which contains a fusion peptide highly conserved across flaviviruses; and domain III, with an immunoglobulin-like fold, which is involved in the interaction with cellular receptors.

Protein E is a multifunctional glycoprotein that plays a central role in several stages of the viral life cycle. This protein is the main target for virus-neutralizing antibodies, mediates the interaction with the cellular receptors, and is the engine driving the fusion between the viral and cellular membranes *(*Heinz, F. X., and S. L. Allison. 2003. Flavivirus structure and membrane fusion. Adv Virus Res. 59:63-97. Modis, Y., S. Ogata, D. Clements, and S. C. Harrison. 2004. Structure of the dengue virus envelope protein after membrane fusion. Nature 427:393-319. Rey 2004. Chen, Y., T. Maguire, R. E. Hileman, J. R. Fromm, J. D. Esko, R. J.Linhardt, and R. M. Marks. 1997. Dengue virus infectivity depends on envelope protein binding to target cell heparan sulfate. Nat. Med. 3:866-871*.* Navarro-Sanchez, E., R. Altmeyer, A. Amara, O. Schwartz, F. Fieschi, J. L. Virelizier, F. Arenzana-Seisdedos, and P. Despres. 2003. Dendritic-cell-specific ICAM3-grabbing non-integrin is essential for the productive infection of human dendritic cells by mosquito-cell-derived dengue viruses. EMBO Rep. 4:1-6. Tassaneetrithep, B., T. H. Burgess, A. Granelli-Piperno, C. Trumpfheller, J. Finke, W Sun, M. A. Eller, K. Pattanapanyasat, S. Sarasombath, D. L. Birx, R. M. Steinman, S. Schlesinger, and M. A. Marovich. 2003. DC-SIGN (CD209) mediates dengue virus infection of human dendritic cells. J. Exp. Med. 197:823-829*).*

This protein is anchored to the viral membrane, and its functions are associated to large conformational changes, both in tertiary and quaternary structure. During the intracellular stages of virus formation, E is found as a heterodimer together with the preM protein *(*Allison, S. L., K. Stadler, C. W Mandl, C. Kunz, and F. X. Heinz. 1995. Synthesis and secretion of recombinant tick-borne encephalitis virus protein E in soluble and particulate form. J. Virol. 69:5816-5820. Rice, C. M. 1996. Flaviviridae: the viruses and their replication, p. 931-959. In B. N. Fields, D. N. Knipe, P. M. Howley, R. M. Chanock, J. L. Melnick, T. P. Monath, B. Roizman, and S. E. Straus (ed.), Virology, 3rd ed. Lippincott-Raven, Philadelphia, Pa*.).* During this stage the virions are said to be immature, and are defective for mediating membrane fusion, as evidenced in their dramatically lower infectivity *in vitro* when compared to mature extracellular virions *(*Guirakhoo, F., Heinz, F. X., Mandl, C. W, Holzmann, H. & Kunz, C. Fusion activity of flaviviruses: comparison of mature and immature (prM containing) tick-borne encephalitis virions. J. Gen. Virol. 72, 1323-1329,1991. Guirakhoo, F., Bolin, R. A. & Roehrig, J. T. The Murray Valley encephalitis virus prM protein confers acid resistance to virus particles and alters the expression of epitopes within the R2 domain of E glycoprotein. Virology 191, 921-931,1992*).* It is postulated that the role of the heterodimers is to prevent the binding of protein E to the membrane during the traffic of the virions through intracellular compartments that could, due to their acidic pH, trigger the membrane fusion process. Besides, it is possible that the preM protein functions as a chaperone for the folding and assembly of protein E *(*Lorenz, I. C., Allison, S. L., Heinz, F. X. & Helenius, A. Folding and dimerization of tick-borne encephalitis virus envelope proteins prM and E in the endoplasmic reticulum. J. Virol. 76, 5480-5491, 2002*).* During secretion of the virions out of the cell, preM is enzymatically processed by host proteases (furins), leaving E free to associate as homodimers and therefore triggering a reorganization of the viral envelope that ends with the formation of mature virions *(*Stadler, K., Allison, S. L., Schalich, J. & Heinz, F. X. Proteolytic activation of tick-borne encephalitis virus by furin. J. Virol. 71, 8475-8481, 1997. Elshuber, S., Allison, S. L., Heinz, F. X. & Mandl, C. W Cleavage of protein prM is necessary for infection of BHK-21 cells by tick-borne encephalitis virus. J. Gen. Virol. 84, 183-191, 2003*).* The structure of mature virions has been determined by electronic cryomicroscopy at a resolution of 9.5 Å *(*Zhang W, Chipman PR, Corver J, Johnson PR, Zhang Y, Mukhopadhyay S, Baker TS, Strauss JH, Rossmann MG, Kuhn RJ. Visualization of membrane protein domains by cryo-electron microscopy of dengue virus. Nat Struct Biol. 2003, 10: 907-12. Kuhn, R. J. et al. Structure of dengue virus: implications for flavivirus organization, maturation, and fusion. Cell 108, 717-725, 2002*),* and that of immature virions, at 12.5 Å (Zhang, Y. et al. Structures of immature flavivirus particles. EMBO J. 22, 2604-2613 , 2003). These virions have a T=3 icosahedral symmetry. In the mature virions the protein E dimers lay on a plane parallel to the viral membrane, covering its surface almost completely. Mature virions are completely infective, and it is in this conformation that the virus interacts with the cellular receptors and the antibodies elicited by the host. Once the virus engages the cellular receptors, it is internalized through receptor-mediated endocytosis, and eventually reaches the endosomes, where the slightly acid pH triggers the conformational change in protein E that starts the membrane fusion process *(*Allison, S. L. et al. Oligomeric rearrangement of tick-borne encephalitis virus envelope proteins induced by an acidic pH. J. Virol. 69, 695-700, 1995). In this process the protein reorganizes from dimers to trimers. The post-fusogenic structure of protein E has been recently determined *(*Modis, Y., Ogata, S., Clements, D. & Harrison, S. C. Structure of the dengue virus envelope protein after membrane fusion. Nature 427, 313-319 (2004*).* Bressanelli, S. et al. Structure of a flavivirus envelope glycoprotein in its low pH-induced membrane fusion conformation. EMBO J. 23, 728-738 (2004*),* showing that trimer formation involves important rearrangements in tertiary structure, with the monomers associating in parallel while the tip of domain II, containing the fusion peptide, interacts with the membranes. By analyzing together the crystal structures and the resolved virion structures it becomes evident that throughout the viral life cycle protein E is subjected to rearrangements in which its ternary and quaternary structure, as well as the virion itself, changes dramatically.

Protein E is the main target of the neutralizing antibodies generated during the viral infection. An infection with a single serotype elicits long-lived antibodies which are neutralizing against viruses of the homologous serotype. During the first months after the infection these antibodies can neutralize heterologous serotypes as well, but this activity slowly decreases until it disappears, about 9 months post-infection *(*Halstead S.B. Neutralization and antibody-dependent enhancement of dengue viruses. Adv Virus Res. 2003;60: 421-67. Sabin, A. B. 1952. Research on dengue during World War II. Am. J. Trop. Med. Hyg. 1: 30-50*.)*

The antibodies generated against one serotype generally display a decreased affinity when interacting with viruses of a different serotype; a phenomenon that is explained at the molecular level by variations in the aminoacid sequence of protein E among DV serotypes. An interaction of sufficiently low affinity can result in an antibody that fails to neutralize, but is still able to bind the viral surface in amounts enough to facilitate the internalization of the virus into cells carrying Fc receptors *(*Halstead, S. B., and E. J. O'Rourke. 1977. Dengue viruses and mononuclear phagocytes. I. Infection enhancement by non-neutralizing antibody. J. Exp. Med. 146:201-217. Littaua, R., I. Kurane, and F. A. Ennis. 1990. Human IgG Fc receptor II mediates antibody-dependent enhancement of dengue virus infection. J. Immunol. 144: 3183-3186*).*

Additionally, during a secondary infection the titer of the low-affinity antibody population surpasses that of the new high-affinity antibodies generated by the incoming DV serotype, due to the faster activation of pre-existing memory B-cells and plasma cells when compared to naive B-cells. The antibody profile during convalescence from secondary infections is greatly influenced by the serotype of the primary DV infection, a fact that is just a manifestation of the phenomenon known as original antigenic sin" *(*Halstead, S.B., Rojanasuphot, S., and Sangkawibha, N. 1983. Original antigenic sin in dengue. Am. J. Trop. Med. Hyg. 32:154-156*).*

On the other hand, it is known that highly potent neutralizing monoclonal antibodies (mAbs) can trigger immunoamplification at high dilutions *(*Brandt, W E., J. M. McCown, M. K. Gentry, and P. K. Russell. 1982. Infection enhancement of dengue type 2 virus in the U-937 human monocyte cell line by antibodies to flavivirus cross-reactive determinants.

Infect. Immun. 36:1036-1041. Halstead, S. B., C. N. Venkateshan, M. K. Gentry, and L. K. Larsen. 1984. Heterogeneity of infection enhancement of dengue 2 strains by monoclonal antibodies. J. Immunol 132:1529-1532. Morens, D. M., S. B. Halstead, and N. J. Marchette. 1987. Profiles of antibody-dependent enhancement of dengue virus type 2 infection. Microb. Pathog. 3:231-237*).*

The antigenic structure of the flaviviral protein E has been intensely studied, using murine mAb panels and a group of biochemical and biological analyses that includes competition assays, sensitivity of the interaction to procedures such as reduction of the disulphide bridges and treatment with SDS, assays for binding to proteolytic fragments and synthetic peptides, assays for viral neutralization or inhibition of hemagglutination, generation of escape mutants, serological tests, etc. *(*Heinz. T. Roehrig, J.T., Bolin, R.A. and Kelly, R.G. Monoclonal Antibody Mapping of the Envelope Glycoprotein of the Dengue 2 Virus, Jamaica, VIROLOGY 246, 317-328, 1998. Heinz, F. X , and Roehrig, J. T. (1990). Flaviviruses. In "Immunochemistry of Viruses. II. The Basis for Serodiagnosis and Vaccines" (M.H.V. Van Regenmortel and A.R. Neurath, Eds.), pp. 289-305. Elsevier, Amsterd*am.* Mandl, C. W., Guirakhoo, F. G., Holzmann, H., Heinz, F. X., and Kunz, C. (1989). Antigenic structure of the flavivirus envelope protein E at the molecular level, using tick-borne encephalitis virus as a model. J. Virol. 63, 564-571. I. L. Serafin and J. G. Aaskov. Identification of epitopes on the envelope (E) protein of dengue 2 and dengue 3 viruses using monoclonal antibodies. Arch Virol (2001) 146: 2469-2479. Three antigenic domains, A, B and C, have been defined, which correspond to the three structural domains II, III and I, respectively. The antibodies recognizing a particular epitope usually show very similar functional characteristics. The recognition of epitopes from domain A (equivalent to structural domain II) is destroyed by the reduction of disulphide bridges, and the mAbs recognizing these epitopes inhibit hemagglutination, neutralize viral infection and inhibit virus-mediated membrane fusion. Particularly, epitope A1, defined for Dengue Virus, is recognized by mAbs with group-type specificity, *i.e.* they are highly cross-reactive among different flaviviruses. The mAbs 4G2 (anti-DV2) and 6B6C (anti-JEV) recognize this epitope. Binding to this epitope is diminished by several orders of magnitude in immature virions, and is not enhanced by acid pH treatment of mature virions *(*Guirakhoo, F., R. A. Bolin, and J. T. Roehrig. 1992. The Murray Valley encephalitis virus prM protein confers acid resistance to virus particles and alters the expression of epitopes within the R2 domain of E glycoprotein. Virology 191:921-931*).*

**Vaccine development**

No specific treatments against DV and its most severe manifestations are currently available. Mosquito control is costly and not very efficient. Although the clinical treatments based on a proper management of fluids to correct the hypovolemia caused by DHF has decreased its mortality, these treatments are still problematic in many underdeveloped nations. It has been estimated that 30 000 deaths per year are attributable to DHF, and the morbidity and associated costs of this disease are comparable to those of other diseases which constitute first priority targets of public health spending *(*Shepard DS, Suaya JA, Halstead SB, Nathan MB, Gubler DJ, Mahoney RT, Wang DN, Meltzer MI. Cost-effectiveness of a pediatric dengue vaccine. Vaccine. 2004, 22(9-10):1275-80*).*

Several vaccine candidates against dengue are currently in different stages of development *(*Barrett, A.D. 2001. Current status of flavivirus vaccines. Ann. N. Y. Acad. Sci. 951:262-271. Chang GJ, Kuno G, Purdy DE, Davis BS. 2004 Recent advancement in flavivirus vaccine development. Expert Rev Vaccines. 2004 3(2):199-220 ). The strategies tried so far include attenuated live vaccines, chimeric viruses, plasmid DNA and subunit vaccines. Attenuated strains from the four serotypes have been developed using standard methodologies for viral propagation in primary kidney cells of dogs and monkeys *(*Bhamarapravati, N., and Sutee, Y. 2000. Live attenuated tetravalent dengue vaccine. Vaccine. 18: 44-47. Eckels, K.H., et al. 2003. Modification of dengue virus strains by passage in primary dog kidney cells: preparation of candidate vaccines and immunization of monkeys. Am. J. Trop. Med. Hyg. 69:12-16. Innis, B.L., and Eckels, K.H. 2003. Progress in development of a live-attenuated, tetravalent dengue virus vaccine by the United States Army Medical Research and Materiel Command. Am. J. Trop. Med. Hyg. 69:1-4). The advance of this strategy has been limited by the lack of animal models and *in vitro* markers of attenuation for humans. In this same research avenue, there is work in which cDNA clones have been obtained from the four serotypes and have then been treated to introduce attenuating mutations and variations that, in theory, greatly decrease the possibility of reversion to virulent phenotypes (Blaney, J.E, Jr., Manipon, G.G., Murphy, B.R., and Whitehead, S.S. 2003. Temperature sensitive mutations in the genes encoding the NS1, NS2A, NS3, and NS5 nonstructural proteins of dengue virus type 4 restrict replication in the brains of mice. Arch. Virol. 148:999-1006. Durbin, A.P., et al. 2001. Attenuation and immunogenicity in humans of a live dengue virus type-4 vaccine candidate with a 30 nucleotide deletion in its 3'-untranslated region. Am. J. Trop. Med. Hyg. 65:405-413. *Patent:* Zeng L, Markoff L, WO0014245, 1999*)* Another strategy has been the creation of chimeric flaviviral variants for the four serotypes, introducing the preM and E structural proteins from one dengue serotype into an attenuated background of Yellow Fever Virus (YFV), dengue or other virus that contribute the Core and other non-structural proteins *(*Guirkhoo F, Arroyo J, Pugachev KV et al. Construction, safety, and immunogenicity in non-human primates of a chimeric yellow fever-dengue virus tetravalent vaccine. J Virol 2001; 75: 7290-304. Huang CY, Butrapet S, Pierro DJ et al. Chimeric dengue type 2 (vaccine strain PDK-53)/dengue type 1 virus as a potential candidate dengue type 1 virus vaccine. J Virol 2000; 74: 3020-28. Markoff L, Pang X, Houng HS, et al. Derivation and characterization of a dengue 1 host-range restricted mutant virus that is attenuated and highly immunogenic in monkeys. J Virol 2002; 76: 3318-28. *Patent: Stockmair and schwan Hauesser,* W09813500, 1998. *Patent:* Clark and Elbing: WO9837911, 1998. *Patent:* Lai CJ, US6184024, 1994.*)*

In general, multiple questions persist about the potential benefits of live attenuated vaccines, given the possibility of occurrence of phenomena such as reversions to virulent phenotypes, viral interference and intergenomic recombination *(*Seligman SJ, Gould EA 2004 Live flavivirus vaccines: reasons for caution. Lancet. 363(9426):2073-5*).* The vaccines based on plasmid DNA expressing recombinant proteins are still in the early stages of development, as well as those based in recombinant antigens *(*Chang, G.J., Davis, B.S., Hunt, A.R., Holmes, D.A., and Kuno, G. 2001. Flavivirus DNA vaccines: current status and potential. Ann. N. Y. Acad. Sci. 951:272-285*.* Simmons, M., Murphy, G.S., Kochel, T., Raviprakash, K., and Hayes, C. G. 2001. Characterization of antibody responses to combinations of a dengue-2 DNA and dengue-2 recombinant subunit vaccine. Am. J. Trop. Med. Hyg. 65:420-426*. Patent: Hawaii Biotech Group, Inc;* WO9906068 1998*.* Feighny, R., Borrous, J. and Putnak R. Dengue type-2 virus envelope protein made using recombinant baculovirus protects mice against virus challenge. Am. J. Trop. Med. Hyg. 1994. 50(3). 322-328*;* Deubel, V., Staropoli, I., Megret, F., et al. Affinity-purified dengue-2 virus envelope glycoprotein induces neutralising antibodies and protective immunity in mice. Vaccine. 1997. 15, 1946-1954*)*

Several vaccine candidates based on the strategies described above have showed protection in animal models, and some have been found to be safe and immunogenic during the early stages of clinical trials.

The main hurdle for vaccine development, however, is the need for achieving equally effective protection against the four serotypes. It is agreed that an infection with one dengue serotype induces lifelong immunity against the same serotype in humans. However, immunizing against only one serotype achieves protection against other serotypes (heterotypic immunity) only for a short period of time ranging from 2 to 9 months *(*Sabin, A. B. 1952. Research on dengue during World War II. Am. J. Trop. Med. Hyg. 1:30-50*).* Besides, a suboptimal level of protection against a specific serotype might sensitize the vacinee and increase the risk of appearance of severe manifestations associated to a heterologous immune response of a pathological nature, upon later infection with that serotype *(*Rothman AL 2004 Dengue: defining protective versus pathologic immunity J. Clin. Invest 113:946-951*).* However, the development of effective tetravalent formulations of the available live attenuated or recombinant subunit vaccines has turned out to be a difficult challenge, requiring the use of complicated, multi-dose immunization schedules.

**Antibodies: Passive immunization**

One alternative to the use of vaccines for the prevention of dengue infection is the use of neutralizing antibodies for passive immunization. Humanized chimpanzee antibodies have been obtained for this purpose, including 5H2, which neutralizes dengue 4 (Men, R., T. Yamashiro, A. P. Goncalvez, C. Wernly, D. J. Schofield, S. U. Emerson, R. H. Purcell, and C. J. Lai. 2004. Identification of chimpanzee Fab fragments by repertoire cloning and production of a full-length humanized immunoglobulin G1 antibody that is highly efficient for neutralization of dengue type 4 virus. J. Virol. 78: 4665-4674), and 1A5, which is cross-neutralizing against the four serotypes (Goncalvez, A.P., R. Men, C. VIlernly, R. H. Purcell, and C.J. Lai. 2004. Chimpanzee Fab fragments and a derived humanized immunoglobulin G1 antibody that efficiently cross-neutralize dengue type 1 and type 2 viruses. J. Virol. 78: 12910-12918*).*

The use of passive immunization might be useful both for prophylactic and therapeutic means, taking into account that the level of viremia is an important predictor for the severity of the disease *(*Wang, W K. D. Y. Chao, C. L. Kao, H. C. Wu, Y. C. Liu, C. M. Li, S. C. Lin, J. H. Huang, and C. C. King. 2003. High levels of plasma dengue viral load during defervescence in patients with dengue haemorrhagic fever: implications for pathogenesis. Virology 305:330-338. Vaughn,D.W., Green, S., Kalayanarooj, S., Innis, B.L., Nimmannitya, S., Suntayakorn, S., Endy, T.P., Raengsakulrach, B., Rothman, A.L., Ennis, F.A. and Nisalak, A. Dengue Viremia Titer, Antibody Response Pattern, and Virus Serotype Correlate with Disease Severity. J. Infect. Dis. 2000;181:2-9*).* However, the administration of antibodies is not free of potential pitfalls. According to the antibody-dependant enhancement (ADE) theory, if the concentration of neutralizing antibodies decreases to subneutralizing levels, the virus-antibody immunocomplexes may amplify viral entry to the cells bearing Fc receptors, thus increasing the level of viral replication. Therefore, high antibody levels would be required to avoid endangering the patient for more severe manifestations of the disease.

One possible solution is the obtention of antibody molecules with an Fc modified in such a way that the interaction with its receptors is significantly decreased. In this sense, a particularly attractive strategy is the mutation of residues in the Fc that affect directly the interaction with FCγR-I, FCγR-II and FCγRIII but not with FCRn, since the latter is involved in antibody recycling and therefore is pivotal in determining the half-life of the antibody *in vivo.*

Another alternative is the identification of neutralizing antibodies incapable of mediating ADE. At least one antibody has been described with these characteristics *(Patent: Bavarian Nordic Res. Inst* WO9915692, 1998), which neutralizes DV2 without mediating ADE in an *in vitro* model. However, there are no descriptions of similar antibodies against other serotypes, and there is no available data on the characterization of this type of antibodies in *in vivo* models.

An additional obstacle is the fact that the available animal models do not reproduce faithfully the course and characteristics of the infection in humans.

Another strategy related to the use of antibodies is the obtention of bispecific complexes between anti-dengue and anti-erythrocyte complement receptor 1 antibodies. These heteropolymers would bind the virus to erythrocytes, therefore greatly increasing the rate of viral clearance from blood to tissues *(*Hahn CS, French OG, Foley P, Martin EN, Taylor RP. 2001. Bispecific monoclonal antibodies mediate binding of dengue virus to erythrocytes in a monkey model of passive viremia. J ImmunoL 2001 166:1057-65.*).*

**Detailed description of the invention**

The invention describes how to obtain effective molecules for prophylactic and/or therapeutic treatment against the four serotypes of Dengue Virus and other flaviviruses, by using an area or epitope in the surface of E-protein ('E' for Envelope), which is highly conserved in flaviviruses, as a target for said molecules. When used for vaccine design, the invention allows the generation of a neutralizing and protective effect, which is of similar magnitude for all four Dengue Virus serotypes, by circumscribing the antibody response to this region of E-protein and therefore eliminating responses against more variable regions of this protein, which can elicit serotype- or subcomplex-specific neutralizing antibodies that can lead to immunoamplification during later infection with other serotypes. Since the described area of E-protein is a topographic epitope, the invention includes the design of mutations and stabilizing connections to guarantee the correct folding and secretion of the E-protein subdomain that includes the aforementioned epitope. When used for the development of agents for passive immunization with prophylactic or therapeutic purposes, the invention also defines recombinant molecules capable of binding two, three or multiple symmetric copies of this epitope on the surface of mature flaviviral virions, said recombinant molecules having neutralizing and protective characteristics which are superior to those of natural antibodies and/or their FAb fragments due to their higher avidity and better potential for interfering with the structural changes undergone by the virions during the early stages of the viral replication cycle:

In a first embodiment, the invention describes the design of recombinant proteins that reproduce the antigenic and structural features of the E-protein epitope mentioned above. One of the described recombinant proteins is recognized by a mouse monoclonal antibody capable of neutralizing all four serotypes of Dengue Virus that also recognizes other flaviviruses. The immunization with this chimeric recombinant protein induces an antibody response that is neutralizing and protective against the four Dengue Virus serotypes, as well as other flaviviruses. The invention describes a method for designing the chimeric recombinant protein in such a way that the E-protein domain containing the common flaviviral neutralizing epitope folds correctly. This epitope is topographic in nature, and therefore its antigenicity is dependant upon the 3D structure of the molecule. The molecules obtained with this invention can be used in the pharmaceutical industry for the obtention of vaccine preparations against Dengue Virus and other flaviviruses, as well as for the design of diagnostic systems containing these proteins.

The second embodiment of this invention describes the design of other recombinant proteins with a potent neutralizing profile against the four serotypes of Dengue Virus and other flaviviruses. The aminoacid sequence of these proteins contains a binding domain, a spacer segment, and a multimerization domain. The binding domain is capable of binding to an epitope of the E protein that is highly conserved across all flaviviruses, which is contained in the proteins described on the first object of this invention, described above. In a variant of this embodiment, the binding domains are single-chain antibody fragments that recognize the conserved epitope. The spacer segments are sequences 3-20 aminoacids long, enriched in residues which are preferably hydrophilic, polar and with a small side chain, therefore conferring the spacer a high degree of mobility. These spacers must not interfere with the folding of the binding and multimerization domains, and must additionally be resistant to cleavage by serum proteases.

The multimerization domains described in the present invention are proteins or protein domains that associate in their native state preferably as dimers or trimers, although quaternary structures of higher order of association are not discarded. These domains are selected from human serum or extracellular proteins, so as to avoid the possible induction of autoantibodies. An essential property of the multimerization domains considered in this invention is the absence of any interactions with Fc receptors, which are involved in the antibody-mediated process of immunoamplification of Dengue Virus infections. The quaternary structure of the multimerization domain may depend on covalent or non-covalent interactions.

In one of the variants, the multimerization domain is based on the Fc fragment from human antibodies, including the hinge region since it mediates the formation of inter chain disulphide bridges that stabilize the dimeric structure. These Fc fragments are devoid of carbohydrate chains, either through chemical or enzymatic deglycosylation, or through their production on a host which does not glycosylate proteins, such as the bacterium *Escherichia coli.* The non-glycosylated Fc domains can also be obtained in cells from higher eukaryotes, provided that their sequence has been modified to remove the NXT/S motif. Non-glycosylated Fc domains can no longer bind to FcγR receptors I to III, which are mediators of immunoamplification *in vitro.* However, they remain fully competent for interacting with the FcRn receptor, which is a desirable property for obtaining a long half-life *in vivo.*

In another variant, the multimerization domain is a helicoidal, trimer-forming fragment of human matrilin.

The connection of the binding and multimerization domains through flexible spacers allows the simultaneous binding of the chimeric protein to multiple adjacent E-protein monomers on the icosahedral structure of flaviviral mature virions. This way, a sequence variant of [binding domain]-[spacer]-[multimerization domain] that yielded a dimeric protein would be able to bind simultaneously two E-protein monomers. Similarly, if the variant yields a trimeric protein, three monomers would be simultaneously bound.

The neutralization titer of the chimeric proteins described in the second embodiment of this invention is higher than that reached by FAb fragments and even complete antibodies. These recombinant proteins bind the virions with higher avidity, and the simultaneous engagement of several E monomers interferes with the necessary changes in quaternary structure during the process of membrane fusion. The molecules obtained with the practice of this invention can be used in the pharmaceutical industry for the obtention of prophylactic and/or therapeutic agents against Dengue Virus and other flaviviruses, as well as for the development of diagnostic systems containing said molecules.

**Design of chimerical proteins for vaccine purposes**

The currently accepted point of view is that an effective Dengue vaccine should induce a neutralizing antibody response against the four Dengue serotypes.

However, the viral envelope E-glycoprotein is variable among the serotypes. This sequence variability cause that the global antibody response against the protein is neutralizing against the homolog serotype but not against the heterolog serotypes, this way increasing the possibility of rising infection immune-enhancing antibodies.

The current invention describes a method aimed at designing subunit vaccines against Dengue virus, which induce an immune response uniformly neutralizing and protective against the four serotypes. At first place the design is based on the identification of patches or epitopes exposed at the surface of the protein, which conservation is total or very high among serotypes and are also exposed on the surface of the mature virions. Carrying out a residue conservation analysis on the protein, it was possible to identify a cluster of exposed and conserved residues. (figure 1 and 2, table 1).The total surface area of the cluster is 417 Å$^2$, belonging to 25 residues. This area is comparable with the typical values corresponding to the binding surface involved in protein-antibody interactions. The epitope is topographic, including residues located far apart on the primary structure of the protein, but close in the three dimensional structure.

At second place, the invention describes the design of recombinant chimerical proteins which contain the conserved epitope, maximizing the ratio between conserved/variable residues presented to the immune system and achieving the stabilization of the three dimensional structure of the epitope in a similar way as it appears in the context of the whole E-protein. Two possible topologies are described:

B-L-C and C-L-B,

where B is the segment Leu237-Va1252 and C is the segment Lys64-Thr120 of the E-glycoprotein from dengue 2 or the homolog segments from the other serotypes or other flavivirus, or similar protein sequences displaying more than 80% of sequence identity with respect to any of the above mentioned segments. The homolog segments B and C corresponding to flavivirus sequences are defined by the use of sequence alignment computer programs, which align pair of sequences or multiple sequences such like BLAST, FASTA y CLUSTAL *(*Altschul, S.F., Gish, W., Miller, W., Myers, E.W & Lipman, D.J. 1990, Basic local alignment search tool. J. Mol. Biol. 215: 403-490*.* Pearson WR, Lipman DJ. Improved tools for biological sequence comparison. Proc Natl Acad Sci U S A. 1988;85:2444-8. Higgins D., Thompson J., Gibson T. Thompson J. D., Higgins D. G., Gibson T. J. 1994; CLUSTAL W: improving the sensitivity of progressive multiple sequence alignment through sequence weighting,position-specific gap penalties and weight matrix choice. Nucleic Acids Res. 22:4673-4680*).* These sequence alignments allow also define homolog residues (also referred as equivalent or corresponding residues) in the sequence of flavivirus, which correspond to the highly conserved residues identified in table 1 of the example 1 for the particular case of the sequence from dengue 2.

For both described topologies, L are linker sequences with a size of typically between 1 and 10 residues, whose role is to connect segments B and C in a stabilizing manner regarding the folding of the chimerical protein and allowing the 3D structure of the epitope to be similar to the structure displayed in the context of the whole E-protein. In both topological variants of the chimerical protein, the conserved epitope is completely included, excluding the rest of the E-protein which is more variable. The chimerical protein represents a sub-domain of the structural domain II of the envelope glycoprotein. This sub-domain is located at the tip of domain II and is structurally conformed by two anti-parallel beta sheets, packed against each other.

The major beta sheet is composed by three beta strands (segment C) and the minor is a beta hair pin loop (segment B).

The sub-domain contains two disulfide bridges and it is connected to the rest of the E-glycoprotein through four points, which is consistent with the topographic nature of the conserved epitope. However, the contact surface between the sub-domain and the rest of the protein is 184 Å$^2$, which represents only the 12% of the total solvent accessible surface of the sub-domain. This fact is consistent with the feasibility to achieve the correct folding of the sub-domain by designing stabilizing connections or linkers as described above for the two topological variants. The invention includes the possibility of increasing the thermodynamic stability of the chimerical protein by means of mutations in residues which are not accessible to the virion surface and hence not involved in the interaction with antibodies.

An essential novelty of the present invention is the idea that it is possible to develop a subunit vaccine based on a unique protein chain, which is effective against the four Dengue serotypes. The current approaches based on recombinant protein candidates consist on the use of four recombinant envelope proteins, one for each serotype, which are combined in a vaccine formulation (*Patente: Hawaii Biotech Group, Inc;* WO9906068 1998). E-protein fragments have also been evaluated as possible candidates, but till now the efforts have been focused on domain III, expressed as fusion proteins with carrier proteins (*Patente: Centro de Ingenieria Genética y Biotecnología;* WO/2003/008571. Simmons M, Murphy GS, Hayes CG. Short report. Antibody responses of mice immunized with a tetravalent dengue recombinant protein subunit vaccine. Am J Trop Med Hyg. 2001; 65:159-61. Hermida L, Rodriguez R, Lazo L, Silva R, Zulueta A, Chinea G, Lopez C, Guzman MG, Guillen G. A dengue-2 Envelope fragment inserted within the structure of the P64k meningococcal protein carrier enables a functional immune response against the virus in mice. J Virol Methods. 2004 Jan;115(1):41-9*).* Domain III is able to induce a neutralizing antibody response, but this response is serotype specific and therefore a vaccine candidate should include sequences from the four serotypes.

The chimerical protein PMEC1 of the example 1 of the present invention corresponds to the topology B-L-C, with sequences of the fragment B and C from dengue 2 and a two residues Gly-Gly linker sequence. It is also described a gene which codifies for the chimerical protein PMEC1. The plasmid pET-sPMEC1-His6 codify for the protein PMEC1 fused at the N-terminus to the signal peptide pe/B and at the C-terminus to a sequence codifying for six histidines (Sequence No. 12).

The chimerical protein PMEC1 was obtained soluble in the periplasm of the bacteria *E. coli*. An easily scalable purification process was developed based on metal chelates chromatography (IMAC), which allowed obtaining pure protein preparations suitable for further studies. The purified protein was analyzed by mass spectrometry and the obtained mass/z signal corresponds to the theoretical valued calculated from the amino acid sequence of PMEC1, assuming the formation of two disulfide bridges. The protein PMEC1 shows a strong recognition by hyper-immune ascitic fluids obtained against the four Dengue virus serotypes and by the mAb 4G2. This recognition depends on the correct formation of the disulfide bridges, suggesting that the protein PMEC1 has a conformation similar to the one adopted by the corresponding region of the native E-protein.

By immunizing mice with the chimerical protein PMEC1, it was obtained a neutralizing and protective response, characterized by high titers against the four Dengue serotypes.

An IHA assay was also performed and positive titers were obtained against the four serotypes. Titers of 1:1280 against the four serotypes were obtained in the *in vitro* neutralization test. Finally, a protection assay was carried out in mice, showing protection in 80-90% of animals against the four serotypes.

**Modeling the complex formed by mAb 4G2 and the E-protein**

The example No. 8 shows the modeling of the structure of the complex formed by mAb 4G2 and the E-protein. This antibody recognizes and neutralizes the four Dengue serotypes and other flavivirus.

The model was obtained using the CLUSPRO protein-protein docking method (http://structure.bu.edu/Projects/PPDocking/cluspro.html). In the study, the crystallographic structure of FAb 4G2 (PDB file 1uyw) and the PDB files loan and loam corresponding to the dimeric structure of E-protein from dengue 2 were used as input files. The table 8 shows the values corresponding to the characteristic surface parameters of the E-protein-Fab interface, the values calculated for the modeled complex are similar to the typical values obtained for protein-antibody complexes which crystallographic structure has been solved (table 9).

The obtained model indicates that the epitope recognized by mAb 4G2, includes the highly conserved region identified in this invention. The table 1 shows the set of residues conforming the predicted structural epitope (residues making contacts with the antibody) and those residues belonging to the highly conserved surface patch. According to the model, the 71% of residues from the predicted structural epitope, belong to the highly conserved area.

Later, a model of the complex in the context of the mature virion was obtained by docking the previously predicted model on the cryo-electron microscopy structure of dengue 2 virus. This way, a model was obtained where all epitopes (180 copies) recognized by mAb 4G2 on the virion surface are occupied by FAb chains.

The inter-atomic distance between the C-terminus of the heavy chains corresponding to those FAbs bound to E-protein dimers is 100 Å. The same distance calculated for FAbs bound to monomers of the asymmetric unit, which are not associated as dimers, is 120 and 80 Å.

These distances are not stereo-chemically compatibles with the sequence and structure of the IgG molecule, suggesting that mAb 4G2 binds to the virus in a monovalent way.

This prediction is supported by the results shown in the example 12, which indicate that the FAb and the mAb 4G2 have very similar neutralizing titers. This finding contrast with data obtained for other antiviral antibodies, whose divalent binding causes an increase in the neutralizing capacity of 2-3 orders of magnitude *(*Drew PD, Moss MT, Pasieka TJ, Grose C, Harris WJ, Porter AJ. Multimeric humanized varicella-zoster virus antibody fragments to gH neutralize virus while monomeric fragments do not. J Gen Virol. 2001; 82:1959-63. Lantto J, Fletcher JM, Ohlin M. A divalent antibody format is required for neutralization of human cytomegalovirus via antigenic domain 2 on glycoprotein B. J Gen Virol. 2002; 83: 2001-5).

This property of the mAb 4G2 could be common to various antiflavivirus antibodies, as is the case for the chimpanzee antibody 1A5, which recognizes an epitope located also in domain A of the E-protein *(*Goncalvez AP, Men R, Wernly C, Purcell RH, Lai CJ. Chimpanzee Fab fragments and a derived humanized immunoglobulin G1 antibody that efficiently cross-neutralize dengue type 1 and type 2 viruses. J Virol. 2004; 78: 12910-8). In general, the balance between the neutralizing capacity of the mAb and its FAb, depends on the epitope, the identity of the antibody and the stereochemical details of the complex. Accordingly, the mAb 4E11 which recognizes an epitope located on domain B, is 50 times more neutralizing that its corresponding FAb (Thullier, P., P. Lafaye, F. Megret, V. Deubel, A. Jouan, and J. C. Mazie. 1999. A recombinant Fab neutralizes dengue virus in vitro. J. Biotechnol. 69:183-190*).*

**Design of multivalent neutralizing molecules**

The current invention describes the design and development of molecules capable to bind simultaneously two or three copies of the highly conserved epitope on the virion surface. The virion exposes a total of 180 copies of the conserved epitope described in the present invention. They could be grouped as 90 pairs of epitopes corresponding to E-protein dimers or 60 triplets matching the three copies of E-protein present in the asymmetric unit of the virion. The herein described molecules are capable of divalent or trivalent binding and display an improved binding affinity for the virion and a neutralizing capacity which is various order more potent compared to the neutralizing antibodies recognizing the conserved epitope described in this invention. The described molecules neutralize the four Dengue virus serotypes and other flavivirus and therefore are useful for the prophylactic and/or therapeutic treatment of Dengue and alternatively of other flavivirus.

The sequences of the divalent or trivalent protein molecules of the present invention are described by the following formula:

[S]-[L]-[D] or [S]-[L]-[T],

where [S] is the sequence of a single chain antibody fragment (scFv), which recognizes the conserved epitope described in this invention, [L] is a linker sequence of size typically between 3-20 amino acids, [D] is the sequence of a protein or its fragment which forms dimers and [T] is a protein or its fragment which forms trimers. The segments [D] and [T] are proteins or protein domains which do not interact with FC receptors capable of mediate immune-enhancement of the viral infection. This way, it is possible to prevent the enhancement of the viral infection of FC receptor bearing cells at sub-neutralizing concentrations of divalent or trivalent molecules of the present invention. Therefore, the described molecules are superior compared to antibodies regarding their incapability to mediate an ADE like effect.

Furthermore, these molecules have a larger size compared to the scFvs and hence display a larger half time of life *in vivo.*

The sequences [D] and [T] correspond to extra-cellular human proteins, preferably from serum. This way it is possible to prevent the induction of an autoantibody response that would appear against intra-cellular and/or foreign proteins.

In general, the domains [D] and [T] could be replaced by multimerization domains capable of forming larger oligomers, if suitable linker sequences are chosen which allow multivalent binding to occur.

The designed multimerization (including dimerization and trimerization) allows increasing the avidity of the fragments and improving their intrinsic capacity of neutralization. Virus binding at multiple points further stabilizes the structure of the mature virion, which interferes with the changes in quaternary structure associated to the membrane fusion process. Moreover, the increase in the molecular size causes a raise in the half time of life *in vivo.* These recombinant proteins, which include antibody Fv fragments, could become effective therapeutic and/or prophylactic agents for the control of epidemic outbreaks.

The current invention describes a gene which codifies for a chimerical protein named TB4G2. The plasmid pET-TB4G2-LH codifies for the protein TB4G2 fused at the N-terminus to the signal peptide pe/B and at the C-terminus to a sequence codifying for 6 histidines (Sequence No. 16).

The chimerical protein TB4G2 contains the following elements from the N- to the C-terminus: (a) the variable domain of the light chain of mAb 4G2 (Sequence No. 25), (b) a flexible spacer sequence (Sequence No. 26), (c) the variable domain of the heavy chain of mAb 4G2 (Sequence No. 27), (d) a flexible spacer sequence of 15 residues (Sequence No. 28), (e) a fragment of human matrilin, which allows the molecule to trimerize in solution (Sequence No. 51).

The chimerical protein TB4G2 corresponds to the topological variant [S]-[L]-[T], where [S] is a scFv fragment of mAb 4G2, [L] is a spacer sequence of 15 residues composed by GLY and SER residues, and [T] is a trimerization domain of human matrilin which forms a helical coiled-coil trimeric structure with the alpha helices aligned in a parallel conformation (Dames SA, Kammerer RA, Wiltscheck R, Engel J, Alexandrescu AT. NMR structure of a parallel homotrimeric coiled coil. Nat Struct Biol. 9998; 5: 687-91).

This matrilin fragment forms covalent trimers stabilized by disulfide bridges formed between cysteins located at the N-terminus of the helix. The signal peptide pe/B allows the periplasmic location of the protein TB4G2 and hence its correct folding in *vivo,* which includes the correct formation of disulfide bridges of the binding domain and the trimerization domain.

According to the models of the complex formed between the virion and the Fv 4G2, the distances measured between the C-terminus of the Fv heavy chains corresponding to Fv fragment bound to the three E-protein monomers of the asymmetric unit are 36, 58 and 70 Å. These three C-terminal atoms are circumscribed in a sphere with a radius of 35 Å, which indicates that the spacer segment [L] must adopt conformations compatible with this distance.

In theory, a segment of 15 residues adopting an extended conformation has a dimension of 52 Å from the N- to the C-terminus. However, such conformation is not necessarily the most stable and in general the structural properties of peptides are determined by their sequences. Peptides rich in GLY and SER are essentially flexible, and are able to adopt multiple conformations in solution. As shown in the example 9, the prediction of peptide conformation using PRELUDE *(*Rooman MJ, Kocher JP, Wodak SJ. Prediction of protein backbone conformation based on seven structure assignments. Intluence of local interactions.J Mol Biol. 1991; 221:961-79*)* indicates that the most favorable conformation predicted for the sequence [L] (Sequence No 28) correspond to a distance between the N- and C-terminus of about 35 Å. This finding point out that the design of the chimerical protein TB4G2 is structurally compatible with the capacity of achieving a simultaneous binding to the three E-protein monomers present in the asymmetric unit of the virion.

The chimerical protein TB4G2 was obtained in soluble form in the periplasm of the bacteria *E. coli.* An easily scalable purification process was developed based on metal chelates chromatography (IMAC), which allowed obtaining pure protein preparations. The purified protein was analyzed by SDS-PAGE electrophoresis. The protein TB4G2 previously treated under reductive condition migrates to a band corresponding to the mass of a monomer and to a trimer when treated under not reductive condition.

Finally, in order to compare the neutralizing capacity of protein TB4G2 with respect to the mAb 4G2 and its fragments FAb and $(FAb')_2$, a neutralization test was carried out against the four Dengue virus serotypes in BHK-21 cells. The protein TB4G2 showed similar neutralization titers against the four serotypes, which are two-three orders more potent compared with the antibody and its fragments.

The present invention describes a gene (Sequence No 17), which codifies for a chimerical protein named MA4G2. The chimerical protein MA4G2 (Sequence No 56) contains the following elements from the N-terminus to the C-terminus: (a) the variable domain of the light chain of mAb 4G2 (Sequence No. 25), (b) a flexible spacer sequence (Sequence No. 26), (c) the variable domain of the heavy chain of mAb 4G2 (Sequence No. 27), (d) a flexible spacer sequence of 3 residues (Gly-Gly-Gly), (e) the hinge segment, the CH2 and the CH3 domains of the human IgG1 immunoglobulin molecule. In the CH2 domain of the human IgG1, the protein has been mutated in position ASN297->GLN.

The chimerical protein MA4G2 corresponds to the topological variant [S]-[L]-[D], defined in the present invention, where [S] is a single chain scFv fragment of mAb 4G2, [L] is a three residues spacer segment of sequence GLY-GLY-GLY and [D] is a segment containing the hinge segment, the CH2 and the CH3 domains of the human IgG1 immunoglobulin molecule. The hinge segment mediates the formation of intermolecular disulfide bridges between two identical protein chains, resulting in a stable dimeric structure. The mutation ASN297->GLN in the CH2 domain of the human IgG1 prevents the glycosilation of the protein in Eucariotes and precludes the binding to the FcyR I-III. These receptors mediate the ADE phenomena *in vitro.* This way, unlikely the mAb 4G2, the designed chimerical protein lacks the risks associated to ADE at sub-neutralizing concentrations. However, the chimerical protein retains the capacity of interacting with the FcRn receptor, a property favorable to achieve longer half time of live *in vivo,* in a similar manner to the antibody molecules.

The plasmid pET-MA4G2-LH (Sequence No 20) codifies for the protein MA4G2 fused at the N-terminus to the signal peptide *pel*B (Sequence No 24) and at the C-terminus to a tail of 6 Histidines. The signal peptide pelB allows the localization of the protein MA4G2 in the periplasm, where occurs the correct formation of the intramolecular disulfide bridges (binding domains, CH2 and CH3) and between the hinge-segments (intermolecular bridges). The histidine tail allows the purification of the protein by metal chelates chromatography.

The 3D model of the complex formed by the protein MA4G2 and the E-protein dimers (example 9), as well as the results of the neutralization tests (example 12) indicate that the chimerical protein MA4G2 is stereo-chemically compatible with a simultaneous binding to the monomers associated as dimers in the structure of the mature virions. This way, bivalency results in a significant increase of the biological activity of the protein.

An essential aspect of the present invention consists in the finding that molecules capable of binding to the herein described highly conserved surface patch of the E-protein, interfere with the biological function of this protein, and such molecules constitute potential candidates for antiviral agents of wide spectrum against flavivirus. As shown in the example 12, fragments of mAb 4G2 including the scFv display a neutralizing activity similar to the whole mAb 4G2, indicating that bivalency is not required for the antiviral activity. These results also show that the antiviral activity of the fragments depends on the interference with the biological activity of the E-protein and this interference is mediated by binding to the described highly conserved area of the protein. Furthermore, the observed antiviral activity is of wide spectrum against flavivirus. Therefore, attractive methods for the identification of antiviral molecules with these properties are those which allow identifying proteins, peptides and drug-like molecules which bind to the described highly conserved surface area. Such methods are those based in blocking the binding to the E-protein of those antibodies which recognize the highly conserved surface area, like the mAb 4G2, its corresponding FAb and (Fab')2 fragments or the chimerical proteins described in the present invention. Those methods could be based on immune-enzymatic assays, radio-immune assays, assays with fluorescent dyes and these assays allows quantifying the binding of molecules to the E-protein, virions or the herein described chimerical proteins which display the highly conserved surface area.

These assays could be useful to identify potential antiviral molecules effective against a wide spectrum of flavivirus, by means of in vitro screening of libraries of chemical compounds including those generated by methods of combinatorial chemistry.

The identification of candidate molecules could be carried out using computer aided virtual screening methods. These methods are based on computational procedures like the molecular docking of chemical compounds. Using these methods, it is possible to model the binding of chemical compounds to proteins and to quantify the interaction strength or binding energy, which is predicted or calculated from the modeled complex coordinates by means of scoring functions.

Examples of these computational procedures of molecular docking are the programs GOLD *(*Jones, G. y cols., 1997. Development and validation of a genetic algorithm for flexible docking. J. Mol. Biol. 267, 727-748), DOCK (Kuntz, I.D. y cols., 1982 A geometric approach to macromolecule-ligand interactions. J. Mol. Biol. 161, 269-288) and FLEXX *(*Olender, R. and Rosenfeld, R., 2001. A fast algorithm for searching for molecules containing a pharmacophore in very large virtual combinatorial libraries. J. Chem. Inf. Comput. Sci. 41, 731-738*)*. These methods allow large virtual libraries of molecules like ZINC database *(*Irwing, J.J. and Scoichet, B.K., 2005. Zinc - A free Database of commercially available compounds for virtual screening. J. Chem. Inf. Model. 45, 177-182*)* to be screened and determine which molecules are expected to bind the active site selected on the receptor protein. Regarding the present invention, the binding site is the previously described highly conserved surface area. The crystallographic structures of E-protein available in the PDB database could be used as source for atomic coordinates, or alternatively computational models could be used, which are obtained by means of methods like protein modeling by homology.

**DESCRIPTION OF THE FIGURES.**

**Figure 1.** Graphic representation of the conservation profile corresponding to the surface residues of the flaviviral E-protein. Conservation is represented in a grey scale basis, residues showing more conservation among the flavivirus sequences are darker. The highly conserved surface patch of domain II is encircled. The conservation values were calculated using the program CONSURF, considering a multiple sequence alignment of those flavivirus sequences available in SWISSPROT database. The conservation values were mapped on the protein surface using Pymol.

**Figure 2.** Graphic representation of the conservation profile corresponding to the surface residues of the E-protein from Dengue virus. Conservation is represented in a grey scale basis, residues showing more conservation among the flavivirus sequences are darker. The highly conserved surface patch of domain II is encircled. The conservation values were calculated using the program CONSURF, considering a multiple sequence alignment of sequences corresponding to the four Dengue virus serotypes which are available in SWISSPROT database. The conservation values were mapped on the protein surface using Pymol.

**Figure 3**. Model of the three dimensional structure of the chimerical protein PMEC1. B is the segment Leu237-Va1252 and C is the segment Lys64-Thr120 of the E-glycoprotein of Dengue 2 virus. L is the linker segment consisting of two residues. The 3D model of the protein was obtained using the WHATIF program package and the graphic was made using Pymol.

**Figure 4.** Plasmid pET-sPMEC1.

**Figure 5.** Plasmid pET-scFv 4G2 LH.

**Figure 6A.** Plasmid pET-TB4G2 LH.

**Figure 6B.** Plasmid pET-MA4G2 LH.

**Figure 7.** Physicochemical characterization of the chimerical protein PMEC1-His6. A: SDS-PAGE electrophoresis of the protein purified by immobilized metal affinity chromatography (lane 1) and reduced and carbamidomethylated (lane 2). B: RP-C4 reversed phase chromatographic analysis of the protein, the arrow indicates the location of the major peak. C: Mass spectra corresponding to the major peak collected by reversed-phase chromatography.

**Figure 8.** Summarizing scheme of the results obtained in 13 computational simulation of molecular docking (using the CLUSPRO program) preformed in order to predict the structure of the complex formed by the Fv fragment of the mAb 4G2 and the E-protein from dengue 2. The columns show in a grey scale basis the structural properties of the first 30 solutions (clusters) obtained in each simulation. The solutions are represented by three properties. The first property shows the E-protein domain involved in binding, from lighter to darker gray corresponds to domain II, I and III respectively. Two colors mean simultaneous binding to two domains. L and T means that the epitope involves the linker connecting domains I and III or the fusion peptide respectively (tip of domain II). The second property is represented by three colors, white means binding to the inner surface of the virion, gray is a lateral binding and black means binding to the outer surface of the virion. The third case is the biologically relevant assuming that antibody binding does not depend on major structural changes of the virion structure. The third property correspond to the antibody paratope, gray means that binding involves the antibody CDRs (relevant solutions), white indicates that binding does not involves CDRs (irrelevant solutions). The solutions compatible with the available experimental data are shown using arrows. Their properties correspond to the colors light gray - black - gray. The first two rows located at the top of the graphic indicates the definition of ligand and receptor used in the simulations and includes the PDBfile identifier corresponding to the E-protein crystal structure used in the simulation. The protein-protein docking program (dot or zdock) used in the each simulation is shown below every column.

**figure 9.** Modeling the complex formed between the mature virion from Dengue 2 virus and 180 copies of the FAb 4G2. The model was obtained by docking the previously predicted structure of the FAb4G2-E-protein complex into the structure of the mature virion obtained by cryoelectron micrscopy (1THD). The distances calculated between the C-terminus of the heavy chains of the FAbs bound to three monomers of E-protein found in the asymmetric unit.

**Figura 10.** Computer model of the complex formed by the chimerical protein MA4G2 and the E-protein dimer. The figure was obtained using the program Pymol.

**Figura 11.** Prediction of conformer stability corresponding to the peptide sequence $(GGGS)_3$GGG. Energy of conformers is shown as a function of the distance between the N- and the C-terminus. The prediction was performed using the program PRELUDE.

## EXAMPLES

### EXAMPLE No. 1 - Design of the chimerical protein PMEC.

With the aim of identifying conserved patches on the surface of the E-protein, a conservation analysis was carried out using the CUNSURF method (*ConSurf*: *identification of functional regions in proteins by surface-mapping of phylogenetic information.* Glaser, F., Pupko, T., Paz, I., Bell, R.E., Bechor-Shental, D., Martz, E. and Ben-Tal, N.; 2003; Bioinformatics 19: 163-164). A highly conserved surface patch is observed on the tip of domain II, which is conserved among the four Dengue virus serotypes and the rest of flavivirus (figure 1 and 2).

The highly conserved surface patch defines a topographic epitope, conformed by residues located close in the three dimensional structure but distant in the sequence of the E-protein. This surface area is comprised on a structural sub-domain located at the extreme of domain II and it is conformed by two lineal segments of E-protein, Leu237-Val252 (segment B) and Lys64-Thr120 (segment C). The table 1 shows the list of residues of the sub-domain, which are located on the outer surface of the virion and hence accessible to the interaction with antibodies. Highly conserved residues define the area or epitope identified by this invention.

The inspection of the structure of domain II of E-protein, indicates that the sub-domain presents structurally independent domains like properties. The contact surface to the rest of the protein is 184 $Å^2$, which represents only the 12% of the total solvent accessible surface area of the sub-domain. Moreover, this portion of the structure is defined as a structural domain in the CATH database (CATH domain 1 svb03, *http://www.biochem.ucl.ac.uk/bsm/cath/cath.html).*

**Table 1.** Definition of relevant residues of the present invention, which are located on the extreme of domain II of E-protein and exposed on the virion surface.

| AA | No. E DEN -2 | No. PMEC1 | ACC (A2) | CONS | epitope |
|---|---|---|---|---|---|
| HIS | 244 | 8 | 40.8 | **-1.074/-0.792** | |
| LYS | 246 | 10 | 43 | -0.539/**-0.792** | X |
| LYS | 247 | 11 | 41.2 | **-0.667**/-0.334 | X |
| GLN | 248 | 12 | 4 | **-0.7021-0.792** | X |
| ASP | 249 | 13 | 19.5 | 0.366/-0.298 | X |
| VAL | 251 | 15 | 13.8 | 0.726/0.835 | X |
| VAL | 252 | 16 | 11.7 | **-0.724/-0.792** | X |
| LYS | 64 | 19 | 26.5 | 0.426/0.271 | X |
| LEU | 65 | 20 | 9.3 | -0.335/-0.383 | X |
| THR | 66 | 21 | 11.7 | 0.210/-0.152 | X |
| ASN* | 67 | 22 | 30.2 | 0.417/**-0.792** | X |
| THR | 68 | 23 | 22.3 | 0.952/1.062 | X |
| THR | 69 | 24 | 14.6 | **-0.745/-0.792** | X |
| THR | 70 | 25 | 23.7 | **-0.781/-0.792** | X |
| GLU | 71 | 26 | 13.3 | 2.146/2.661 | X |
| SER | 72 | 27 | 12.4 | -0.431/-0.492 | X |
| ARG | 73 | 28 | 21.5 | -0.284/-0.792 | X |
| CYS | 74 | 29 | 12.7 | **-1.074/-0.792** | X |
| LEU | 82 | 37 | 6.2 | **-0.811/-0.792** | X |
| ASN | 83 | 38 | 39.4 | 4.302/2.327 | X |
| GLU | 84 | 39 | 11.2 | **-0.677/-0.792** | X |
| GLU | 85 | 40 | 17 | **-0.861/-0.792** | |
| ASP | 87 | 42 | 11.7 | **-0.486/-0.792** | |
| ARG | 89 | 44 | 30.8 | 2.051/0.179 | |
| PHE | 90 | 45 | 6.4 | 2.777/4.283 | X |
| VAL | 97 | 52 | 1.7 | **-0.766/-0.792** | X |
| ARG | 99 | 54 | 10.5 | **-0.898/-0.792** | X |
| GLY | 100 | 55 | 1.3 | **-0.796/-0.792** | X |
| TRP | 101 | 56 | 15.4 | **-1.074/-0.792** | X |
| GLY | 102 | 57 | 14.8 | **-1.074/-0.792** | X |
| ASN | 103 | 58 | 21.7 | **-1.074/-0.792** | X |
| GLY | 104 | 59 | 18 | **-0.775/-0.792** | X |
| CYS | 105 | 60 | 1.8 | **-1.074/-0.792** | X |
| GLY | 106 | 61 | 16.3 | **-1.074/-0.792** | X |
| MET | 118 | 73 | 13.6 | **-0.877/-0.349** | X |

**AA:** amino acid, **No. E DEN2:** number of the residue in the sequence of E-protein from dengue 2, **No. PMCE1:** number of the residue in the sequence of the chimerical protein PMEC1, **ACC:** Solvent accessible surface area calculated with WHATIF (Vriend G. WHAT IF. a molecular modeling and drug design program. J Mol Graph. 1990; 8: 52-6, 29). Calculations were performed on an atomic model of E-protein, which was obtained by docking independently the 3D structure of the structural domains I, II and III (PDB file loan) on the structure of the mature virion (PDB file 1THD). **CONS:** Conservation scores calculated with CONSURF, using two sequence alignments, taking into account flavivirus sequences and sequences from the four dengue virus serotypes respectively. Negative values indicate higher conservation and bold highlight the values corresponding to the residues defined as highly conserved, **epitope**: Residues making contacts to FAb 4G2 according to the 3D model obtained by molecular docking in the Example 8. Those residues are considered which have at least one atom whose van der waals sphere is separated by less than 3 A from the van der waals sphere of an atom of FAb 4G2, *ASN22 glycosilated in DEN2 virus.

In order to obtain an independently folded sub-domain, it is necessary in first place to connect the two segments in a unique polypeptide chain. Two possible connections or topologies are possible:

B-L-C y C-L-B

where L is a linker or spacer segment. The linker needs to be stereo-chemically compatible with the three dimensional structure of the sub-domain and in the best case provide a stabilizing effect on the thermodynamic stability of the chimerical protein. The distance between the alpha carbons of residues Val252 and Lys64 is 6.6 Å, therefore the topology B-L-C could be obtained by the use of linkers of one or more residues. An structural analysis of possible connecting turns on the PDB data base, searching for turns compatible with the structure of the anchor segments (DGINS command in the DGLOOP menu of WHATIF program package) indicates that connections of two residues are more common than connections of one residue. In the case of the topology C-L-B, the distance between the alpha carbons of the residues Thr120 and Leu237 is 11.1 Å, consistent with connections of 3-4 residues or more.

The chimerical protein PMEC1 (sequence 14) of the present invention corresponds to a topology B-L-C, with fragment B and C corresponding to sequences from dengue 2 virus and a two residues Gly-Gly linker sequence.

As B and C segment sequences could be chosen not only the sequences corresponding to DEN2 virus, but also the homolog sequences from other flavivirus, including but not limiting DEN1, DEN3, DEN4, Japanese Encephalitis virus, Tick-born Encephalitis virus, West Nile virus, Murray Valley Encephalitis virus, St Louis Encephalitis virus, LANGAT virus, Yellow Fever virus, Powassan virus (sequences 29-42).

Moreover, the chimerical proteins designed according to the method described above, could be mutated at one or multiple residues, with the aim to increase the thermodynamic stability of the protein or the efficiency of folding process. Those residues described in table 1, which are not accessible to the virion surface and to the interaction with antibodies, could be mutated. The residues susceptible to be mutated are those residues which are buried on the 3D structure of the protein and/or are located in the lateral or inner surface of the 3D/4D structure of the E-protein present in the mature virion.

The mutated protein could be obtained by experimental combinatorial methods like the filamentous phage libraries. The proteins could also be designed using theoretical methods like FOLDX, POPMUSIC and Rosseta.

The sequences 43-50 correspond to analogs of the chimerical protein PMEC1 mutated at multiple positions. Three dimensional models of this proteins show a good packing and quality. Mutations at the exposed surface of the protein are also possible, especially at residues which are not strictly conserved among the Dengue virus serotypes and other flavivirus, with the condition that these mutations must not affect the interaction with protective and neutralizing antibodies recognizing the conserved sub-domain of E-protein.

**EXAMPLE No. 2 - Construction of plasmid pET-sPMEC1**

In order to obtain a recombinant gene coding for the protein PMEC1 (Sequence No. 1), the gene coding for protein E from the DEN2 virus (Sequence No. 2), strain 1409, genotype Jamaica, present on plasmid p30-VD2 *(*Deubel V., Kinney R.M., Trent D.W; "Nucleotide sequence and deduced amino acid sequence of the structural proteins of dengue type 2 virus, Jamaica genotype", Virology 155(2):365-377, 1986**)** was used. This gene codes for the protein shown in Sequence No. 3. Using the method of Agarwal *et al.. (*Agarwal KL, Büchi H, Caruthers MH, Gupta N, Khorana HG, Kumas A, Ohtsuka E, Rajbhandary UL, van de Sande JH, Sgaramella V, Weber H, Yamada T, Total synthesis of the Gene for an alanine transfer ribonucleic acid from yeast, 1970, Nature 227, 27-34*),* and starting from oligonucleotides synthesized on solid phase by phosphoramidite chemistry *(*Beaucage SL, Caruthers MH, Deoxynucleoside phosphoramidites- A new class of key intermediates for deoxypolynucleotide synthesis., Tetrahedron Letters, 1981, 22, 1859), a double stranded DNA molecule coding for the PMEC1 protein was obtained (Sequence No. 4). The sequence of this DNA molecule has the following elements: 1) A recognition site for the *Nco* I restriction enzyme, containing the start codon coding for the aminoacid methionine (M), followed by a codon coding for the aminoacid Alanine (A) (Sequence No. 5); 2) A fragment corresponding to the sequence, from position 709 to position 756, of the gene for protein E of virus Dengue 2 strain Jamaica 1409 (Sequence No. 6), coding for the peptide sequence shown in Sequence No. 7, that in turn corresponds to positions 237 to 252 of Sequence No. 3; 3) A linker segment coding for two successive Glycines (Sequence No. 8); 4) A fragment corresponding to the sequence spanned by positions 190 to 360 of Sequence No. 2, which codes for the peptide sequence shown in Sequence No. 9 (which corresponds to positions 64-120 of Sequence No. 3), where a silent mutation has been introduced that eliminates the *Nco* I restriction site present in positions 284-289 of said sequence (Sequence No. 10); and 5) A recognition site for the *Xho* I restriction enzyme, containing two codons that code for the aminoacids Leucine (L) and Glutamic acid (E), respectively (Sequence No. 11). This synthetic molecule was digested with the *Nco* I and *Xho* I restriction enzymes (Proimega Benelux b.v., The Netherlands) in the conditions specified by the manufacturer, and ligated using T4 DNA ligase (Promega Benelux, b.v., The Netherlands), in the conditions specified by the manufacturer, to plasmid pET22b (Novagen, Inc., USA) previously digested identically. The reaction was transformed into the *Escherichia coli* strain XL-1 Blue *(*Bullock WO, Femández JM, Short JM. XL-1Blue:

A high efficiency plasmid transforming recA Escherichia coli K12 strain with beta-galactosidase selection. Biotechniques 1987;5:376-8*)* according to Sambrook *et al. (*Sambrook J, Fritsch EF, Maniatis T. Molecular cloning: A laboratory manual. New York, USA: Cold Spring Harbor Laboratory Press; 1989), and the plasmids present in the surviving colonies in selective medium were screened by restriction analysis. One of the resulting recombinant plasmids was denominated pET-sPMEC1 (Figure 4), and its sequence (Sequence No. 12) was verified through automatic Sanger sequencing.

The plasmid pET-sPMEC1 codes for the protein PMEC1 fused, on its N-terminal end, to the *pelB* leader peptide and, on its C-terminal end, to a sequence coding for 6 histidines (Sequence No. 13). This arrangement allows, on one hand, the processing of this protein in the host through cleavage of the leader peptide and secretion to the *E. coli* periplasm, where the prevailing oxidizing conditions facilitate correct folding and formation of the disulphide bridges of PMEC1, and also allows, on the other hand, easy purification of this protein through immobilized metal affinity chromatography (IMAC) *(*Sulkowski, E. (1985) Purification of proteins by IMAC. Trends Biotechnol. 3, 1-7*).* The final sequence of the protein, denominated PMEC1-His6, after processing and secretion to the periplasm, is shown in Sequence No. 14.

**EXAMPLE No. 3 - Expression and purification of PMEC1-His6**

Plasmid pET-sPMEC1 was transformed *(*Sambrook J, Fritsch EF, Maniatis T. Molecular cloning: A laboratory manual. New York, USA: Cold Spring Harbor Laboratory Press; 1989*)* into the *E. coli* strain BL21 (DE3) *(*Studier, F. W and B. A. Moffatt. "Use of bacteriophage T7 RNA polymerase to direct selective high-level expression of cloned genes. " J. Mol.Biol. 189.1 (1986): 113-30*),* and a 50 mL culture of Luria-Bertani medium supplemented with ampicillin at 50 μg/mL (LBA) was inoculated with a single, isolated colony and grown for 12 hours at 30° C at 350 r.p.m. With this culture, 1 L of LBA medium was inoculated to a starting optical density at 620 nm (OD620) of 0.05, which was then grown for 8 h at 28° C until late exponential phase. This culture was then induced by the addition of isopropylthiogalactoside (IPTG), and grown in the same conditions for an additional period of 5 hours.

The culture obtained as described above was centrifuged at 5000 x g for 30 min. at 4° C and the periplasmic fraction was extracted from the resulting biomass using the method of Ausubel et al. *(*Ausubel, F.M., Brent, R., Kingston, R.E., Moore, D.D., Seidman, J.G., Smith, J. A. and Struhl, K (1989) in Current Protocols in Molecular Biology. John Wiley & Sons, New York*).* This fraction was dialyzed against 50 mM phosphate buffer pH 7 / 20 mM imidazole using a membrane with a 1000 Da cut-off, and protein PMEC1-His6 was obtained from the dialyzate by immobilized metal affinity chromatography *(*Sulkowski, E. 1985, Purification of proteins by IMAC. Trends Biotechnol. 3, 1-7), using Ni-NTA agarose (Qiagen Benelux B.V., The Netherlands) following the instructions of the manufacturer.

**EXAMPLE No. 4 - Physical and chemical characterization of the chimeric protein PMEC1-His6**

The preparation of PMEC1-His6 purified by IMAC shows a major band on SDS-PAGE (figure 7A) that migrates at an apparent mass corresponding to that expected for this protein (approximately 9500 Da), evidencing the high degree of purity of the preparation. The same figure shows that the band corresponding to reduced and carbamidomethylated PMEC1-His6 (lane 2, figure 7A) has a slightly reduced electrophoretic migration when compared to the non-reduced sample (Lane 1, figure 7A). This behavior indicates that the protein is properly folded, with the cysteines involved in intramolecular disulphide bridges.

A 80 μg aliquot of PMEC1-His6 was analyzed in a C4 4.6x250 mm (J.T.Baker, USA) reversed-phase HPLC column. The chromatographic run was carried out at 37°C, using a high pressure chromatographic system fitted with two pumps and a controller. The elution of the protein was achieved by a 10 to 60 % (v/v) linear acetonitrile gradient in 0.1% (v/v) trifluoroacetic acid, at a flow of 0.8 mL/min. and with the detection filter set at 226 nm. The chromatogram yielded a single peak, confirming the high homogeneity of the preparation (figure 7B).

The peak from the RP-HPLC was analyzed by mass spectrometry with the goal of measuring the molecular mass of the protein with a higher accuracy and verifying the oxidation status of the disulphide bridges. The spectra were acquired on a hybrid mass spectrometer with octagonal geometry QTOF-2TM (Micromass, UK), fitted with a Z-spray electronebulization ionization source. The acquired spectra were processed using the MassLynx version 3.5 (Micromass, UK) software application. The mass spectrum of the major species of the PMEC1-His6 preparation has a molecular mass of 9219.51 Da (figure 7C), and this value is only 0.05 Da off the expected value according to the sequence of the gene. This analysis confirmed that the cysteines residues on the molecule are engaged in disulphide bridges, and discarded the presence of undesired post-translational modifications such as N- or C-terminal degradation or modification of susceptible aminoacids.

**EXAMPLE No. 5 - Antigenic characterization of PMEC1**

Purified PMEC1 protein was characterized by dot blotting using monoclonal and polyclonal murine antibodies as well as dengue reactive human sera (table 2 and 3). As negative control was employed the recombinant protein DIIIe2

consisting on the domain III of the E protein of Den-2 virus (genotype Jamaica) fused to a hexa-histidine tag. Different from PMEC1, DIIIe2 corresponds to a region of higher sequence variability on the E protein. Recombinant domain III is strongly recognized by anti-Den hyperimmune ascitic fluids (HIAF) exhibiting a marked specificity for the homologous serotype and losing most of the reactivity for the reduction of the disulfide bond of this domain. This serotype specificity in the reactivity of antibodies toward domain III has also been found in human antibody response. Mab 3H5 was also included as a control in the assay. Different from Mab 4G2, 3H5 recognizes a serotype specific epitope within domain III of Den-2. Reactivity of these two Mabs is affected by the treatment with a reducing agent (Roehrig JT, Volpe KE, Squires J, Hunt AR, Davis BS, Chang GJ. Contribution of disulfide bridging to epitope expression of the dengue type 2 virus envelope glycoprotein. J Virol. 2004; 78: 2648-52).

**Table 2.** Reactivity of monoclonal and polyclonal antibodies toward PMEC1 protein in dot blotting

| *Abs*** | *Specificity**** | *PMEC1** | *PMCE1-RC** | *DIIIe2* | *DIIIe2_RC* |
|---|---|---|---|---|---|
| HIAF | DEN-1 | +++ | - | + | - |
| HIAF | DEN-2 | +++ | - | +++ | + |
| HIAF | DEN-3 | +++ | - | + | - |
| HIAF | DEN-4 | +++ | - | + | - |
| HIAF | TBE | ++ | - | - | - |
| HIAF | YFV | ++ | - | - | - |
| HIAF | SLV | +++ | - | - | - |
| Mab 4G2 | GF | +++ | - | - | - |
| Mab 3H5 | DEN-2 | - | - | - | +++ |

* Ten micrograms of purified PMEC1 and DIIIe2 proteins were loaded to nitrocellulose membrane. RC: reduced and carbamidomethylated protein. Signal intensity was evaluated in a scale of + to +++.
** HIAFs were used at 1:100 dilution. 3H5 and 4G2 Mabs were employed at 10 μg/mL.
*** TBE: Tick borne encephalitis virus, YFV: Yelow fever virus, SLV, Saint Louis virus, GF: cross-reactive to flavivirus serogroup.

PMEC1 was recognized by HIAF obtained against the four serotypes of Den as well as for the Mab 4G2. Among the rest of the HIAF obtained against different flaviviruses that were evaluated, anti-SLE exhibited the highest reactivity toward PMEC1 with similar signal intensity as obtained for anti-Den HIAF. Anti-TBE and anti-YF HIAF also recognized PMEC1 even though with lower intensity. Reactivity of the different HIAF was highly dependent on the presence of the disulfide bonds of PMEC1 indicating that the protein is correctly folded in a similar conformation as the native structure of E protein on the virus.

PMEC1 protein was also characterized in dot blotting through the reactivity with human sera from persons that had been infected with Den on different epidemiological situations. In the assay were included sera from convalescents of primary infection with the four virus serotypes (i.e. Den1, Den2, Den3 and Den4). Sera from individuals that had suffered secondary infection with Den2 or Den3 were also used. Human antibodies were employed as pools of sera from three individuals infected in the same epidemic and that experimented similar clinical symptoms and with similar serology results. Each serum was evaluated for the presence of IgM antibodies against the viral antigens and PMEC1 protein.

**Table 3**. Reactivity of human antibodies toward PMEC1 protein in dot blotting.

| | *PI** | | | | *SI*** | | Mab 4G2 | Mab 3H5 |
|---|---|---|---|---|---|---|---|---|
| | DEN-1 | DEN-2 | DEN-3 | DEN-4 | DEN-2 | DEN-3 | | |
| *PMEC1**** | ++ | ++ | ++ | ++ | +++ | +++ | +++ | - |
| *PMCE1-RC* | - | - | - | - | - | - | - | - |
| *DIIIe2* | - | ++ | - | - | ++ | ++ | - | +++ |
| *DIIIe2-RC* | - | - | - | - | - | - | - | - |
| DEN Ag | +++ | +++ | +++ | +++ | +++ | +++ | +++ | ++ |

(continued)

| | PI* | | | | SI** | | Mab 4G2 | Mab 3H5 |
|---|---|---|---|---|---|---|---|---|
| | DEN-1 | DEN-2 | DEN-3 | DEN-4 | DEN-2 | DEN-3 | | |
| Neg Ctrl | - | - | - | - | - | - | - | - |

* Pool of sera from convalescents of primary infection for (PI) for DEN-1, DEN-2, DEN-3 and DEN-4.
** Pool of sera from convalescents of secondary infection for (SI) for DEN-2 and DEN-3.
*** Ten micrograms of purified PMEC1 and DIIIe2 proteins were loaded to nitrocellulose membrane.
RC: reduced and carbamidomethylated protein. Signal intensity was evaluated in a scale of + to +++.
DEN Ag: Pool of viral antigens of the four serotypes. Viral antigens were obtained from the supernatant of infected Vero cells.
Neg Ctrl: Control preparation consisting on the supernatant of non-infected Vero cells. Human sera were used at 1:400 dilution. 3H5 and 4G2 Mabs were employed at 10 μg/mL.

Human sera from individuals infected with the different virus serotypes recognized PMEC1 with similar intensity. Strongest signals were obtained with sera from individuals that suffered secondary infection which corresponded with the higher antiviral titers by ELISA as well.

**EXAMPLE No. 6 - Characterization of antibody response obtained by immunization with PMEC1 protein.**

A group of 80 Balb/c mice were injected by intraperitoneal (i.p) route with 20 μg of purified PMEC1 emulsified with Freund's adjuvant. Ten mice were bled after the fourth dose and the sera were collected for further serological analysis. The anti-Den antibody titers measured by ELISA were similarly high for the four serotypes of the virus (Table 4). In parallel, the functionality of the Abs elicited was measured by inhibition of hemaglutination (IHA) and plaque reduction neutralization (PRNT) tests. In the IHA assay anti-PMEC1 antibodies yielded positive titers against the four Den serotypes (Table 5). Also neutralization titers of 1/1280 were obtained against the four viruses (Table 6).

**Table 4.** Anti-Den antibodies titer of sera obtained by immunization with PMEC1 protein.

| | Antibody titer determined by ELISA* | | | |
|---|---|---|---|---|
| **mouse** | **anti-DEN-1** | **anti-DEN-2** | **anti-DEN-3** | **anti-DEN-4** |
| 1 | 1: 128000 | >1: 128 000 | 1: 64000 | 1:128 000 |
| 2 | >1: 128 000 | >1: 128 000 | >1: 128 000 | >1: 128 000 |
| 3 | 1: 64000 | >1: 128 000 | 1: 128 000 | 1: 128 000 |
| 4 | >1: 128 000 | >1: 128 000 | >1: 128 000 | >1: 128 000 |
| 5 | 1: 128 000 | 1: 128 000 | 1: 128 000 | 1: 32000 |
| 6 | 1: 64000 | 1: 64000 | >1: 128 000 | 1: 64000 |
| 7 | >1: 128 000 | >1: 128 000 | >1: 128 000 | >1: 128 000 |
| 8 | 1: 128 000 | 1: 64000 | 1: 128 000 | 1: 128 000 |
| 9 | >1: 128 000 | >1: 128 000 | >1: 64 000 | >1: 128 000 |
| 10 | >1: 128 000 | >1: 128 000 | >1: 64 000 | >1: 128 000 |

*Antibody titers were determined by end-point dilution. Each serum was evaluated in parallel with a viral antigen preparation obtained from suckling mice brain infected with each virus serotype as described (Clarke, D. M., Casals, J. Techniques for hemaglutination and hemaglutination-inhibition with arthropode-bome viruses. American Journal of Tropical Medicine and Hygiene 1958. 7:561-573). A similar preparation obtained from brain of non-inoculated mice was used as negative control.

**Table 5.** IHA titer of antibodies generated by immunization with PMEC1 protein.

| | Titer of IHA* | | | |
|---|---|---|---|---|
| Mouse | anti-DEN-1 | anti-DEN-2 | anti-DEN-3 | anti-DEN-4 |
| 1 | 1:640 | 1:320 | 1:640 | 1:640 |
| 2 | 1:640 | 1:640 | 1:640 | 1:320 |
| 3 | 1:320 | 1:320 | 1:320 | 1:320 |
| 4 | 1:10 | 1:5 | 1:10 | 1:10 |
| 5 | 1:640 | 1:640 | 1:640 | 1:640 |
| 6 | 1:640 | 1:320 | 1:640 | 1:640 |
| 7 | 1:640 | 1:640 | 1:640 | 1:640 |
| 8 | 1:1280 | 1:640 | 1:1280 | 1:1280 |
| 9 | 1:320 | 1:320 | 1:320 | 1:640 |
| 10 | 1:90 | 1:5 | 1:10 | 1:10 |
| * IHA titers were defined as the maximal dilution inhibiting goose erythrocytes hemaglutination caused by 8 hemaglutinating viral units. | | | | |

**Table 6.** Viral neutralization assay using sera from animals immunized with PMEC1 protein

| | Viral neutralization titer* | | | |
|---|---|---|---|---|
| Mouse | anti-DEN-1 | anti-DEN-2 | anti-DEN-3 | anti-DEN-4 |
| 1 | 1: 1280 | 1: 1280 | 1: 1280 | 1: 1280 |
| 2 | 1: 1280 | 1: 1280 | 1: 1280 | 1: 640 |
| 3 | 1: 640 | 1: 160 | 1: 320 | 1: 320 |
| 4 | 1: 80 | <1: 40 | <1: 40 | <1: 40 |
| 5 | 1: 1280 | 1: 1280 | 1: 1280 | 1: 1280 |
| 6 | 1: 640 | 1: 1280 | 1: 1280 | 1: 1280 |
| 7 | 1: 320 | 1: 640 | 1: 640 | 1: 640 |
| 8 | 1: 1280 | 1: 1280 | 1: 1280 | 1: 1280 |
| 9 | 1: 1280 | 1: 320 | 1: 1280 | 1: 640 |
| 10 | <1: 40 | 1: 320 | 1: 320 | 1: 320 |
| * Neutralizing titers were defined as the dilution yielding 50% reduction of viral plaques in BHK-21 cells. | | | | |

**EXAMPLE No. 7. - Protection assay**

Animals immunized with PMEC1 that remained after bleeding were divided in groups and used to perform the challenge study. Four groups of 15 animals were inoculated by i.c injection with 100 LD50 of a live neuroadapted strain of one of the four serotypes of the virus and observed for 21 days. A fifth group of 10 animals did not received the viral challenge. Positive controls consisted on groups of 15 animals immunized and subsequently challenge with the homologous serotype of the virus (i.e. Den1, Den2, Den3 and Den4). Another four groups of 15 mice each were employed as negative controls of the experiment; these animals received PBS as immunogen and were challenge with the different viral serotypes. Groups of animals immunized with PMEC1 exhibited between 80% to 90% of animal survival, while mice immunized with PBS develop symptoms of encephalitis between days 7-11 after viral inoculation and died before day 21 (Table 7). One hundred percent of the animals from the virus-immunized groups were protected.

**Table 7.** Survival percentage in mice immunized with the protein variants to the challenge with lethal Den virus.

| Immunogen | Virus serotype | Survival percentage* |
|---|---|---|
| PBS | DEN-1 | 0 |
| PBS | DEN-2 | 0 |
| PBS | DEN-3 | 0 |
| PBS | DEN-4 | 0 |
| DEN-1 | DEN-1 | 100 |
| DEN-2 | DEN-2 | 100 |
| DEN-3 | DEN-3 | 100 |
| DEN-4 | DEN-4 | 100 |
| PMEC1 | DEN-1 | 86 |
| PMEC1 | DEN-2 | 80 |
| PMEC1 | DEN-3 | 90 |
| PMEC1 | DEN-4 | 86 |
| * Calculated with the following expression: (# of surviving mice)/(# of total mice) Surviving mice data was collected 21 days after challenge. | | |

**EXAMPLE 8. Structure prediction of the complex formed by mAb 4G2 and the E-protein.**

In order to model the structure of the antigen-antibody complex, a molecular docking study was performed using the crystallographic structure of the FAb fragment of the mAb 4G2 (1uyw) and two crystallographic structure of the envelope E-protein from dengue 2 virus (PDB files loan and 1oam). The CLUSPRO method was used for predictions (http://nrc.bu.edulcluster/, S.R. Comeau, D.W. Gatchell, S. Vajda, C.J. Camacho. ClusPro: an automated docking and discrimination method for the prediction of protein complexes. (2004) Bioinformatics, 20, 45-50*),* including two different programs for generation of the structures of potential complexes: DOT and ZDOCK *(*Mandell JG, Roberts VA, Pique ME, Kotlovyi V, Mitchell JC, Nelson E, Tsigelny I, Ten Eyck LF. (2001) Protein docking using continuum electrostatics and geometric fit. Protein Eng 14:105-13*.* Chen R, Li L, Weng Z (2003) ZDOCK: An Initial-stage Protein-Docking Algorithm. Proteins 52:80-87*).*

In total, 13 molecular docking simulations were carried out changing the following parameters: the docking program (DOT or ZDOCK), the definition of the ligand and the receptor (Fv fragment or E-protein), the crystallographic structure of the E-protein (1oan or 1oam), the quaternary structure of the E-protein (monomer or dimer), the use of constrains to filtrate solutions which involve the binding site of the Fv fragment or the N-terminal segment (residues 1-120) of the E-protein (Attract option in DOT). The figure 7 shows a scheme summarizing the results of the simulations. Those complexes were considered as potential solutions, which predict an epitope localized in the domain II of the E-protein, this epitope is accessible to the interaction with antibodies according to the structure of the virion and the paratope is the hyper-variable region of the antibody. The location of the epitope A1 (epitope recognized by mAb 4G2) in domain II is supported by experimental data and it has also been determined that related antibodies directed against the same epitope, recognize a proteolytic fragment consisting in amino acids 1-120 of the E-protein *(*Roehrig, J.T., Bolin, R.A. and Kelly, R.G. Monoclonal Antibody Mapping of the Envelope Glycoprotein of the Dengue 2 Virus, Jamaica. 1998, Virology 246: 317-328*).*

Six potential solutions were obtained, which were structurally very similar. The table 8 shows the values corresponding to the interface parameter of the predicted E-protein-Fv complex, the values calculated for the predicted complex are similar to those calculated for protein-antibody complexes, whose crystallographic structure has been previously determined experimentally (table 9).

The surface patch of the E-protein contacting the antibody, involves 4 segments of the protein sequence. This finding is consistent with the topographic nature of the epitope, whose recognition depends on the correct folding of the protein, and is susceptible to reduction of the disulfide bridges. The structural epitope defined by the three-dimensional model contains region highly conserved in flavivirus, which is consistent with the wide cross-reactivity of this antibody and with the recognition of the chimerical protein PMEC1 shown the example 5. The model also suggests that the neutralization mechanism of this antibody involves the interfering of E-protein binding to membranes and/or the trimerization associated to the fusion process.

Moreover, the epitope recognized by the antibody coincides with the region involved in the interaction between the E-protein and pre-M, as inferred from the electronic density corresponding to preM in the cryo-electron microscopy studies of the immature virions. The evolutionary pressure related to the conservation of the interaction surface could explain the high conservation of this epitope on the E-protein. Furthermore, the appearance of escape mutants in this surface patch is less probable, because such mutations should be compensated by stabilizing simultaneous mutations on the surface of pre-M. In fact, escape mutants obtained against this antibody are located in the hinge region between domains I and II, and the mutant viruses show a highly attenuated and defects in its capacity to fusion membranes. This constitutes a favorable property the PMEC chimerical proteins of the present invention as recombinant vaccine candidates against flavivirus.

Thereafter, we modeled the interaction between the FAb 4G2 and the E-protein, in the context of the structure of the mature virions. With this aim, we docked the previously modeled complex into the cryo-electron microscopy structure of the mature virions. In order to obtain the model we used: 1) the PDB file 1THD corresponding to the structure of the virion obtained by cryo-electron microscopy , 2) the coordinates of the complex formed by the FAb 4G2 and the monomer of the E-protein, which was previously modeled by molecular docking, 3) the icosahedrical symmetry operations corresponding to the file 1THD were applied to the complex previously modeled by molecular docking.

This way, a model was obtained in which all copies of the epitope recognized by the mAb 4G2 (180) present on the virion, are occupied by FAbs (figure 9).

**Table 8.** Interface properties of the model corresponding to the complex formed by the mAb 4G2 and the E-protein.

| ***Interface parameters**** | ***Value for Fv*** | ***Value for E*** |
|---|---|---|
| Interface surface accessibility | 1070.14 | 1034.10 |
| % Interface surface accessibility | 10.60 | 5.20 |
| Planarity | 2.46 | 2.50 |
| Length y Width | 32.62 & 24.75 | 39.74 & 26.44 |
| Ratio Length/Width | 0.67 | 0.55 |
| Interface segments | 7 | 4 |
| % Interface polar atoms | 51.78 | 46.49 |
| % Interface apolar atoms | 48.20 | 53.50 |
| Secondary structure | Beta | Beta |
| Hydrogen bonds | 16 | 16 |
| Salt bridge | 0 | 0 |
| Disulfide bridge | 0 | 0 |
| Separation Volume | 4618.96 | 4618.96 |
| Index of separation volume | 2.20 | 2.20 |

* protein-protein interface parameters were calculated using the following web server http://www.biochem.ucl.ac.uk/bsm/PP/server/

An inspection of the distance between the C-terminal residues of the heavy chains of the FAb fragments indicates that antibody bivalent binding is not possible without major changes in the structure of the virion. This observation is consistent with the results obtained in the example 12, showing that equimolar amounts of FAb and mAb display very similar neutralizing titers. This finding contrast with an increase of 2-3 orders of magnitude expected for a bivalent binding mode.

**Table 9.** Characteristic interface properties of protein-antibody complexes*.

| ***Interface parameters*** | | ***Protein-antibody*** |
|---|---|---|
| No of examples | | 6 |
| **Interface ASA (Å$^2$)** | Media | 777 |
| | sd | 135 |
| **Planarity** | Media | 2.2 |
| | sd | 0.4 |
| **Circularity** | Media | 0.8 |
| | sd | 0.1 |
| **Segmentation** | Media | 4 |
| | sd | 1.83 |
| **Hydrogen Bonds per 100(Å$^2$)** | Media | 1.1 |
| | sd | 0.5 |
| **Separation index** | Media | 3.0 |
| | sd | 0.8 |
| *Jones, S. and Thornton, J.M. (1996). Principles of Protein-Protein Interactions Derived From Structural Studies PNAS. Vol. 93 p.13-20. http: //www.biochem.ucl.ac.uk/bsm/PP/server/ | | |

**EXAMPLE 9. Design of the chimerical proteins MA4G2 (bivalent) and TB4G2 (trivalent).**

In this example we show the design of chimerical proteins related to the mAb 4G2 binding site, which can bind simultaneously two or three copies of E-protein monomers displayed in the mature virion. The modeling studies of the example 8 indicates that the distances separating the C-terminal residues of the heavy chains of FAbs associated to E-protein monomers located in the asymmetric unit of the virion are 80, 100 and 120 Å respectively. These values are too large to allow antibody bivalent binding to the virion (figure 9). The distances calculated between C-terminal residues corresponding to Fabs bound to E-protein monomer from different asymmetric units are still larger. However, the distances calculated between the C-terminal residues of the heavy chains of Fv fragments bound to the asymmetric unit are 36, 58 and 70 Å respectively. These three atoms are circumscribed in a circle of 35 Å in radius, indicating that trivalent binding is possible by the fusion to trimerization domains through linker segments of 10-15 residues.
Similarly, the corresponding distance between the C-terminus of the heavy chains of Fv fragments bound to E-protein dimers is 36 Å, indicating that bivalent binding is possible by the fusion to dimerization domains with small linker segments of 5-10 residues.

**Design of a miniantibody type molecule (bivalent binding)**

As an example of a bivalent binding molecule we designed the chimerical protein MA4G2. Its sequence contains from the N- to the C-terminus the following segments:

1- scFv, single chain Fv fragment of the mAb 4G2 of the type VL-linker-VH (Sequence No. 25, 26 and 27), VL is the variable region of the light chain of mAb 4G2 and VH is the variable region of the heavy chain of the same antibody.
2- GGG, three glycine residues linker segment
3- Hinge-CH2-CH3, corresponds to the sequence of the hinge segment and the constant domains 2 and 3 of the human IgG1 molecule and the glycosilation site N297 have been mutated to Glycine (Sequence No. 52)

The protein MA4G2 can be expressed in eucariotes and procariotes, and it associates as dimers due to the formation of intermolecular disulfide bridges between the cystein residues located the hinge region, this way displaying a human FC domain at the C-terminal part of the molecule. The hinge region displays adequate spacing and flexibility and therefore a three residue linker (GGG) is enough as connector between the scFv domain and the hinge-FC segment. The figure 10 shows a model of the 3D structure of the complex formed by the chimerical protein MA4G2 and an E-protein dimer, indicating the feasibility of bivalent binding to the virion.
The presence of the mutation at the glycosilation site allows obtaining non-glycosilated FC bearing molecules in Eucariotes. The non-glycosilated FC domains do not bind to the receptors FcyRI-III, which are able to mediate infection immune-enhancement *in vitro (* Lund, J., Takahashi, N., Pound, J. D., Goodall, M., and Jefferis, R. 1996, J. Immunol. 157, 4963-4969. Lund, J., Takahashi, N., Pound, J. D., Goodall, M., Nakagawa, H., and Jefferis, R. 1995, FASEB. J. 9, 115-119*).* This way, unlike the original antibody molecule, the designed protein lack any risk of mediate ADE at sub-neutralizing concentrations. Furthermore, the chimerical protein retains the FcRn receptor binding properties, which is desired to display long half time of life in vivo, similar to the natural antibodies.

**Chirnerical trivalent protein TB4G2**

As an example of a trivalent binding molecule, we designed the chimerical protein TB4G2, whose sequence is described as following structure:
scFv-Linker-T
where,

1- scFv, single chain Fv fragment of the mAb 4G2 of the type VL-linker-VH (Sequence No. 25, 26 and 27), VL is the variable region of the light chain of mAb 4G2 and VH is the variable region of the heavy chain of the same antibody
2- Linker, is a linker segment of sequence $(GGGS)_3$GGG (Sequence No. 28)
3-T is a helical trimerization domain human matrilin (Sequence No. 51)

The trimerization domain of matrilin is an alpha helix which trimerizes as a parallel coiled-coil structure. The trimer includes six intermolecular disulfide bridges formed by two cystein residues located close to the N-terminus of each monomer. This trimeric helicoidal structure is highly stable dG = 7kcal/mol at 50°C *(*Wiltscheck R, Kammerer RA, Dames SA, Schulthess T, Blommers MJ, Engel J, Alexandrescu AT. Heteronuclear NMR assignments and secondary structure of the coiled coil trimerization domain from cartilage matrix protein in oxidized and reduced forms. Protein Sci. 1997; 6: 1734-45*).* The disulfide bridges ensure the covalent linked trimeric quaternary structure even at very low concentrations, which compares favorably with trimers based in non-covalent interactions only.
The linker segment is composed by the amino acids Gly and Ser and it is very flexible. Amino acid sequences of similar composition have been used very often as linker sequences in protein engineering. Although a segment of 10 residues can provide an spacing of 35 Å necessary for trivalent binding to the virion, it is only true if the segment adopt a fully extended conformation. In solution, the linker segment can display multiple conformations in thermodynamic equilibrium and adopting a unique extended conformation would imply a significant entropic energetic lost. In order to explore the structural properties of the linker segment, we performed a structure prediction of the 15 residue $(GGGS)_3$GGG sequence using the program prelude. This method is based on statistical potentials describing local interactions and it has been previously used for peptide structure prediction. The figure 11 shows a plot of the energy values vs the distance between the N- and C-terminus for the predicted more favorable conformations. The energy minimum corresponds to dimensions of 35 Å and the most extended conformations (more than 40 A) are very unfavorable. Therefore, the computations indicate that the sequence chosen as linker segment is adequate for the design of trivalent binding molecules.

**EXAMPLE No. 10 - Obtention of plasmids coding for a single-chain antibody fragment (scFv 4G2), a trivalent molecule (TB4G2), and a single-chain miniantibody (MA4G2) with the variable regions from antibody 4G2.**

In order to obtain a single chain antibody fragment, a multimeric protein, and a single chain miniantibody (MA4G2) with the variable regions from monoclonal antibody 4G2, the method of Agarwal *et al. (*Agarwal KL, Büchi H, Caruthers MH, Gupta N, Khorana HG, Kumas A, Ohtsuka E, Rajbhandary UL, van de Sande JH, Sgaramella V, Weber H, Yamada T, Total synthesis of the Gene for an alanine transfer ribonucleic acid from yeast, (1970), Nature 227, 27-34*)* was used to synthesize, starting from oligonucleotides synthesized on solid phase through phosphoramidite chemistry *(*Beaucage SL, Caruthers MH, Deoxynucleoside phosphoramidites- A new class of key intermediates for deoxypolynucleotide synthesis., Tetrahedron Letters, (1981), 22, 1859*),* double stranded DNA molecules (Sequence No. 15, Sequence No. 16 and Sequence No. 17), each of which was digested with the restriction enzymes *Nco* I and *Xho* I (Promega Benelux b.v., The Netherlands) under the conditions specified by the manufacturer. Each digested molecule was then ligated using T4 DNA ligase (Promega Benelux, b.v., The Netherlands), under the conditions specified by the manufacturer, to plasmid pET22b (Novagen, Inc., USA), previously digested with the same enzymes. The ligations were transformed into the *E. coli* strain XL-1Biue (Bullock WO, Femández JM, Short JM. XL-1Blue: A high efficiency plasmid transforming recA Escherichia coli K12 strain with beta-galactosidase selection. Biotechniques 1987;5:376-8*),* following the conditions described by Sambrook *et al. (*sambrrook J, Fritsch EF, Maniatis T. Molecular cloning: A laboratory manual. New York, USA: Cold Spring Harbor Laboratory Press, 1989*),* and the plasmids present in the resulting colonies growing on selective medium were screened using restriction analysis. The sequence of several recombinant plasmids from each transformation was verified by automatic Sanger sequencing, and for each reaction a representative molecule was chosen whose sequence matched the expected sequence. These plasmids were denominated pET-scFv 4G2 LH (Figure 5, Sequence No. 18) for the expression of the single-chain antibody fragment, pET-TB4G2 LH (Figure 6A, Sequence No. 19) for the expression of the multimeric sequence, and pET-MA4G2 LH (Figure 6B, Sequence No. 20) for the expression of the single chain miniantibody carrying the variable regions from antibody 4G2.

These plasmids can be used for the expression in *Escherichia coli,* through induction with isopropylthiogalactoside (IPTG) and under the T7 promoter, of the proteins coded by the aforementioned synthetic bands (Sequence No. 15, Sequence No. 16 and Sequence No. 17), which, in their respective immature, unprocessed forms (Sequence No. 21, Sequence No. 22 and Sequence No. 23) contain the following elements in an N- to C-terminal direction: For the unprocessed protein scFv 4G2 LH, a) The *pelB* signal peptide (Sequence No. 24), b) The aminoacids M (Methionine) and A (Alanine), introduced due to the nature of the *Nco* I site, c) the variable region of the light chain of monoclonal antibody 4G2 (Sequence No. 25), d) a flexible spacer (linker) (Sequence No. 26), e) the variable region of the heavy chain of the monoclonal antibody 4G2 (Sequence No. 27), f) the aminoacids L (Leucine) and E (Glutamic acid), introduced due to the cloning strategy, and g) a C-terminal segment of 6 histidines; for the unprocessed protein TB4G2 LH: a) The *pelB* signal peptide (Sequence No. 24), b) The aminoacids M (Methionine) and A (Alanine), introduced due to the nature of the *Nco* I site, c) the variable region of the light chain of monoclonal antibody 4G2 (Sequence No. 25, d) d) a flexible spacer (linker) (Sequence No. 26), e) the variable region of the heavy chain of the monoclonal antibody 4G2 (Sequence No. 27), f) a flexible spacer (linker) (Sequence No. 28), g) a fragment from human matrilin that confers the molecule the property of being able to trimerize in solution (Sequence No. 51), h) the aminoacids L (Leucine) and E (Glutamic acid), introduced due to the cloning strategy, and e) a C-terminal segment of 6 histidines; and for the unprocessed MA4G2 LH protein: a) The *pelB* signal peptide (Sequence No. 24), b) The aminoacids M (Methionine) and A (Alanine), introduced due to the nature of the *Nco* I site, c) the variable region of the light chain of monoclonal antibody 4G2 (Sequence No. 25), d) a flexible spacer (linker) (Sequence No. 26), e) the variable region of the heavy chain of the monoclonal antibody 4G2 (Sequence No. 27), f) a flexible spacer (linker) composed of three successive glycines (G), g) a fragment of the constant region of the IgG1 human immunoglobulins that contains the hinge and the CH2 and CH3 domains, where the aminoacid C (Cysteine) of the hinge has been changed by mutagenesis to an S (Serine) and the potential glycosylation site of the CH2 domain has been eliminated by mutating an N (Asparagine) to a Q (Glutamine) (Sequence No. 52), h) h) the aminoacids L (Leucine) and E (Glutamic acid), introduced due to the cloning strategy, and e) a C-terminal segment of 6 histidines.

These elements allow the processing of these proteins (scFv 4G2, TB4G2 and MA4G2) through leader peptide cleavage and their secretion to the *E. coli* periplasm, where the prevalent oxidizing conditions allow their correct folding and formation of their disulphide bridge, and also facilitate their purification using immobilized metal affinity chromatography (IMAC) (Sulkowski, E. (1985) Purification of proteins by IMAC. Trends Biotechnol. 3, 1-7). The final sequences of scFv 4G2, TB4G2 and MA4G2 after posttranslational processing and secretion are shown in Sequence No. 53, Sequence No. 54 and Sequence No. 55.

**EXAMPLE No. 11 - Expression and purification of scFv 4G2, TB4G2 and MA4G2**

The purification of scFv 4G2, TB4G2 and MA4G2 from plasmids pET-scFv4G2 LH, pET-TB4G2 LH y pET-MA4G2, respectively, used the process described as follows: The corresponding plasmid was transformed following the instructions of Sambrook *et al.* (Sambrook J, Fritsch EF, Maniatis T. Molecular cloning: A laboratory manual. New York, USA: Cold Spring Harbor Laboratory Press; 1989) into the BL21 (DE3) *E. coli* strain *(*Studier, F. W. and B. A. Moffatt. "Use of bacteriophage T7 RNA polymerase to direct selective high-level expression of cloned genes." J.Mol.Biol. 189.1 (1986): 113-30*),* and an isolated colony was used to inoculate a 50 mL culture of Luria-Bertani medium supplemented with ampicillin at 50 μg/mL (LBA), which was grown for 12 hours at 30° C at 350 r.p.m. With this culture, 1 L of LBA medium was inoculated to a starting optical density at 620 nm (OD620) of 0.05, which was then grown for 8 h at 28° C until late exponential phase. This culture was then induced by the addition of isopropylthiogalactoside (IPTG), and grown in the same conditions for an additional period of 5 hours.

The culture obtained as described above was centrifuged at 5000 x g for 30 min. at 4° C and the periplasmic fraction was extracted from the resulting biomass using the method of Ausubel et al. *(*Ausubel, F.M., Brent, R., Kingston, R.E., Moore, D.D., Seidman, J.G., Smith, J.A. and Struhl, K (1989) in Current Protocols in Molecular Biology. John Wiley & Sons, New York*).* This fraction was dialyzed against 50 mM phosphate buffer pH 7 / 20 mM imidazole using a membrane with a 1000 Da cut-off, and protein PMEC1-His6 was obtained from the dialyzate by immobilized metal affinity chromatography *(*Sulkowski, E. 1985, Purification of proteins by IMAC. Trends Biotechnol. 3, 1-7), using Ni-NTA agarose (Qiagen Benelux B.V., The Netherlands) following the instructions of the manufacturer

**Example 12. Neutralization of viral infection by MAG4G2 and TB4G2 proteins.**

The characterization of the biological activity of MA4G2 y TB4G2 chimeric proteins was performed using a plaque reduction neutralization assay in BHK-21 cells (Table 10). This same assay was employed to compare the biological activity of chimeric proteins with Mab 4G2, its Fab and Fab2 fragments and scFv4G2 (Table 10).

Fab and Fab2 fragments were obtained by digestion with papain and pepsin of Mab 4G2. After protease digestion Fab and Fab2 were separated from the Fc fragment by affinity chromatography with immobilized protein A. Fab and Fab2 isoforms were further purified by ion exchange chromatography. Neutralizing titers were defined as the dilution of the molecule yielding 50% reduction of the viral plaque number. Dilution of the different molecules was adjusted to obtain equimolar concentration in the assays.

**Table 10.** Viral neutralization assay of MA4G2, TB4G2, Mab4G2 and Mab4G2 Fab, Fab2 and scFv4G2 fragments.

| | **Viral neutralization titer*** | | | |
|---|---|---|---|---|
| **Molecule** | **anti-DEN-1** | **anti-DEN-2** | **anti-DEN-3** | **anti-DEN-4** |
| Mab 4G2 | 1: 1288 | 1: 1280 | 1: 320 | 1: 128 |
| Fab 4G2 | 1: 1280 | 1: 1280 | 1: 320 | 1: 128 |
| Fab2 4G2 | 1: 1280 | 1: 1280 | 1: 320 | 1: 128 |
| scFv4G2 | 1: 1280 | 1: 1280 | 1: 320 | 1: 128 |
| TB4G2 | 1: 128000 | 1: 128000 | 1: 64000 | 1: 32000 |
| MA4G2 | 1:128000 | 1:128000 | 1: 64000 | 1: 32000 |
| Mab Hep1 | <1: 40 | <1: 40 | <1: 40 | <1: 40 |
| * Neutralizing titers were defined as the dilution yielding 50% reduction of viral plaques in BHK-21 cells. | | | | |

## Claims

1. A topographic and highly conserved area, **characterized by** being exposed on the mature virion, and which represents an epitope shared among flaviviruses, that can be used in the development of wide-spectrum molecules for the prevention and/or treatment of infections due to Dengue Virus 1-4 and other flaviviruses.

2. A topographic and highly conserved area according to claim 1; wherein this area is an epitope of protein E from the envelope of flaviviruses that is defined by the following residues of the E protein from Dengue Virus 2 or the corresponding epitopes from other flaviviruses: ASN67, THR69, THR70, SER72, ARG73, CYS74, LEU82, GLU84, GLU85, ASP87, VAL97, ARG99, GLY100, TRP101, GLY102, ASN103, GLY104, CYS105, GLY106, MET118, HIS244, LYS246, LYS247, GLN248, VAL252.

3. A topographic area according to claims 1 and 2, **characterized by** being exposed on the surface of the E protein of the following flaviviruses: West Nile Virus, St. Louis Encephalitis Virus, Dengue1, Dengue2, Dengue3, Dengue4, Japanese Encephalitis Virus, Kunjin Virus, Kyasanur Forest Disease Virus, Tick-borne Encephalitis Virus, Murray Valley Virus, LANGAT virus, Louping III Virus and Powassan virus.

4. Molecules according to claim 1, useful for the prevention and/or treatment of the infections due to Dengue Virus 1-4 and other flaviviruses, based on the topographic area described in claims 1-3, **characterized by** their ability to induce a response of neutralizing antibodies which cross-react with the four serotypes of Dengue Virus and other flaviviruses in individuals immunized with said molecules.

5. Molecules according to claim 4, wherein said molecules are recombinant proteins or chimeric peptides with Sequence identification numbers 14 and 29-50.

6. Protein molecules according to claim 4, whose primary structure is defined by the sequence A-B-L-C wherein A is the sequence of a peptide of 0 to 30 aminoacids, B is the sequence of the fragment Leu237-Va1252 of protein E from Dengue Virus 2 or the homologous sequence from Dengue Virus 1, 3 , 4, or any other flavivirus, L is a sequence of 3 to 10 aminoacids that functions as a stabilizing linker, and C is the sequence of the fragment Lys64-Thr120 of protein E from Dengue Virus 2 or the homologous sequence from Dengue Virus 1, 3 , 4, or any other flavivirus.

7. Protein molecules according to claim 4, whose primary structure is defined by the sequence A-C-L-B wherein A is the sequence of a peptide of 0 to 30 aminoacids, B is the sequence of the fragment Leu237-Va1252 of protein E from Dengue Virus 2 or the homologous sequence from Dengue Virus 1, 3 , 4, or any other flavivirus, L is a sequence of 3 to 10 aminoacids that functions as a stabilizing linker, and C is the sequence of the fragment Lys64-Thr120 of protein E from Dengue Virus 2 or the homologous sequence from Dengue Virus 1, 3 , 4, or any other flavivirus.

8. A protein according to claims 6 and 7 wherein A is a bacterial signal peptide.

9. A protein according to claims 6 and 7 wherein A is a yeast or mammalian signal peptide.

10. A synthetic or recombinant fusion protein, **characterized by** being formed by the molecules described in claims 4-9, and an N- or C-terminal fusion to one or more peptide or protein fragments that enhance its protective or therapeutic effect and/or facilitate its purification and/or detection.

11. A recombinant or synthetic fusion protein according to claim 10, wherein the Nor C-terminal fusion partner is one or more peptide or protein fragments containing helper T-cell epitopes.

12. A recombinant or synthetic fusion protein according to claim 10, wherein the Nor C-terminal fusion partner is a Histidine tag.

13. A nucleic acid coding for a protein corresponding to claims 4 to 12.

**14.** A prokaryote or eukaryote host cell containing a nucleic acid according to claim 13.

**15.** A pharmaceutical composition **characterized by** containing one or more proteins according to claims 4 to 12, capable of inducing on the receiving organism an immune response of neutralizing and protective antibodies which are cross-reactive with Dengue Virus 1-4.

**16.** A pharmaceutical composition **characterized by** containing one or more proteins according to claims 4 to 12, capable of inducing on the receiving organism an immune response of neutralizing and protective antibodies which are cross-reactive with other flaviviruses.

**17.** A pharmaceutical composition **characterized by** being able to induce on the receiving organism an immune response of neutralizing and protective antibodies which are cross-reactive with Dengue Virus 1-4 and other flaviviruses, based on the use of live vectors or naked DNA containing genes coding for the proteins described in claims 4 to 12.

**18.** Synthetic or recombinant protein or peptide molecules according to claims 5 to 12, useful as diagnostic reagents for the detection of anti-flavivirus antibodies.

**19.** Molecules according to claim 1, useful for the prevention and/or treatment of infections due to Dengue Virus 1-4 and other flaviviruses, based on the topographic area described in claims 1-3, **characterized by** their ability to prevent or attenuate the viral infection due to their interaction with said topographic area.

**20.** Molecule according to claim 19, wherein said molecule is a human antibody or an antibody produced in other species.

**21.** An antibody according to claim 20, wherein said antibody is cross-reactive with different flaviviruses and is neutralizing for viral infection.

**22.** Molecule according to claims 19 to 21 to be used in the prevention and/or treatment of infections due to Dengue Virus 1-4 or other flaviviruses, wherein said molecule is a recombinant or proteolytic fragment of the antibody.

**23.** A molecule according to claim 22, wherein said molecule is a recombinant single-chain Fv fragment of the antibody (scFv).

**24.** A molecule according to claim 23, **characterized by** being linked, with or without spacers, to a protein sequence that confers said molecule the ability to assemble as a molecule with polyvalent binding to mature virions.

**25.** A molecule according to claim 24, wherein the protein sequence linked to said molecule contains a spacer and the hinge, CH2 and CH3 regions of a human immunoglobulin, with the sequence specified in Sequence No. 55 and 56.

**26.** A molecule according to claim 24, wherein the protein sequence linked to said molecule contains a spacer and a trimerization domain with the sequence specified in Sequence No. 54.

**27.** A nucleic acid coding for a protein corresponding to claims 18 to 26.

**28.** A prokaryote or eukaryote host cells containing a nucleic acid according to claim 27.

**29.** A pharmaceutical composition **characterized by** containing one or more proteins according to claims 18 to 26, capable of preventing or attenuating the infection due to Dengue Virus 1-4.

**30.** A pharmaceutical composition **characterized by** containing one or more proteins according to claims 18 to 26, capable of preventing or attenuating the infection due to other flaviviruses.

**31.** Synthetic or recombinant protein or peptide molecules according to claims 18 to 26, useful as diagnostic reagents for the detection of flaviviruses.

**32.** A molecule useful as a wide-spectrum therapeutic candidate against flaviviruses that is identified with a method that comprises the contact of said molecule with the area or conserved epitope of protein E according to claims 1-3, wherein this contact or binding indicates that said molecule is a wide-spectrum therapeutic candidate.

**33.** A molecule according to claim 32, wherein said molecule is selected among the following classes of compounds: proteins, peptides, peptidomimetics and small molecules

**34.** A method according to claim 32, wherein said molecule in included in a library of compounds.

**35.** A method according to claim 34, wherein said library of compounds is generated by combinatorial methods.

**36.** A method according to claim 32, wherein said contact is measured by an *in vitro* assay.

**37.** A method according to claim 36, wherein said assay is performed by blocking the binding of molecules according to claims 19-24 to a conserved area according to claims 1-3.

**38.** A method according to claim 36, wherein said method is performed by blocking the binding of molecules according to claims 19-24 to protein molecules according to claims 4-7.

**39.** A method according to claim 32, wherein said union is measured by an *in vivo* assay.

**40.** A method according to claim 32, wherein said method is a computer-aided method that comprises: 1) the atomic coordinates corresponding to the residues that form the highly conserved area of protein E according to claims 1-3, and said coordinates are available on protein structure databases or modeled by computational means or experimentally determined, 2) the atomic coordinates of molecules, which have been determined experimentally or modeled by computational means, 3) a computational procedure of molecular docking that allows the determination of whether this molecule will be able to make contact with the highly conserved area according to claims 1-3.

Figure 1.

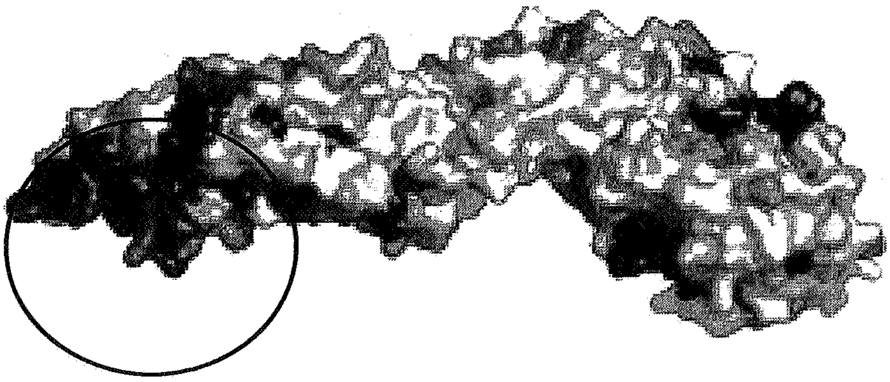

Figure 2.

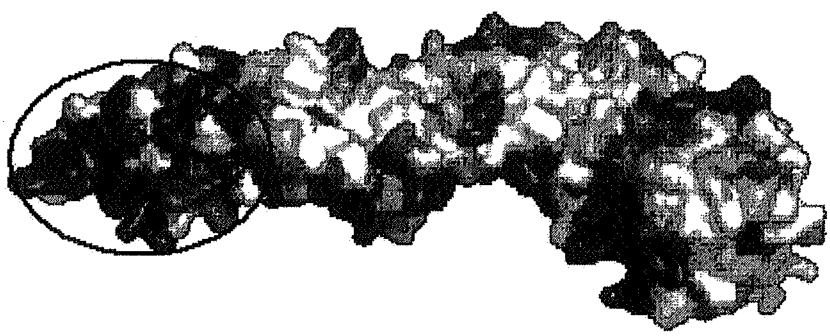

Figure 3.

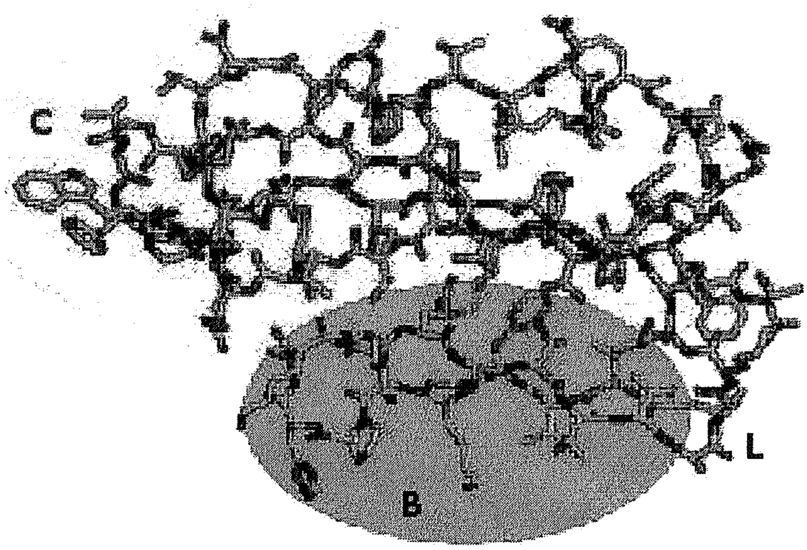


Figure 4.

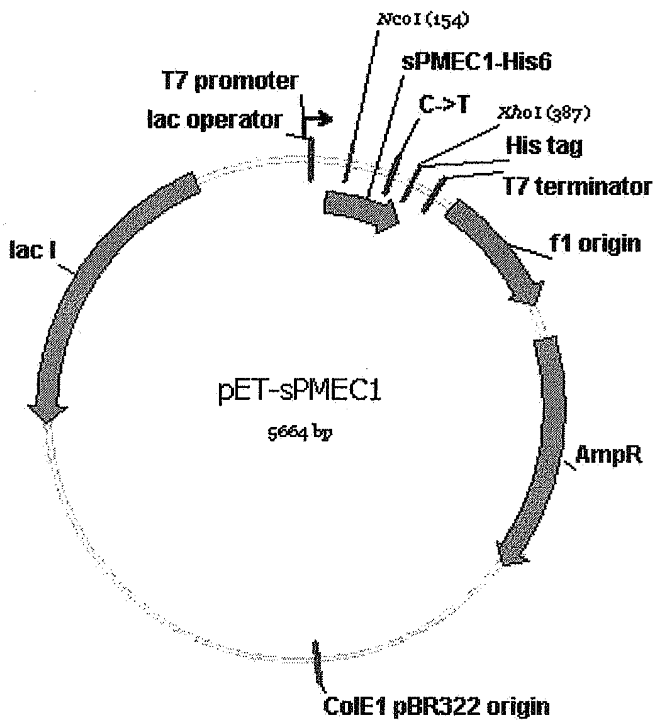

**Figure 5.**

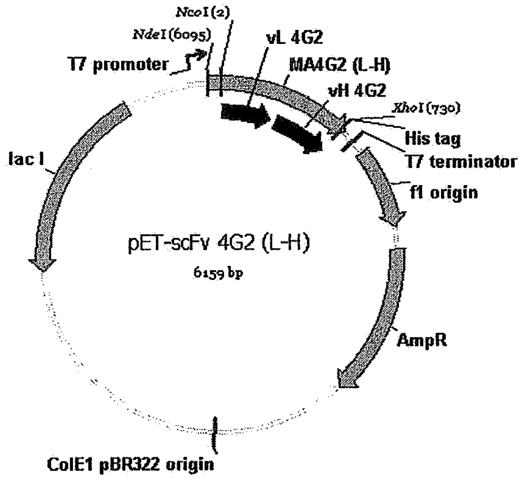


**Figure 6A.**

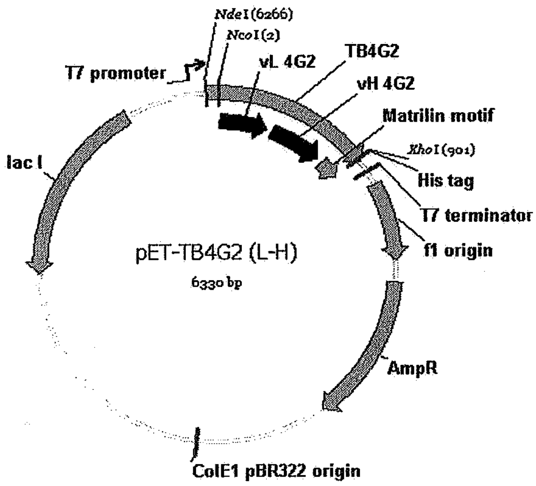


**Figure 6B.**

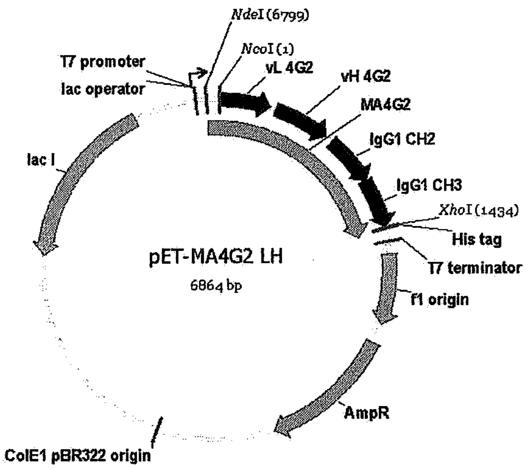

Figure 7

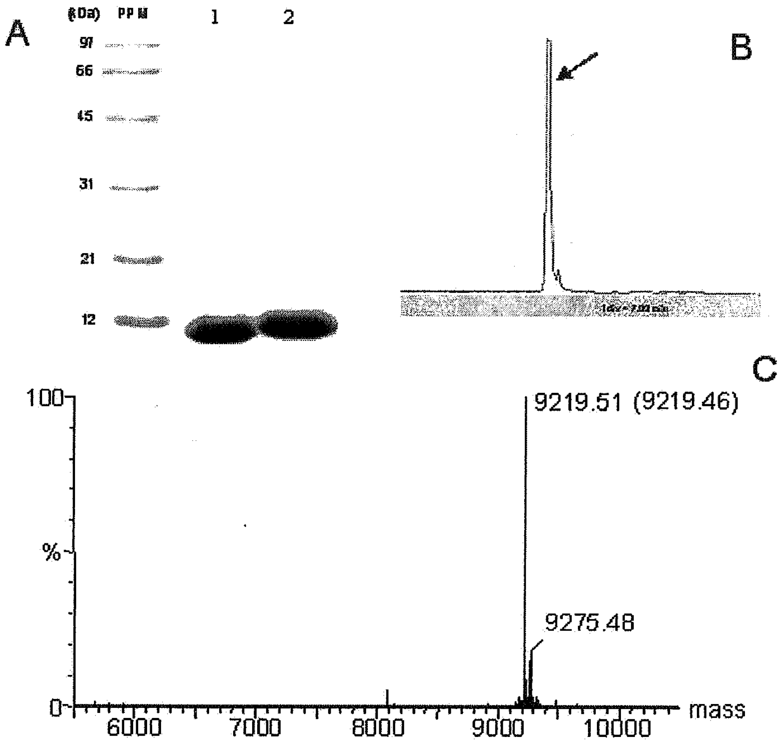


Figure 8

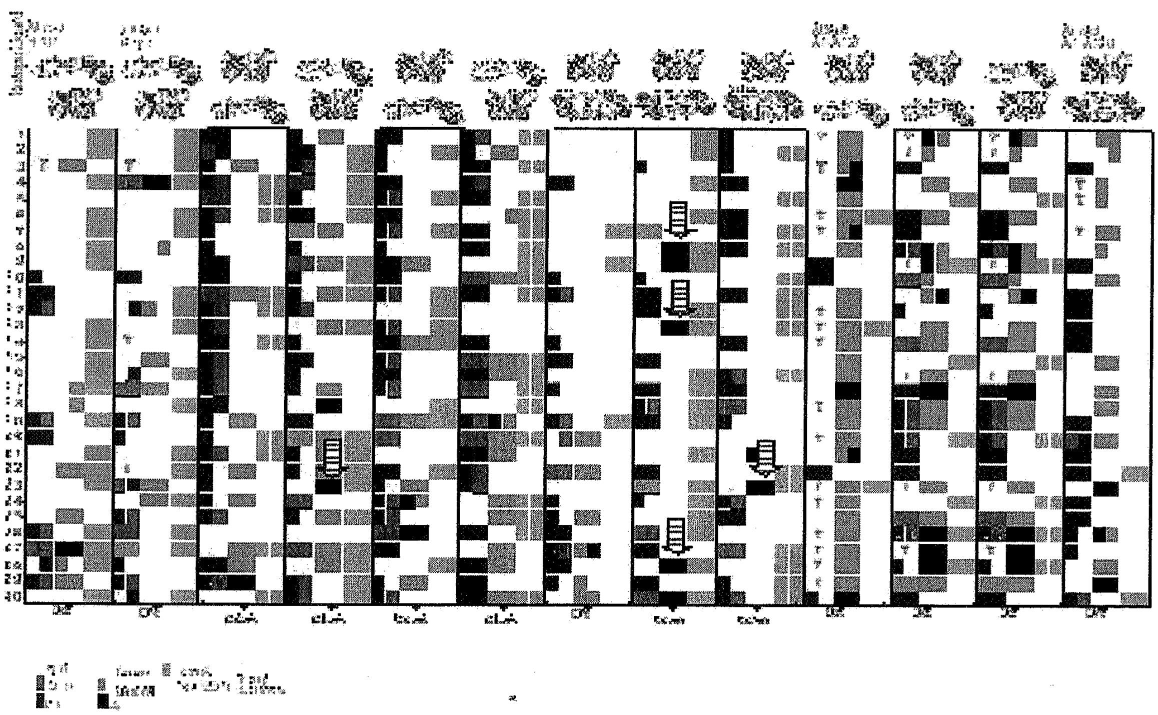

Figure 9.

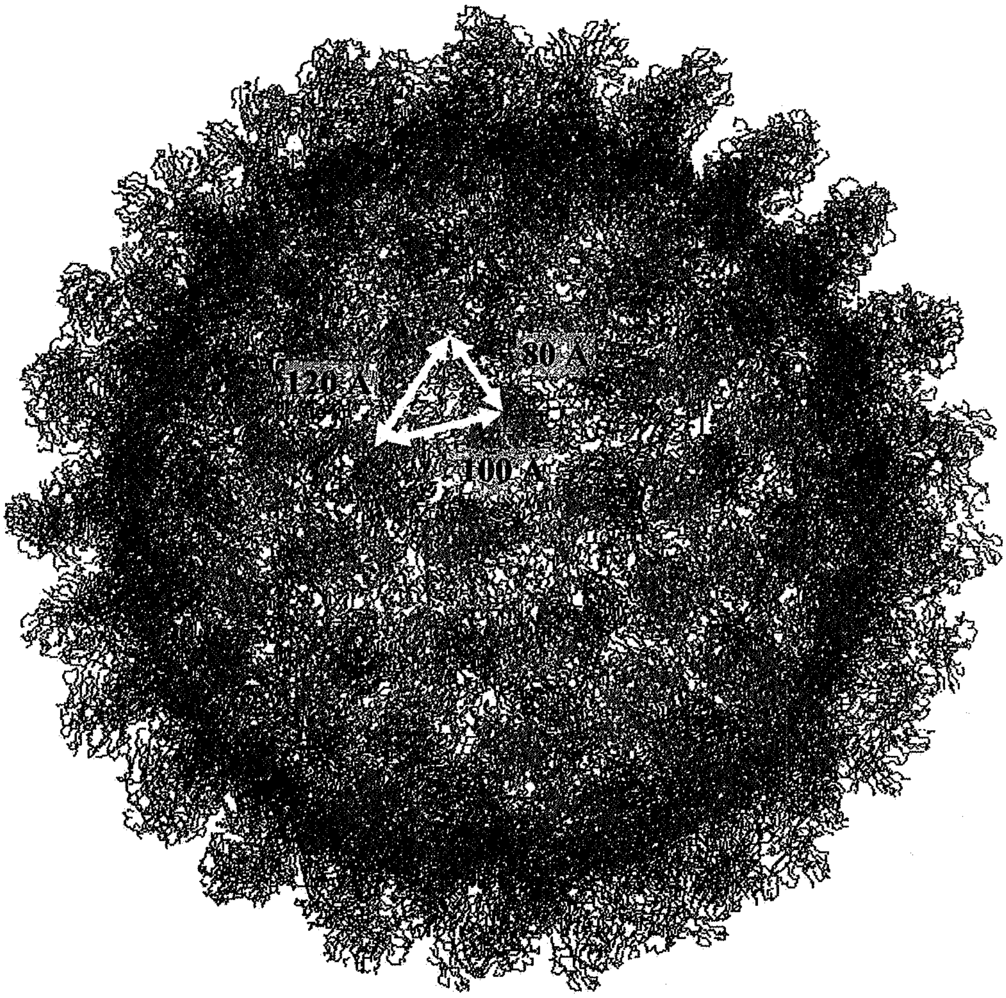

Figure 10.

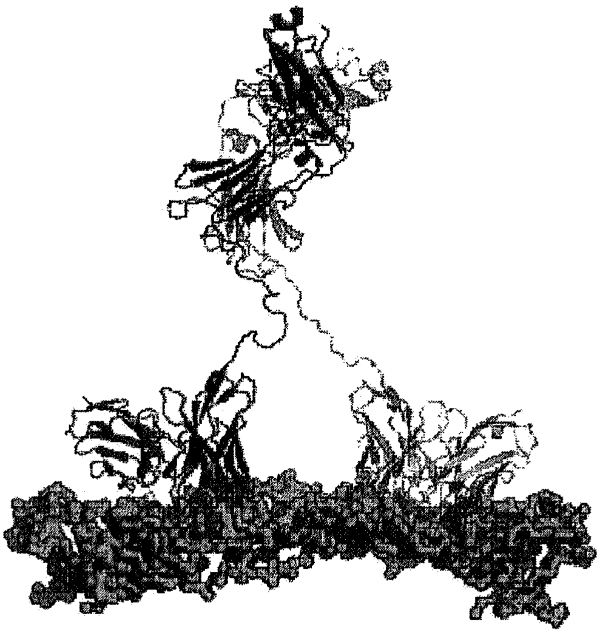

Figure 11.

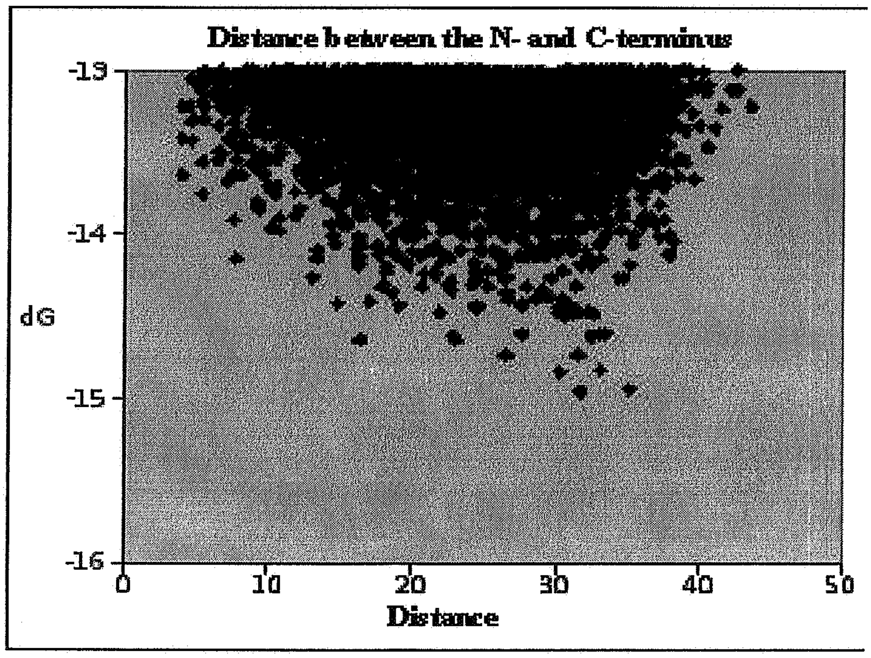

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0014245 A, Zeng L, Markoff L
- WO 9813500 A
- WO 9837911 A, Clark and Elbing
- US 6184024 B, Lai CJ
- WO 9906068 A
- WO 9915692 A
- WO 2003008571 A


### Non-patent literature cited in the description


- **HALSTEAD,S.B.** Neutralization and antibody-dependent enhancement of dengue viruses. *Adv. Virus Res.,* 2003, vol. 60, 421-67421-467
- *Philippines and Thailand. Trans Assoc Physicians,* 1960, vol. 73, 140-155
- **KOURI GP ; GUZMÁN MG.** Bravo JR. Why dengue hemorrhagic fever in Cuba?. *An integral analysis. Trans Roy Soc Trop Med Hyg,* 1987, vol. 72, 821-823
- **HALSTEAD SB.** Pathogenesis of dengue: challenges to molecular biology. *Science,* 1988, vol. 239, 476-481
- **MODIS,Y. ; OGATA, S. ; CLEMENTS, D. ; HARRISON, S.C.** A ligand-binding pocket in the dengue virus envelope glycoprotein. *Proc. Natl. Acad. Sci. U. S. A,* 2003, vol. 100, 6986-6991
- **MODIS, Y. ; OGATA, S. ; CLEMENTS, D. ; HARRISON, S.C.** Variable Surface Epitopes in the Crystal Structure of Dengue Virus Type 3 Envelope Glycoprotein. *J. Virol.,* 2005, vol. 79, 1223-1231
- **REY,F.A. ; HEINZ,F.X. ; MANDL,C. ; KUNZ,C. ; HARRISON,S.C.** The envelope glycoprotein from tick-borne encephalitis virus at 2 A resolution. *Nature,* 1995, vol. 375, 291-298
- **HEINZ, F. X. ; S. L. ALLISON.** Flavivirus structure and membrane fusion. *Adv Virus Res.,* 2003, vol. 59, 63-97
- **MODIS, Y. ; S. OGATA ; D. CLEMENTS ; S. C. HARRISON.** Structure of the dengue virus envelope protein after membrane fusion. *Nature,* 2004, vol. 427, 393-319
- **CHEN, Y. ; T. MAGUIRE ; R. E. HILEMAN ; J. R. FROMM ; J. D. ESKO ; R. J.LINHARDT ; R. M. MARKS.** Dengue virus infectivity depends on envelope protein binding to target cell heparan sulfate. *Nat. Med.,* 1997, vol. 3, 866-871
- **NAVARRO-SANCHEZ, E. ; R. ALTMEYER ; A. AMARA ; O. SCHWARTZ ; F. FIESCHI ; J. L. VIRELIZIER ; F. ARENZANA-SEISDEDOS ; P. DESPRES.** Dendritic-cell-specific ICAM3-grabbing non-integrin is essential for the productive infection of human dendritic cells by mosquito-cell-derived dengue viruses. *EMBO Rep.,* 2003, vol. 4, 1-6
- **TASSANEETRITHEP, B. ; T. H. BURGESS ; A. GRANELLI-PIPERNO ; C. TRUMPFHELLER ; J. FINKE ; W SUN ; M. A. ELLER ; K. PATTANAPANYASAT ; S. SARASOMBATH ; D. L. BIRX.** DC-SIGN (CD209) mediates dengue virus infection of human dendritic cells. *J. Exp. Med.,* 2003, vol. 197, 823-829
- **ALLISON, S. L. ; K. STADLER ; C. W MANDL ; C. KUNZ ; F. X. HEINZ.** Synthesis and secretion of recombinant tick-borne encephalitis virus protein E in soluble and particulate form. *J. Virol.,* 1995, vol. 69, 5816-5820
- Flaviviridae: the viruses and their replication. **RICE, C. M.** Virology. Lippincott-Raven, 1996, 931-959
- **GUIRAKHOO, F. ; HEINZ, F. X. ; MANDL, C. W ; HOLZMANN, H. ; KUNZ, C.** Fusion activity of flaviviruses: comparison of mature and immature (prM containing) tick-borne encephalitis virions. *J. Gen. Virol.,* 1991, vol. 72, 1323-1329
- **GUIRAKHOO, F. ; BOLIN, R. A. ; ROEHRIG, J. T.** The Murray Valley encephalitis virus prM protein confers acid resistance to virus particles and alters the expression of epitopes within the R2 domain of E glycoprotein. *Virology,* 1992, vol. 191, 921-931
- **LORENZ, I. C. ; ALLISON, S. L. ; HEINZ, F. X. ; HELENIUS, A.** Folding and dimerization of tick-borne encephalitis virus envelope proteins prM and E in the endoplasmic reticulum. *J. Virol.,* 2002, vol. 76, 5480-5491
- **STADLER, K. ; ALLISON, S. L. ; SCHALICH, J. ; HEINZ, F. X.** Proteolytic activation of tick-borne encephalitis virus by furin. *J. Virol.,* 1997, vol. 71, 8475-8481

- **ELSHUBER, S. ; ALLISON, S. L. ; HEINZ, F. X. ; MANDL, C. W.** Cleavage of protein prM is necessary for infection of BHK-21 cells by tick-borne encephalitis virus. *J. Gen. Virol.,* 2003, vol. 84, 183-191
- **ZHANG W ; CHIPMAN PR ; CORVER J ; JOHNSON PR ; ZHANG Y ; MUKHOPADHYAY S ; BAKER TS ; STRAUSS JH ; ROSSMANN MG ; KUHN RJ.** Visualization of membrane protein domains by cryo-electron microscopy of dengue virus. *Nat Struct Biol.,* 2003, vol. 10, 907-12
- **KUHN, R. J. et al.** Structure of dengue virus: implications for flavivirus organization, maturation, and fusion. *Cell,* 2002, vol. 108, 717-725
- **ZHANG, Y. et al.** Structures of immature flavivirus particles. *EMBO J.,* 2003, vol. 22, 2604-2613
- **ALLISON, S. L. et al.** Oligomeric rearrangement of tick-borne encephalitis virus envelope proteins induced by an acidic pH. *J. Virol.,* 1995, vol. 69, 695-700
- **MODIS, Y. ; OGATA, S. ; CLEMENTS, D. ; HARRISON, S. C.** Structure of the dengue virus envelope protein after membrane fusion. *Nature,* 2004, vol. 427, 313-319
- **BRESSANELLI, S. et al.** Structure of a flavivirus envelope glycoprotein in its low pH-induced membrane fusion conformation. *EMBO J.,* 2004, vol. 23, 728-738
- **HALSTEAD S.B.** Neutralization and antibody-dependent enhancement of dengue viruses. *Adv Virus Res.,* 2003, vol. 60, 421-67
- **SABIN, A. B.** Research on dengue during World War II. *Am. J. Trop. Med. Hyg.,* 1952, vol. 1, 30-50
- **HALSTEAD, S. B. ; E. J. O'ROURKE.** Dengue viruses and mononuclear phagocytes. I. Infection enhancement by non-neutralizing antibody. *J. Exp. Med.,* 1977, vol. 146, 201-217
- **LITTAUA, R. ; I. KURANE ; F. A. ENNIS.** Human IgG Fc receptor II mediates antibody-dependent enhancement of dengue virus infection. *J. Immunol.,* 1990, vol. 144, 3183-3186
- **HALSTEAD, S.B. ; ROJANASUPHOT, S. ; SANGKAWIBHA, N.** Original antigenic sin in dengue. *Am. J. Trop. Med. Hyg.,* 1983, vol. 32, 154-156
- **BRANDT, W E. ; J. M. MCCOWN ; M. K. GENTRY ; P. K. RUSSELL.** Infection enhancement of dengue type 2 virus in the U-937 human monocyte cell line by antibodies to flavivirus cross-reactive determinants. *Infect. Immun.,* 1982, vol. 36, 1036-1041
- **HALSTEAD, S. B. ; C. N. VENKATESHAN ; M. K. GENTRY ; L. K. LARSEN.** Heterogeneity of infection enhancement of dengue 2 strains by monoclonal antibodies. *J. Immunol,* 1984, vol. 132, 1529-1532
- **MORENS, D. M. ; S. B. HALSTEAD ; N. J. MARCHETTE.** Profiles of antibody-dependent enhancement of dengue virus type 2 infection. *Microb. Pathog.,* 1987, vol. 3, 231-237
- **HEINZ. T. ; ROEHRIG, J.T. ; BOLIN, R.A. ; KELLY, R.G.** Monoclonal Antibody Mapping of the Envelope Glycoprotein of the Dengue 2 Virus, Jamaica. *VIROLOGY,* 1998, vol. 246, 317-328
- Immunochemistry of Viruses. II. The Basis for Serodiagnosis and Vaccines. **HEINZ, F. X ; ROEHRIG, J. T.** Flaviviruses. Elsevier, 1990, 289-305
- **MANDL, C. W. ; GUIRAKHOO, F. G. ; HOLZMANN, H. ; HEINZ, F. X. ; KUNZ, C.** Antigenic structure of the flavivirus envelope protein E at the molecular level, using tick-borne encephalitis virus as a model. *J. Virol.,* 1989, vol. 63, 564-571
- **I. L. SERAFIN ; J. G. AASKOV.** Identification of epitopes on the envelope (E) protein of dengue 2 and dengue 3 viruses using monoclonal antibodies. *Arch Virol,* 2001, vol. 146, 2469-2479
- **GUIRAKHOO, F. ; R. A. BOLIN ; J. T. ROEHRIG.** The Murray Valley encephalitis virus prM protein confers acid resistance to virus particles and alters the expression of epitopes within the R2 domain of E glycoprotein. *Virology,* 1992, vol. 191, 921-931
- **SHEPARD DS ; SUAYA JA ; HALSTEAD SB ; NATHAN MB ; GUBLER DJ ; MAHONEY RT ; WANG DN ; MELTZER MI.** Cost-effectiveness of a pediatric dengue vaccine. *Vaccine,* 2004, vol. 22 (9-10), 1275-80
- **BARRETT, A.D.** Current status of flavivirus vaccines. *Ann. N. Y. Acad. Sci.,* 2001, vol. 951, 262-271
- **CHANG GJ ; KUNO G ; PURDY DE ; DAVIS BS.** Recent advancement in flavivirus vaccine development. *Expert Rev Vaccines,* 2004, vol. 3 (2), 199-220
- **BHAMARAPRAVATI, N. ; SUTEE, Y.** Live attenuated tetravalent dengue vaccine. *Vaccine,* 2000, vol. 18, 44-47
- **ECKELS, K.H. et al.** Modification of dengue virus strains by passage in primary dog kidney cells: preparation of candidate vaccines and immunization of monkeys. *Am. J. Trop. Med. Hyg.,* 2003, vol. 69, 12-16
- **INNIS, B.L. ; ECKELS, K.H.** Progress in development of a live-attenuated, tetravalent dengue virus vaccine by the United States Army Medical Research and Materiel Command. *Am. J. Trop. Med. Hyg.,* 2003, vol. 69, 1-4
- **BLANEY, J.E, JR. ; MANIPON, G.G. ; MURPHY, B.R. ; WHITEHEAD, S.S.** Temperature sensitive mutations in the genes encoding the NS1, NS2A, NS3, and NS5 nonstructural proteins of dengue virus type 4 restrict replication in the brains of mice. *Arch. Virol.,* 2003, vol. 148, 999-1006

- **DURBIN, A.P. et al.** Attenuation and immunogenicity in humans of a live dengue virus type-4 vaccine candidate with a 30 nucleotide deletion in its 3'-untranslated region. *Am. J. Trop. Med. Hyg.,* 2001, vol. 65, 405-413
- **GUIRKHOO F ; ARROYO J ; PUGACHEV KV et al.** Construction, safety, and immunogenicity in non-human primates of a chimeric yellow fever-dengue virus tetravalent vaccine. *J Virol,* 2001, vol. 75, 7290-304
- **HUANG CY ; BUTRAPET S ; PIERRO DJ et al.** Chimeric dengue type 2 (vaccine strain PDK-53)/dengue type 1 virus as a potential candidate dengue type 1 virus vaccine. *J Virol,* 2000, vol. 74, 3020-28
- **MARKOFF L ; PANG X ; HOUNG HS et al.** Derivation and characterization of a dengue 1 host-range restricted mutant virus that is attenuated and highly immunogenic in monkeys. *J Virol,* 2002, vol. 76, 3318-28
- **SELIGMAN SJ ; GOULD EA.** Live flavivirus vaccines: reasons for caution. *Lancet,* 2004, vol. 363 (9426), 2073-5
- **CHANG, G.J. ; DAVIS, B.S. ; HUNT, A.R. ; HOLMES, D.A. ; KUNO, G.** Flavivirus DNA vaccines: current status and potential. *Ann. N. Y. Acad. Sci.,* 2001, vol. 951, 272-285
- **SIMMONS, M. ; MURPHY, G.S. ; KOCHEL, T. ; RAVIPRAKASH, K. ; HAYES, C. G.** Characterization of antibody responses to combinations of a dengue-2 DNA and dengue-2 recombinant subunit vaccine. *Am. J. Trop. Med. Hyg.,* 2001, vol. 65, 420-426
- **FEIGHNY, R. ; BORROUS, J. ; PUTNAK R.** Dengue type-2 virus envelope protein made using recombinant baculovirus protects mice against virus challenge. *Am. J. Trop. Med. Hyg.,* 1994, vol. 50 (3), 322-328
- **DEUBEL, V. ; STAROPOLI, I. ; MEGRET, F. et al.** Affinity-purified dengue-2 virus envelope glycoprotein induces neutralising antibodies and protective immunity in mice. *Vaccine,* 1997, vol. 15, 1946-1954
- **ROTHMAN AL.** Dengue: defining protective versus pathologic immunity. *J. Clin. Invest,* 2004, vol. 113, 946-951
- **MEN, R. ; T. YAMASHIRO ; A. P. GONCALVEZ ; C. WERNLY ; D. J. SCHOFIELD ; S. U. EMERSON ; R. H. PURCELL ; C. J. LAI.** Identification of chimpanzee Fab fragments by repertoire cloning and production of a full-length humanized immunoglobulin G1 antibody that is highly efficient for neutralization of dengue type 4 virus. *J. Virol.,* 2004, vol. 78, 4665-4674
- **GONCALVEZ, A.P. ; R. MEN ; C. VILERNLY ; R. H. PURCELL ; C.J. LAI.** Chimpanzee Fab fragments and a derived humanized immunoglobulin G1 antibody that efficiently cross-neutralize dengue type 1 and type 2 viruses. *J. Virol.,* 2004, vol. 78, 12910-12918
- **WANG, W K. ; D. Y. CHAO ; C. L. KAO ; H. C. WU ; Y. C. LIU ; C. M. LI ; S. C. LIN ; J. H. HUANG ; C. C. KING.** High levels of plasma dengue viral load during defervescence in patients with dengue haemorrhagic fever: implications for pathogenesis. *Virology,* 2003, vol. 305, 330-338
- **VAUGHN,D.W. ; GREEN, S. ; KALAYANAROOJ, S. ; INNIS, B.L. ; NIMMANNITYA, S. ; SUNTAYAKORN, S. ; ENDY, T.P. ; RAENGSAKULRACH, B. ; ROTHMAN, A.L. ; ENNIS, F.A.** Dengue Viremia Titer, Antibody Response Pattern, and Virus Serotype Correlate with Disease Severity. *J. Infect. Dis.,* 2000, vol. 181, 2-9
- **HAHN CS ; FRENCH OG ; FOLEY P ; MARTIN EN ; TAYLOR RP.** Bispecific monoclonal antibodies mediate binding of dengue virus to erythrocytes in a monkey model of passive viremia. *J ImmunoL,* 2001, vol. 166, 1057-65
- **ALTSCHUL, S.F. ; GISH, W. ; MILLER, W. ; MYERS, E.W ; LIPMAN, D.J.** Basic local alignment search tool. *J. Mol. Biol.,* 1990, vol. 215, 403-490
- **PEARSON WR ; LIPMAN DJ.** Improved tools for biological sequence comparison. *Proc Natl Acad Sci U S A.,* 1988, vol. 85, 2444-8
- **HIGGINS D. ; THOMPSON J. ; GIBSON T. ; THOMPSON J. D. ; HIGGINS D. G. ; GIBSON T. J.** CLUSTAL W: improving the sensitivity of progressive multiple sequence alignment through sequence weighting,position-specific gap penalties and weight matrix choice. *Nucleic Acids Res.,* 1994, vol. 22, 4673-4680
- **SIMMONS M ; MURPHY GS ; HAYES CG.** Short report. Antibody responses of mice immunized with a tetravalent dengue recombinant protein subunit vaccine. *Am J Trop Med Hyg.,* 2001, vol. 65, 159-61
- **HERMIDA L ; RODRIGUEZ R ; LAZO L ; SILVA R ; ZULUETA A ; CHINEA G ; LOPEZ C ; GUZMAN MG ; GUILLEN G.** A dengue-2 Envelope fragment inserted within the structure of the P64k meningococcal protein carrier enables a functional immune response against the virus in mice. *J Virol Methods.,* January 2004, vol. 115 (1), 41-9
- **DREW PD ; MOSS MT ; PASIEKA TJ ; GROSE C ; HARRIS WJ ; PORTER AJ.** Multimeric humanized varicella-zoster virus antibody fragments to gH neutralize virus while monomeric fragments do not. *J Gen Virol.,* 2001, vol. 82, 1959-63
- **LANTTO J ; FLETCHER JM ; OHLIN M.** A divalent antibody format is required for neutralization of human cytomegalovirus via antigenic domain 2 on glycoprotein B. *J Gen Virol.,* 2002, vol. 83, 2001-5
- **GONCALVEZ AP ; MEN R ; WERNLY C ; PURCELL RH ; LAI CJ.** Chimpanzee Fab fragments and a derived humanized immunoglobulin G1 antibody that efficiently cross-neutralize dengue type 1 and type 2 viruses. *J Virol.,* 2004, vol. 78, 12910-8

- **THULLIER, P. ; P. LAFAYE ; F. MEGRET ; V. DEUBEL ; A. JOUAN ; J. C. MAZIE.** A recombinant Fab neutralizes dengue virus in vitro. *J. Biotechnol.,* 1999, vol. 69, 183-190
- **DAMES SA ; KAMMERER RA ; WILTSCHECK R ; ENGEL J ; ALEXANDRESCU AT.** NMR structure of a parallel homotrimeric coiled coil. *Nat Struct Biol.,* vol. 5, 687-91
- **ROOMAN MJ ; KOCHER JP ; WODAK SJ.** Prediction of protein backbone conformation based on seven structure assignments. Intluence of local interactions. *J Mol Biol.,* 1991, vol. 221, 961-79
- **JONES, G. Y COLS.** Development and validation of a genetic algorithm for flexible docking. *J. Mol. Biol.,* 1997, vol. 267, 727-748
- **KUNTZ, I.D. Y COLS.** A geometric approach to macromolecule-ligand interactions. *J. Mol. Biol.,* 1982, vol. 161, 269-288
- **OLENDER, R. ; ROSENFELD, R.** A fast algorithm for searching for molecules containing a pharmacophore in very large virtual combinatorial libraries. *J. Chem. Inf. Comput. Sci.,* 2001, vol. 41, 731-738
- **IRWING, J.J. ; SCOICHET, B.K.** Zinc - A free Database of commercially available compounds for virtual screening. *J. Chem. Inf. Model.,* 2005, vol. 45, 177-182
- **GLASER, F. ; PUPKO, T. ; PAZ, I. ; BELL, R.E. ; BECHOR-SHENTAL, D. ; MARTZ, E. ; BEN-TAL, N.** *Bioinformatics,* 2003, vol. 19, 163-164
- **VRIEND G.** WHAT IF. a molecular modeling and drug design program. *J Mol Graph.,* 1990, vol. 8, 52-629
- **DEUBEL V. ; KINNEY R.M. ; TRENT D.W.** Nucleotide sequence and deduced amino acid sequence of the structural proteins of dengue type 2 virus, Jamaica genotype. *Virology,* 1986, vol. 155 (2), 365-377
- **AGARWAL KL ; BÜCHI H ; CARUTHERS MH ; GUPTA N ; KHORANA HG ; KUMAS A ; OHTSUKA E ; RAJBHANDARY UL ; VAN DE SANDE JH ; SGARAMELLA V.** Total synthesis of the Gene for an alanine transfer ribonucleic acid from yeast. *Nature,* 1970, vol. 227, 27-34
- **BEAUCAGE SL ; CARUTHERS MH.** Deoxynucleoside phosphoramidites- A new class of key intermediates for deoxypolynucleotide synthesis. *Tetrahedron Letters,* 1981, vol. 22, 1859
- **BULLOCK WO ; FEMÁNDEZ JM.** Short JM. XL-1Blue: A high efficiency plasmid transforming recA Escherichia coli K12 strain with beta-galactosidase selection. *Biotechniques,* 1987, vol. 5, 376-8
- **SAMBROOK J ; FRITSCH EF ; MANIATIS T.** Molecular cloning: A laboratory manual. New York, USA. Cold Spring Harbor Laboratory Press, 1989
- **SULKOWSKI, E.** *Purification of proteins by IMAC. Trends Biotechnol.,* 1985, vol. 3, 1-7
- **STUDIER, F. W ; B. A. MOFFATT.** Use of bacteriophage T7 RNA polymerase to direct selective high-level expression of cloned genes. *J. Mol.Biol.,* 1986, vol. 189.1, 113-30
- **AUSUBEL, F.M. ; BRENT, R. ; KINGSTON, R.E. ; MOORE, D.D. ; SEIDMAN, J.G. ; SMITH, J. A. ; STRUHL, K.** Current Protocols in Molecular Biology. John Wiley & Sons, 1989
- **SULKOWSKI, E.** Purification of proteins by IMAC. *Trends Biotechnol.,* 1985, vol. 3, 1-7
- **ROEHRIG JT ; VOLPE KE ; SQUIRES J ; HUNT AR ; DAVIS BS ; CHANG GJ.** Contribution of disulfide bridging to epitope expression of the dengue type 2 virus envelope glycoprotein. *J Virol.,* 2004, vol. 78, 2648-52
- **CLARKE, D. M. ; CASALS, J.** Techniques for hemaglutination and hemaglutination-inhibition with arthropode-bome viruses. *American Journal of Tropical Medicine and Hygiene,* 1958, vol. 7, 561-573
- **S.R. COMEAU ; D.W. GATCHELL ; S. VAJDA ; C.J. CAMACHO.** ClusPro: an automated docking and discrimination method for the prediction of protein complexes. *Bioinformatics,* 2004, vol. 20, 45-50, http://nrc.bu.edulcluster
- **MANDELL JG ; ROBERTS VA ; PIQUE ME ; KOTLOVYI V ; MITCHELL JC ; NELSON E ; TSIGELNY I ; TEN EYCK LF.** Protein docking using continuum electrostatics and geometric fit. *Protein Eng,* 2001, vol. 14, 105-13
- **CHEN R ; LI L ; WENG Z.** ZDOCK: An Initial-stage Protein-Docking Algorithm. *Proteins,* 2003, vol. 52, 80-87
- **ROEHRIG, J.T. ; BOLIN, R.A. ; KELLY, R.G.** Monoclonal Antibody Mapping of the Envelope Glycoprotein of the Dengue 2 Virus, Jamaica. *Virology,* 1998, vol. 246, 317-328
- **JONES, S. ; THORNTON, J.M.** Principles of Protein-Protein Interactions Derived From Structural Studies. *PNAS,* 1996, vol. 93, 13-20, http://www.biochem.ucl.ac.uk/bsm/PP/server
- **LUND, J. ; TAKAHASHI, N. ; POUND, J. D. ; GOODALL, M. ; JEFFERIS, R.** *J. Immunol.,* 1996, vol. 157, 4963-4969
- **LUND, J. ; TAKAHASHI, N. ; POUND, J. D. ; GOODALL, M. ; NAKAGAWA, H. ; JEFFERIS, R.** *FASEB. J.,* 1995, vol. 9, 115-119
- **WILTSCHECK R ; KAMMERER RA ; DAMES SA ; SCHULTHESS T ; BLOMMERS MJ ; ENGEL J ; ALEXANDRESCU AT.** Heteronuclear NMR assignments and secondary structure of the coiled coil trimerization domain from cartilage matrix protein in oxidized and reduced forms. *Protein Sci.,* 1997, vol. 6, 1734-45

- **BEAUCAGE SL ; CARUTHERS MH.** Deoxynucleoside phosphoramidites- A new class of key intermediates for deoxypolynucleotide synthesis. *Tetrahedron Letters,* 1981, vol. 22, 1859
- **SAMBRROOK J ; FRITSCH EF ; MANIATIS T.** Molecular cloning: A laboratory manual. New York, USA. Cold Spring Harbor Laboratory Press, 1989
- **STUDIER, F. W. ; B. A. MOFFATT.** Use of bacteriophage T7 RNA polymerase to direct selective high-level expression of cloned genes. *J.Mol.Biol.,* 1986, vol. 189.1, 113-30
- **AUSUBEL, F.M. ; BRENT, R. ; KINGSTON, R.E. ; MOORE, D.D. ; SEIDMAN, J.G. ; SMITH, J.A. ; STRUHL, K.** Current Protocols in Molecular Biology. John Wiley & Sons, 1989